# EUROPEAN PATENT APPLICATION

(11) **EP 4 755 389 A1**
(43) Date of publication of application: **10.06.2026**
(21) Application number: 24848139.2
(22) Date of filing: 25.07.2024
(51) Int. Cl.: A61K 35/761, A61K 38/16, A61K 38/17, A61K 31/713, C12N 7/00, C12N 15/86, A61P 27/02

(54) **FUSION POLYPEPTIDE, EXPRESSION CASSETTE CONTAINING CODING GENE OF FUSION POLYPEPTIDE, GENE DELIVERY VECTOR, PHARMACEUTICAL COMPOSITION AND USE**

(30) Priority: 01.08.2023 WO PCT/CN2023/110563
(71) Applicant: InnoVec Biotherapeutics, Haidian District Beijing (CN)
(72) Inventor: WANG, Cheng, Haidian District, Beijing (CN)
(74) Representative: E. Blum & Co. AG
(86) International application number: PCT/CN2024/107562
(87) International publication number: WO 2025/026182

(57) **Abstract**

Provided are a fusion polypeptide, an expression cassette containing a coding gene of the fusion polypeptide, a gene delivery vector, a pharmaceutical composition and a use. Specifically, provided is a fusion polypeptide, containing a secretion signal peptide and an aflibercept polypeptide, wherein the secretion signal peptide is selected from any one among a human albumin secretion signal peptide, a human Opticin protein secretion signal peptide, a human interleukin-2 secretion signal peptide and a mouse receptor tyrosine kinase-like orphan receptor 1 secretion signal peptide. The provided fusion polypeptide, a polynucleotide encoding same, a gene expression cassette containing the polynucleotide, and a gene delivery vector containing the expression cassette can improve the expression of aflibercept and improve the effect of treating ocular diseases by using aflibercept.

## Description

### PRIORITY AND RELATED APPLICATIONS

The present disclosure claims the benefit of a priority of the PCT International Application No. PCT/CN2023/110563, filed on August 1, 2023 and entitled "FUSION POLYPEPTIDE, EXPRESSION CASSETTE CONTAINING CODING GENE OF FUSION POLYPEPTIDE, GENE DELIVERY VECTOR, PHARMACEUTICAL COMPOSITION AND USE", the entire contents of which including the appendices are incorporated herein by reference.

### TECHNICAL FIELD

The present disclosure belongs to the technical field of gene therapy, and specifically relates to a fusion polypeptide, an expression cassette containing a coding gene of the fusion polypeptide, a gene delivery vector, a pharmaceutical composition, and use.

### BACKGROUND

Pathological angiogenesis-associated eye diseases, such as Wet Age-related Macular Degeneration (abbreviated as Wet AMD), are characterized by rapid onset, leading to irreversible and severe vision decline or even loss in patients within a short period. While accounting for only 20% of all AMD cases, Wet AMD is responsible for up to 80% of blindness, which profoundly affects the quality of life of patients and their families. Wet AMD is the third leading cause of blindness globally and a major cause of vision loss in the elderly population. Its incidence in China is rising annually, with approximately 9 million people currently affected by Wet AMD. In China, 15.5% of adults over 50 years old suffer from AMD, and wet AMD accounts for 11.9% of all AMD cases.

There is currently no effective cure for Wet AMD. The mainstream treatment is anti-angiogenesis at present. This therapy relies mainly on regular intravitreal injections of anti-VEGF recombinant proteins or antibodies. This conventional treatment method has significant drawbacks. It requires repeated administration, causes considerable damage to patients, and results in poor compliance and serious side effects. Therefore, there is an urgent need to develop a gene therapy drug capable of treating this disease with a single administration.

Aflibercept is a recombinant fusion protein that acts as a decoy receptor for the vascular endothelial growth factor subtypes A and B (VEGF-A and VEGF-B) and the placental growth factor PIGF. By binding to these ligands, aflibercept is capable of preventing these ligands from binding to the vascular endothelial growth factor receptors (VEGFRs) VEGFR-1 and VEGFR-2 to inhibit neovascularization and reduce vascular permeability. Aflibercept is composed of domain 2 of VEGFR-1 and domain 3 of VEGFR-2 that are fused to the Fc fragment of IgG1. Aflibercept is commercially available under the brand name of EYLEA^{®} (Aflibercept) as an ophthalmic intravitreal injection formulation of aflibercept fusion protein.

In recent years, gene therapy has become mature and safe, with further improved gene transfer and delivery efficiency and substantial progress in clinical research. In 2008, the successful treatment of Leber's congenital amaurosis (LCA) using an adeno-associated virus vector brought gene therapy back into the limelight. In 2012, Glybera - an adeno-associated virus-derived gene therapy drug - was the first approved by the European Medicines Agency (EMA) for marketing, ushering in a new era of gene therapy. In 2017, the U.S. Food and Drug Administration (FDA) approved Luxturna - a gene therapy drug that utilizes an Adeno-Associated Virus 2 (AAV2) vector to deliver the *RPE65* gene to treat vision loss caused by Inherited Retinal Diseases (IRDs). Subsequently, the field of gene therapy has thrived, marked by a growing number of reported clinical cases and drug candidates and an increasing recognition of the safety and efficacy of gene therapy. In gene therapy, the AAV viral vectors are a primary delivery modality, and a major direction for the recombinant AAV-delivered gene therapy is the Central Nervous System (CNS), including the eyes and brain. The eye is a relatively closed environment. The gene therapy of delivering AAV via direct intraocular injection can achieve the therapeutic effect on a variety of hereditary eye diseases. Clinical trials have shown that intraocular administration of the AAV-delivered gene therapy does not cause systemic side effects or elicit a significant immune response.

### SUMMARY

### Technical Problem

Although EYLEA^{®} (Aflibercept) is currently the standard treatment for wet AMD in patients, a gene therapy approach designed to deliver aflibercept to the eye can provide patients with an improved treatment option. This is because the gene therapy allows for extended or sustained *in vivo* release of aflibercept without repeated injections, which can increase the risk of inflammation, infection and other side effects in some patients.

### Solution to Problem

[1]. A fusion polypeptide, comprising a secretory signal peptide and an aflibercept polypeptide, wherein the secretory signal peptide is any one selected from a human albumin secretory signal peptide, a human Opticin protein secretory signal peptide, a human interleukin-2 secretory signal peptide, and a mouse receptor tyrosine kinase-like orphan receptor 1 secretory signal peptide.
[2]. The fusion polypeptide according to [1], wherein an amino acid sequence of the aflibercept polypeptide comprises an amino acid sequence as set forth in SEQ ID NO: 48; and/or
   an amino acid sequence of the secretory signal peptide comprises an amino acid sequence as set forth in any one of SEQ ID NOs: 44 to 47.
[3]. The fusion polypeptide according to [1] or [2], wherein an amino acid sequence of the fusion polypeptide comprises any one selected from the following sequences:
   (i) an amino acid sequence as set forth in any one of SEQ ID NO: 1, 30, 32 or 34;
   (ii) an amino acid sequence having at least 80%, 82%, 85%, 87%, 90%, 92%, 95%, 96%, 97%, 98% or 99% identity to the amino acid sequence as set forth in any one of SEQ ID NO: 1, 30, 32 or 34, and retaining a function of the amino acid sequence as set forth in any one of SEQ ID NO: 1, 30, 32 or 34;
   (iii) an amino acid sequence obtained by adding, substituting, deleting or inserting one or more amino acid residues in the amino acid sequence as set forth in any one of SEQ ID NO: 1, 30, 32 or 34, and retaining a function of the amino acid sequence as set forth in any one of SEQ ID NO: 1, 30, 32 or 34; or
   (iv) an amino acid sequence encoded by a nucleotide sequence that hybridizes under stringency conditions with a polynucleotide sequence encoding the amino acid sequence as set forth in any one of SEQ ID NO: 1, 30, 32 or 34, and the amino acid sequence retaining a function of the amino acid sequence as set forth in any one of SEQ ID NO: 1, 30, 32 or 34, wherein the stringency conditions are moderate stringency conditions, moderate-high stringency conditions, high stringency conditions, or very high stringency conditions.
[4]. An isolated polynucleotide encoding the fusion polypeptide according to any one of [1] to [3], wherein
   preferably, the polynucleotide comprises a nucleotide sequence as set forth in any one of SEQ ID NO: 2, 29, 31 or 33, or a nucleotide sequence having at least 80%, 82%, 85%, 87%, 90%, 92%, 95%, 96%, 97%, 98% or 99% identity to the nucleotide sequence as set forth in any one of SEQ ID NO: 2, 29, 31 or 33.
[5]. A gene expression cassette, comprising the polynucleotide according to [4].
[6]. The gene expression cassette according to [5], wherein the gene expression cassette further comprises, upstream of the polynucleotide encoding the fusion polypeptide, one or more of a promoter, an enhancer, an intron, and a Kozak sequence.
[7]. The gene expression cassette according to [6], wherein the promoter comprises any one of a CBh promoter, a CB7 promoter, and a CMV promoter;
   preferably, the CBh promoter comprises a sequence as set forth in SEQ ID NO: 3 or a sequence having at least 85% identity thereto; and/or
   the CB7 promoter comprises a sequence as set forth in SEQ ID NO: 4 or a sequence having at least 85% identity thereto; and/or
   the CMV promoter comprises a sequence as set forth in SEQ ID NO: 5 or a sequence having at least 85% identity thereto.
[8]. The gene expression cassette according to [6] or [7], wherein the intron comprises a human β-globin intron; and
   preferably, the human β-globin intron comprises a sequence as set forth in SEQ ID NO: 6 or a sequence having at least 85% identity thereto.
[9]. The gene expression cassette according to any one of [5] to [8], wherein the gene expression cassette comprises a polyadenylation region located downstream of the polynucleotide encoding the fusion polypeptide;
   optionally, the polyadenylation region is any one selected from a bovine growth hormone polyadenylation region, a human growth hormone polyadenylation region, and a β-globin polyadenylation region;
   preferably, the polyadenylation region comprises the human growth hormone polyadenylation region; and
   more preferably, the human growth hormone polyadenylation region comprises a sequence as set forth in SEQ ID NO: 7 or a sequence having at least 85% identity thereto.
[10]. The gene expression cassette according to any one of [5] to [9], wherein the gene expression cassette comprises a sequence as set forth in any one of SEQ ID NOs: 8 to 10 and 35 to 43 or a sequence having at least 85% identity to any one of SEQ ID NOs: 8 to 10 and 35 to 43.
[11]. A gene delivery vector, comprising the gene expression cassette according to any one of [5] to [10].
[12]. The gene delivery vector according to [11], wherein the gene delivery vector is a viral vector derived from a virus; and
   preferably, the gene delivery vector is a recombinant adeno-associated virus.
[13]. The gene delivery vector according to [12], wherein the recombinant adeno-associated virus comprises a capsid protein, and the gene expression cassette is encapsidated in the capsid protein; and
   optionally, the capsid protein is any one selected from adeno-associated virus serotypes AAV1, AAV2, AAV3, AAV4, AAV5, AAV6, AAV7, AAV8, AAV9, and AAV10, or a variant thereof.
[14]. The gene delivery vector according to [13], wherein the capsid protein is an AAV2 capsid protein or a variant thereof;
   preferably, the capsid protein is an AAV2 capsid protein variant;
   more preferably, the AAV2 capsid protein variant comprises a sequence as set forth in any one of SEQ ID NOs: 16 and 70 to 83, or a sequence having at least 85% identity to any one of SEQ ID NOs: 16 and 70 to 83; and
   further preferably, the AAV2 capsid protein variant comprises a sequence as set forth in SEQ ID NO: 16 or 82, or a sequence having at least 85% identity to SEQ ID NO: 16 or 82.
[15]. A pharmaceutical composition, comprising the fusion polypeptide according to any one of [1] to [3], the polynucleotide according to [4], the gene expression cassette according to any one of [5] to [10] or the gene delivery vector according to any one of [11] to [14], and
   optionally, a pharmaceutically acceptable carrier.
[16]. Use of the fusion polypeptide according to any one of [1] to [3], the polynucleotide according to [4], the gene expression cassette according to any one of [5] to [10] or the gene delivery vector according to any one of [11] to [14] in preparation of a medicament for preventing and/or treating a disease, wherein
   optionally, the disease is an eye disease;
   preferably, the eye disease is an eye disease associated with ocular neovascularization or with choroidal neovascularization; and
   more preferably, the eye disease is one or more selected from wet age-related macular degeneration, macular edema secondary to retinal vein occlusion, diabetic macular edema, and diabetic retinopathy.
[17]. A method for preventing and/or treating a disease, comprising administering, to a subject, a prophylactically and/or therapeutically effective amount of the fusion polypeptide according to any one of [1] to [3], the polynucleotide according to [4], the gene expression cassette according to any one of [5] to [10], the gene delivery vector according to any one of [11] to [14] or the pharmaceutical composition according to [15], wherein
   optionally, the disease is an eye disease;
   preferably, the eye disease is an eye disease associated with ocular neovascularization or with choroidal neovascularization; and
   more preferably, the eye disease is one or more selected from wet age-related macular degeneration, macular edema secondary to retinal vein occlusion, diabetic macular edema, and diabetic retinopathy.

### Advantageous Effects of the Invention

The fusion polypeptide, the polynucleotide encoding the fusion polypeptide, the gene expression cassette comprising the polynucleotide, and the gene delivery vector comprising the expression cassette provided in the present disclosure can enhance the expression of aflibercept and improve the therapeutic effect of aflibercept on eye diseases.

### BRIEF DESCRIPTION OF THE DRAWINGS

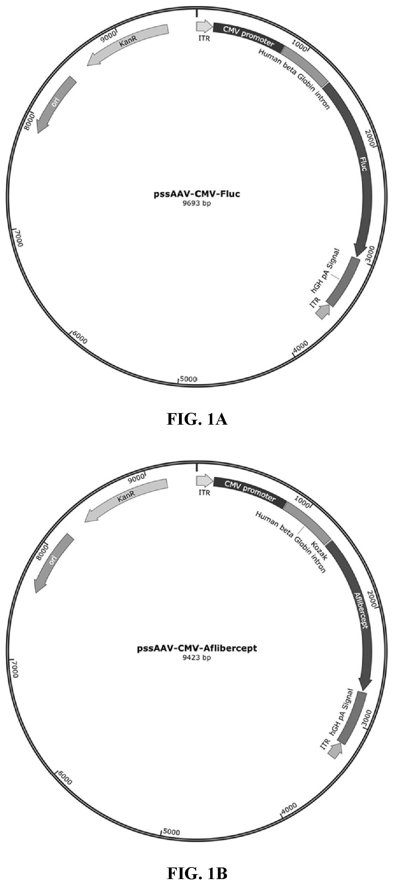

FIG. 1A: Schematic diagram for the structure of the pssAAV-CMV-Fluc-hGH pA vector.
   ITR, inverted terminal repeat (the upstream ITR is approx. 145 bp in length and the downstream ITR is approx. 141 bp in length). CMV promoter, human cytomegalovirus early promoter. Human beta Globin intron. Fluc, firefly luciferase gene coding sequence. hGH polyA, human growth hormone polyadenylation signal. Kan, Kanamycin resistance gene reading frame.
FIG. 1B: Schematic diagram for the structure of the pssAAV-CMV-Aflibercept vector.
   ITR, inverted terminal repeat (the upstream ITR is approx. 145 bp in length and the downstream ITR is approx. 141 bp in length). CMV promoter, human cytomegalovirus early promoter. Human beta Globin intron. Aflibercept, codon-unoptimized coding sequence for aflibercept (containing its native secretory signal peptide sequence). hGH polyA, human growth hormone polyadenylation signal. Kan, Kanamycin resistance gene reading frame.

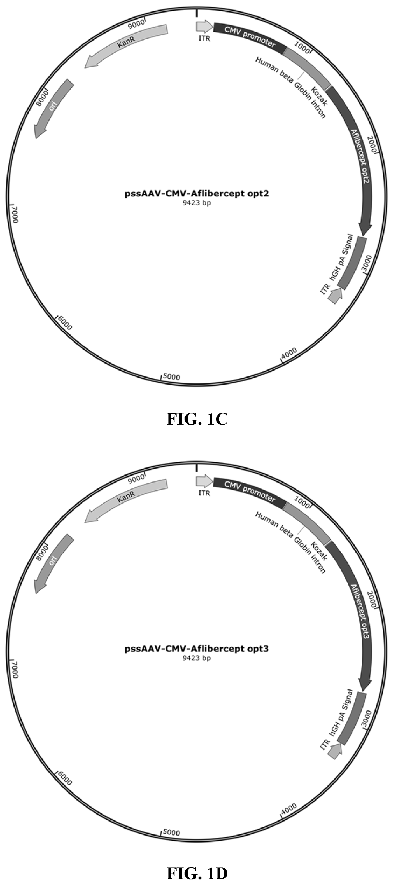

FIG. 1C: Schematic diagram for the structure of the pssAAV-CMV-Aflibercept opt2 vector.
   ITR, inverted terminal repeat (the upstream ITR is approx. 145 bp in length and the downstream ITR is approx. 141 bp in length). CMV promoter, human cytomegalovirus early promoter. Human beta Globin intron. Aflibercept opt2, codon-unoptimized coding sequence for aflibercept (containing its native secretory signal peptide sequence). hGH polyA, human growth hormone polyadenylation signal. Kan, Kanamycin resistance gene reading frame.
FIG. 1D: Schematic diagram for the structure of the pssAAV-CMV-Aflibercept opt3 vector.
   ITR, inverted terminal repeat (the upstream ITR is approx. 145 bp in length and the downstream ITR is approx. 141 bp in length). CMV promoter, human cytomegalovirus early promoter. Human beta Globin intron. Aflibercept opt3, codon-unoptimized coding sequence for aflibercept (containing its native secretory signal peptide sequence). hGH polyA, human growth hormone polyadenylation signal. Kan, Kanamycin resistance gene reading frame.

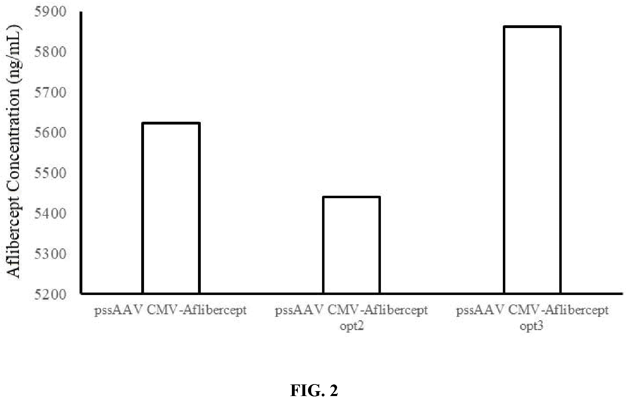

FIG. 2: Effect of codon optimization on aflibercept expression.
   The HEK 293T cells are seeded in a 6-well plate. When growing to 70% to 80% confluency, the cells are transfected with 2 µg/well of pssAAV-CMV-Aflibercept, pssAAV-CMV-Aflibercept opt2, or pssAAV-CMV-Aflibercept opt3 using Lipo3000. At 48 h after transfection, the cells and supernatant are harvested. The aflibercept concentration is determined by Elisa.

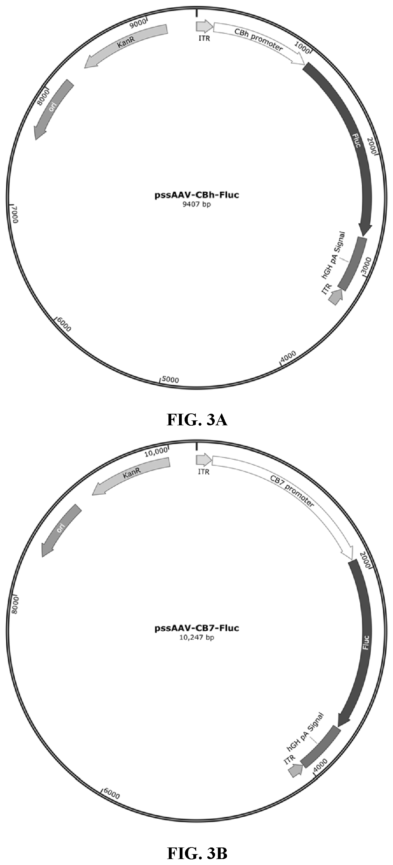

FIG. 3A: Schematic diagram for the structure of the pssAAV-CBh-Fluc-hGH pA vector.
   ITR, inverted terminal repeat (the upstream ITR is approx. 145 bp in length and the downstream ITR is approx. 141 bp in length). CBh promoter, artificially designed promoter composed of a human cytomegalovirus enhancer, a chicken β-actin promoter, and an intron derived partially from chicken β-actin and partially from minute virus of mice (mvm), with the sequence information shown in SEQ ID NO: 3. Fluc, firefly luciferase gene coding sequence. hGH polyA, human growth hormone polyadenylation signal. Kan, Kanamycin resistance gene reading frame.
FIG. 3B: Schematic diagram for the structure of the pssAAV-CB7-Fluc-hGH pA vector.
   ITR, inverted terminal repeat (the upstream ITR is approx. 145 bp in length and the downstream ITR is approx. 141 bp in length). CB7 promoter, artificially designed promoter composed of a human cytomegalovirus enhancer, a chicken β-actin promoter, an intron and the like, with the sequence information shown in SEQ ID NO: 4. Fluc, firefly luciferase gene coding sequence. hGH polyA, human growth hormone polyadenylation signal. Kan, Kanamycin resistance gene reading frame.

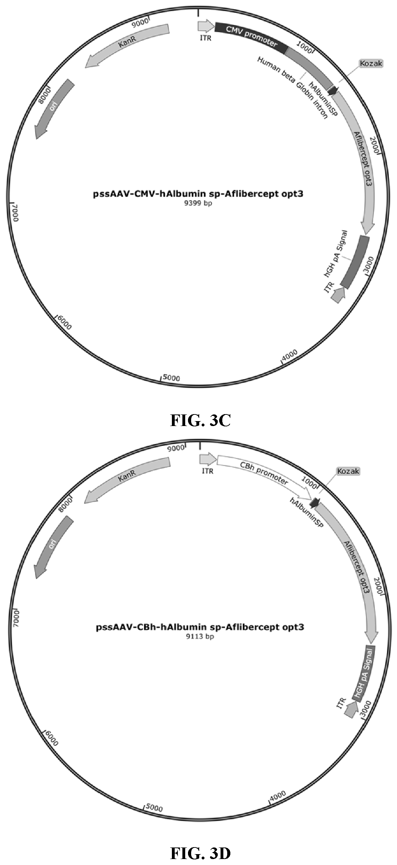

FIG. 3C: Schematic diagram for the structure of the pssAAV-CMV-hAlbumin sp-Aflibercept opt3 vector.
   ITR, inverted terminal repeat (the upstream ITR is approx. 145 bp in length and the downstream ITR is approx. 141 bp in length). CMV promoter, human cytomegalovirus early promoter. Human beta Globin intron. hAlbumin sp, human albumin secretory signal peptide sequence. Aflibercept opt3, codon-optimized aflibercept (without the secretory signal peptide sequence) coding sequence. hGH polyA, human growth hormone polyadenylation signal. Kan, Kanamycin resistance gene reading frame.
FIG. 3D: Schematic diagram for the structure of the pssAAV-CBh-hAlbumin sp-Aflibercept opt3 vector.
   ITR, inverted terminal repeat (the upstream ITR is approx. 145 bp in length and the downstream ITR is approx. 141 bp in length). CBh promoter, artificially designed promoter composed of a human cytomegalovirus enhancer, a chicken β-actin promoter, and an intron derived partially from chicken β-actin and partially from minute virus of mice (mvm), with the sequence information shown in SEQ ID NO: 3. hAlbumin sp, human albumin secretory signal peptide sequence. Aflibercept opt3, codon-optimized aflibercept (without the secretory signal peptide sequence) coding sequence. hGH polyA, human growth hormone polyadenylation signal. Kan, Kanamycin resistance gene reading frame.

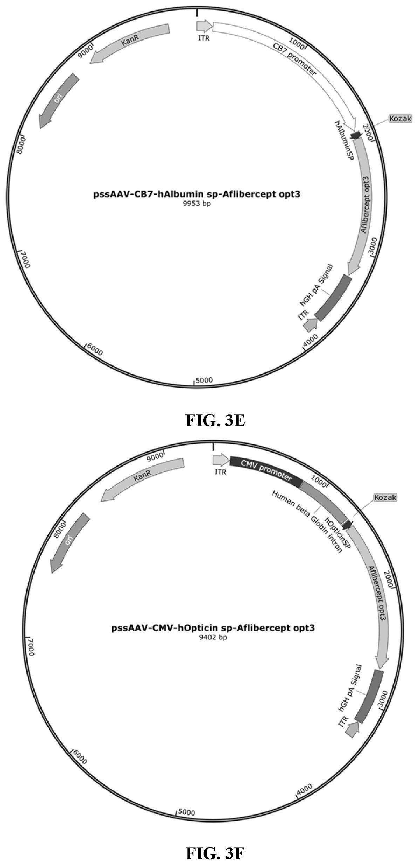

FIG. 3E: Schematic diagram for the structure of the pssAAV-CB7-hAlbumin sp-Aflibercept opt3 vector.
   ITR, inverted terminal repeat (the upstream ITR is approx. 145 bp in length and the downstream ITR is approx. 141 bp in length). CB7 promoter, artificially designed promoter composed of a human cytomegalovirus enhancer, a chicken β-actin promoter, an intron and the like, with the sequence information shown in SEQ ID NO: 4. hAlbumin sp, human albumin secretory signal peptide sequence. Aflibercept opt3, codon-optimized aflibercept (without the secretory signal peptide sequence) coding sequence. hGH polyA, human growth hormone polyadenylation signal. Kan, Kanamycin resistance gene reading frame.
FIG. 3F: Schematic diagram for the structure of the pssAAV-CMV-hOpticin sp-Aflibercept opt3 vector.
   ITR, inverted terminal repeat (the upstream ITR is approx. 145 bp in length and the downstream ITR is approx. 141 bp in length). CMV promoter, human cytomegalovirus early promoter. Human beta Globin intron. hOpticin sp, human Opticin protein secretory signal peptide sequence. Aflibercept opt3, codon-optimized aflibercept (without the secretory signal peptide sequence) coding sequence. hGH polyA, human growth hormone polyadenylation signal. Kan, Kanamycin resistance gene reading frame.

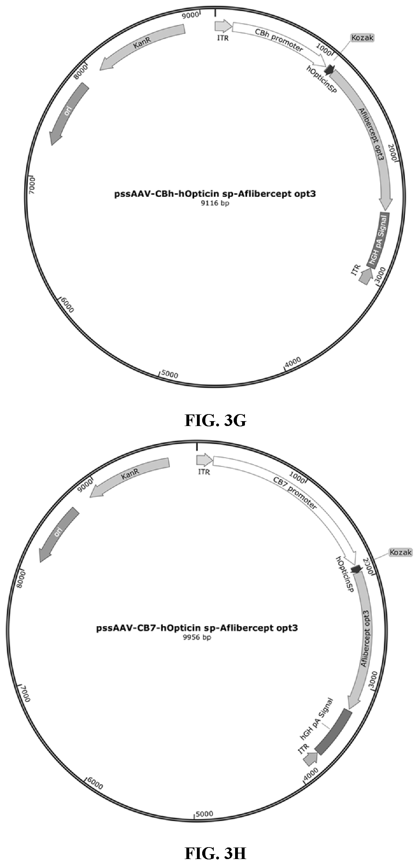

FIG. 3G: Schematic diagram for the structure of the pssAAV-CBh-hOpticin sp-Aflibercept opt3 vector.
   ITR, inverted terminal repeat (the upstream ITR is approx. 145 bp in length and the downstream ITR is approx. 141 bp in length). CBh promoter, artificially designed promoter composed of a human cytomegalovirus enhancer, a chicken β-actin promoter, and an intron derived partially from chicken β-actin and partially from minute virus of mice (mvm), with the sequence information shown in SEQ ID NO: 3. hOpticin sp, human Opticin protein secretory signal peptide sequence. Aflibercept opt3, codon-optimized aflibercept (without the secretory signal peptide sequence) coding sequence. hGH polyA, human growth hormone polyadenylation signal. Kan, Kanamycin resistance gene reading frame.
FIG. 3H: Schematic diagram for the structure of the pssAAV-CB7-hOpticin sp-Aflibercept opt3 vector.
   ITR, inverted terminal repeat (the upstream ITR is approx. 145 bp in length and the downstream ITR is approx. 141 bp in length). CB7 promoter, artificially designed promoter composed of a human cytomegalovirus enhancer, a chicken β-actin promoter, an intron and the like, with the sequence information shown in SEQ ID NO: 4. hOpticin sp, human Opticin protein secretory signal peptide sequence. Aflibercept opt3, codon-optimized aflibercept (without the secretory signal peptide sequence) coding sequence. hGH polyA, human growth hormone polyadenylation signal. Kan, Kanamycin resistance gene reading frame.

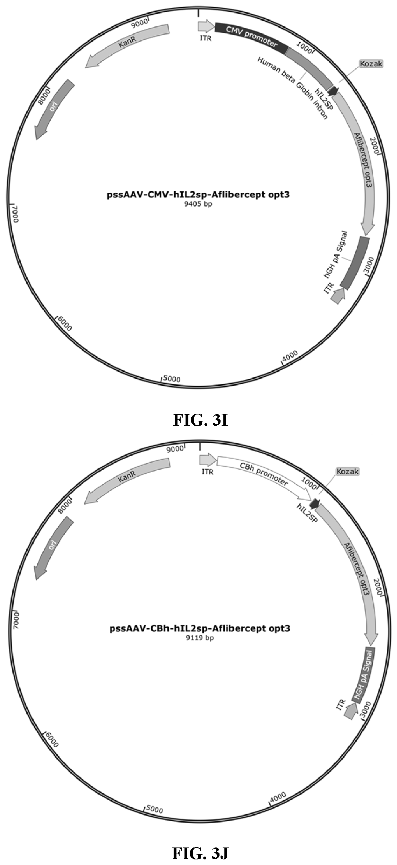

FIG. 3I: Schematic diagram for the structure of the pssAAV-CMV-hIL2sp-Aflibercept opt3 vector.
   ITR, inverted terminal repeat (the upstream ITR is approx. 145 bp in length and the downstream ITR is approx. 141 bp in length). CMV promoter, human cytomegalovirus early promoter. Human beta Globin intron. hIL2sp, human interleukin 2 protein secretory signal peptide sequence. Aflibercept opt3, codon-optimized aflibercept (without the secretory signal peptide sequence) coding sequence. hGH polyA, human growth hormone polyadenylation signal. Kan, Kanamycin resistance gene reading frame.
FIG. 3J: Schematic diagram for the structure of the pssAAV-CBh-hIL2sp-Aflibercept opt3 vector.
   ITR, inverted terminal repeat (the upstream ITR is approx. 145 bp in length and the downstream ITR is approx. 141 bp in length). CBh promoter, artificially designed promoter composed of a human cytomegalovirus enhancer, a chicken β-actin promoter, and an intron derived partially from chicken β-actin and partially from minute virus of mice (mvm), with the sequence information shown in SEQ ID NO: 3. hIL2sp, human interleukin 2 protein secretory signal peptide sequence. Aflibercept opt3, codon-optimized aflibercept (without the secretory signal peptide sequence) coding sequence. hGH polyA, human growth hormone polyadenylation signal. Kan, Kanamycin resistance gene reading frame.

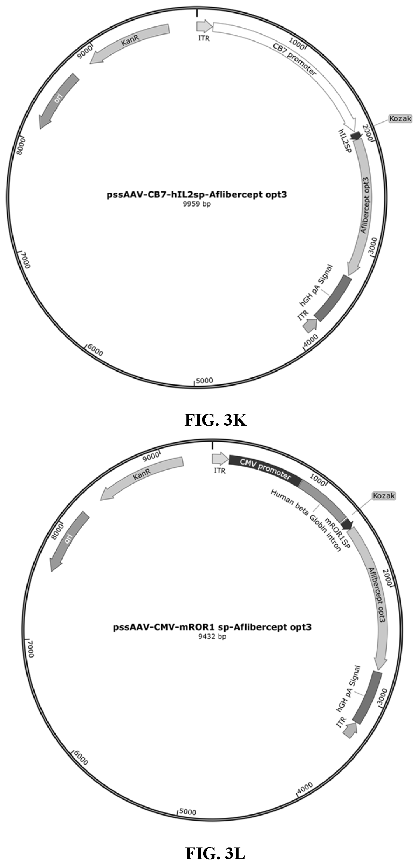

FIG. 3K: Schematic diagram for the structure of the pssAAV-CB7-hIL2sp-Aflibercept opt3 vector.
   ITR, inverted terminal repeat (the upstream ITR is approx. 145 bp in length and the downstream ITR is approx. 141 bp in length). CB7 promoter, artificially designed promoter composed of a human cytomegalovirus enhancer, a chicken β-actin promoter, an intron and the like, with the sequence information shown in SEQ ID NO: 4. hIL2sp, human interleukin 2 protein secretory signal peptide sequence. Aflibercept opt3, codon-optimized aflibercept (without the secretory signal peptide sequence) coding sequence. hGH polyA, human growth hormone polyadenylation signal. Kan, Kanamycin resistance gene reading frame.
FIG. 3L: Schematic diagram for the structure of the pssAAV-CMV-mROR1 sp-Aflibercept opt3 vector.
   ITR, inverted terminal repeat (the upstream ITR is approx. 145 bp in length and the downstream ITR is approx. 141 bp in length). CMV promoter, human cytomegalovirus early promoter. Human beta Globin intron. mROR1 sp, mouse ROR1 protein secretory signal peptide sequence. Aflibercept opt3, codon-optimized aflibercept (without the secretory signal peptide sequence) coding sequence. hGH polyA, human growth hormone polyadenylation signal. Kan, Kanamycin resistance gene reading frame.

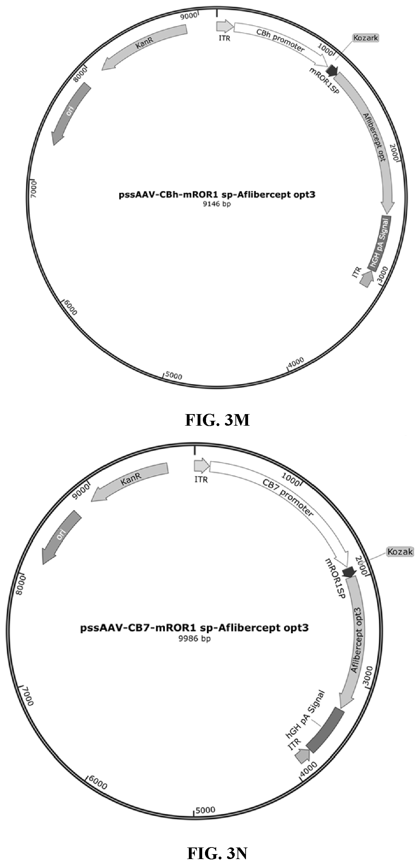

FIG. 3M: Schematic diagram for the structure of the pssAAV-CBh-mROR1 sp-Aflibercept opt3 vector.
   ITR, inverted terminal repeat (the upstream ITR is approx. 145 bp in length and the downstream ITR is approx. 141 bp in length). CBh promoter, artificially designed promoter composed of a human cytomegalovirus enhancer, a chicken β-actin promoter, and an intron derived partially from chicken β-actin and partially from minute virus of mice (mvm), with the sequence information shown in SEQ ID NO: 3. mROR1 sp, mouse ROR1 protein secretory signal peptide sequence. Aflibercept opt3, codon-optimized aflibercept (without the secretory signal peptide sequence) coding sequence. hGH polyA, human growth hormone polyadenylation signal. Kan, Kanamycin resistance gene reading frame.
FIG. 3N: Schematic diagram for the structure of the pssAAV-CB7-mROR1 sp-Aflibercept opt3 vector.
   ITR, inverted terminal repeat (the upstream ITR is approx. 145 bp in length and the downstream ITR is approx. 141 bp in length). CB7 promoter, artificially designed promoter composed of a human cytomegalovirus enhancer, a chicken β-actin promoter, an intron and the like, with the sequence information shown in SEQ ID NO: 4. hOpticin sp, mROR1 sp, mouse ROR1 protein secretory signal peptide sequence. Aflibercept opt3, codon-optimized aflibercept (without the secretory signal peptide sequence) coding sequence. hGH polyA, human growth hormone polyadenylation signal. Kan, Kanamycin resistance gene reading frame.

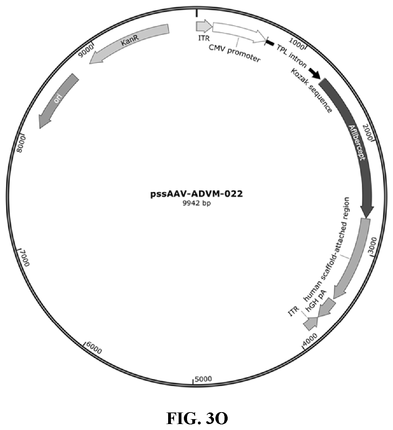

FIG. 3O: Schematic diagram for the structure of the pssAAV-ADVM-022 vector.
   ITR, inverted terminal repeat (the upstream ITR is approx. 145 bp in length and the downstream ITR is approx. 141 bp in length). CMV promoter, human cytomegalovirus early promoter. TPL intron, triple leader sequence. Aflibercept, aflibercept coding sequence. human scaffold-attached region, scaffold attachment region. hGH polyA, human growth hormone polyadenylation signal. Kan, Kanamycin resistance gene reading frame.

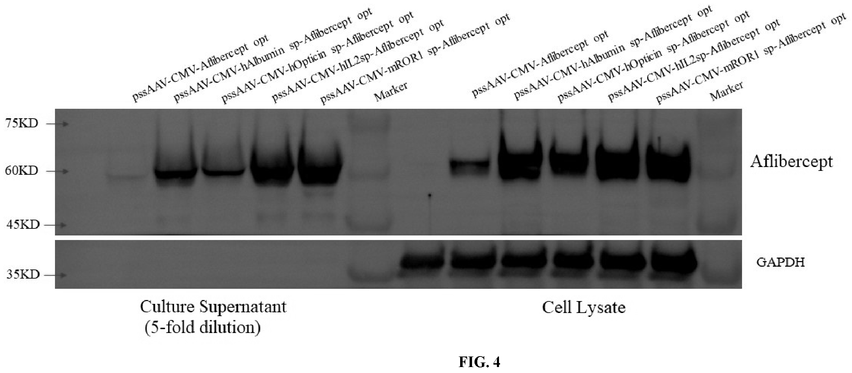

FIG. 4: Effects of different secretory signal peptide sequences on aflibercept secretion.
   The HEK 293T cells are seeded in a 6-well plate. When growing to 70% to 80% confluency, the cells are transfected with 2 µg/well of pssAAV-CMV-Aflibercept opt3, pssAAV-CMV-hAlbumin sp-Aflibercept opt3, pssAAV-CMV-hOpticin sp-Aflibercept opt3, pssAAV-CMV-hIL2sp-Aflibercept opt3 or pssAAV-CMV-mROR1 sp-Aflibercept opt3 using Lipo3000. At 48 h after transfection, the cells and supernatant are harvested. The cell supernatant is diluted 5-fold with sterile PBS. The cells are lysed using Ripa lysate (Beyotime, Beijing), and then the total protein is extracted. The protein concentrations are determined. Subsequently, the total protein concentrations of the cells are normalized. Western blotting is employed to detect aflibercept in both the supernatant and the total cell protein.

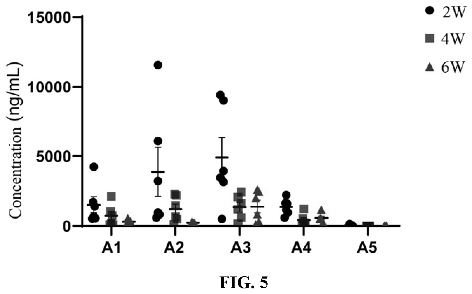

FIG. 5: Expression of different rAAV IVT15-hIL2sp-Aflibercept opt3 in mouse aqueous humor.
   rAAV IVT15-CMV-hIL2sp-Aflibercept opt3, rAAV IVT15-CBh-hIL2sp-Aflibercept opt3, and rAAV IVT15-CB7-hIL2sp-Aflibercept opt3 are each intravitreally injected into both eyes of wild-type male c57 mice (aged 6 to 8 weeks) at a dose of 6 × 10⁹ vg/eye (viral genome, vg). Nine mice are assigned to each virus group. An additional group of mice is included and injected with PBS as a control. These four groups of mice are designated as Group A1: injected with rAAV IVT15-CMV-hIL2sp-Aflibercept opt3; Group A2: injected with rAAV IVT15-CBh-hIL2sp-Aflibercept opt3; Group A3: injected with rAAV IVT15-CB7-hIL2sp-Aflibercept opt3; Group A4: injected with rAAV2 7m8-ADVM-022; and Group A5: injected with PBS, separately. At 2, 4, and 6 weeks after injection, three mice are randomly selected from each group and euthanized. Eyeballs are enucleated from both eyes and the aqueous humor is collected. Subsequently, the aflibercept expression is assayed by Elisa.

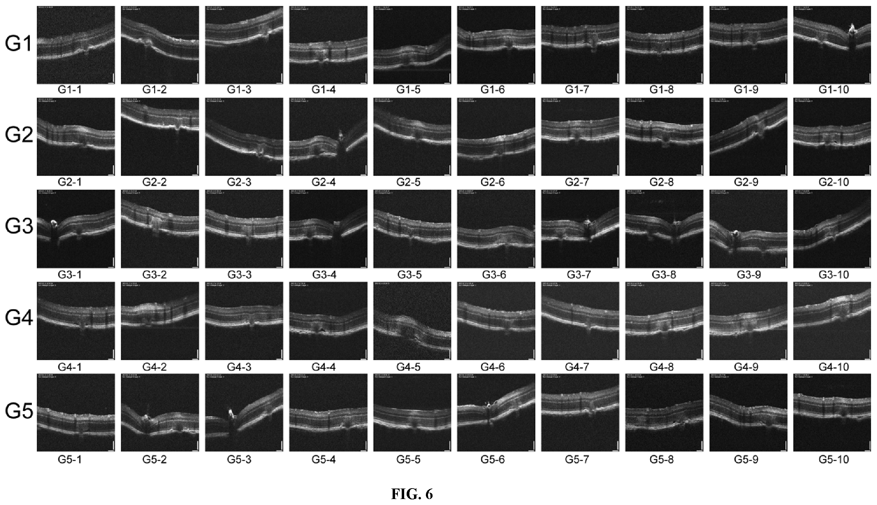

FIG. 6: Construction of mouse choroidal neovascularization (CNV) models.
   rAAV IVT15-CMV-hIL2sp-Aflibercept opt3, rAAV IVT15-CB7-hIL2sp-Aflibercept opt3, and rAAV2 7m8-ADVM-022 are each intravitreally injected into a single eye (right eye) of wild-type male c57 mice (aged 6 to 8 weeks) at a dose of 6 × 10⁹ vg/eye. Ten mice are assigned to each virus group. At the same time, two groups of mice are further introduced, one of which is injected with 1 µL of PBS, whereas the other group is left untreated temporarily. Laser-induced CNV modeling is performed on the right eye of all mice. Three days after modeling, the mice in the untreated group are intravitreally injected with 2.5 µg of Eylea (aflibercept, purchased from Bayer (China) Limited, Beijing, China) into the modeled eye. The four groups of mice are designated as Group G1: injected with rAAV IVT15-CMV-hIL2sp-Aflibercept opt3; Group G2: injected with rAAV IVT15-CB7-hIL2sp-Aflibercept opt3; Group G3: injected with rAAV2 7m8-ADVM-022; Group G4: injected with PBS; and Group G5: injected with Eylea, separately. After modeling, all the mice are subjected to spectral domain optical coherence tomography (SD-OCT) examination.
FIG. 7A: Vascular leakage at Day 7 after modeling in CNV mice treated with various viruses and other drugs.
   For the mice in Groups G1 to G5 (FIG. 6), the modeled eye is subjected to the fluorescein fundus angiography (FFA) at Day 7 after CNV modeling.
FIG. 7B: Vascular leakage at Day 14 after modeling in CNV mice treated with various viruses and other drugs.
   For the mice in Groups G1 to G5 (FIG. 6), the modeled eye is subjected to the FFA at Day 14 after CNV modeling.
FIG. 8: Comparison of vascular leakage at Day 14 vs. Day 7 after modeling in CNV mice treated with various viruses and other drugs.
   For the mice in Groups G1 to G5 (FIG. 6), FFA images photographed at Day 7 and Day 14 after CNV modeling are processed. The fluorescent area is quantified using Image-Pro Plus 6.0. The mean fluorescent area is calculated for each group of mice.
FIG. 9: Neovascular leakage scores of CNV mice treated with various viruses and other drugs.
   For the mice in Groups G1 to G5 (FIG. 6), the area and intensity of fluorescent spots are scored at Day 14 after CNV modeling based on the FFA images. Three technicians with expertise in ophthalmology are invited to score the neovascular leakage (fluorescent spot area and intensity) in a randomized, double-blind manner. The average score is calculated for each group of mice.

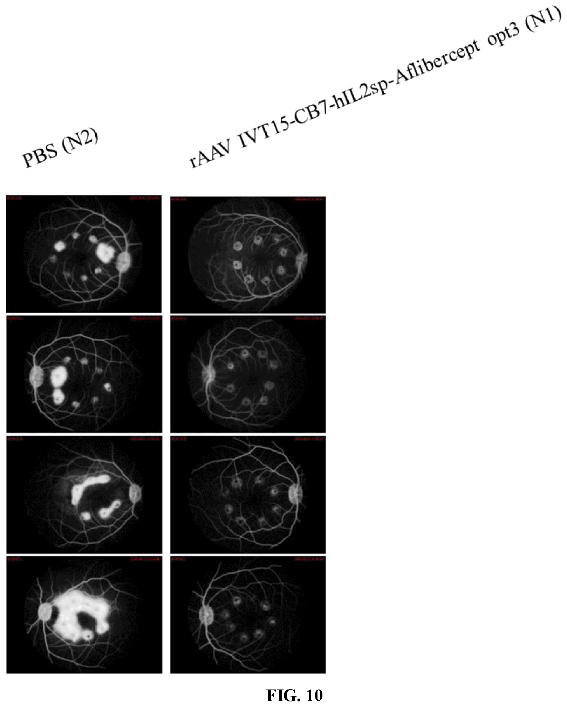

FIG. 10: Vascular leakage at Day 28 after CNV modeling in cynomolgus monkeys treated with virus and control drug.
   rAAV IVT15-CB7-hIL2sp-Aflibercept opt3 is intravitreally injected into both eyes of cynomolgus monkeys (aged 3 to 5 years) at a dose of 2.5 × 10¹¹ vg/eye. Each virus is injected into 2 cynomolgus monkeys. Meanwhile, a group of (two) cynomolgus monkeys is further included and injected with 50 µL of PBS. At Day 28 after injection, both eyes of all the cynomolgus monkeys are subjected to laser-induced CNV modeling. The two groups of cynomolgus monkeys are designated as Group N1: injected with rAAV IVT15-CB7-hIL2sp-Aflibercept opt3; and Group N2: injected with PBS. At Day 14 and Day 28 after modeling, both eyes are subjected to the fluorescein fundus angiography (FFA). The detection results at Day 28 are as shown in FIG. 10.

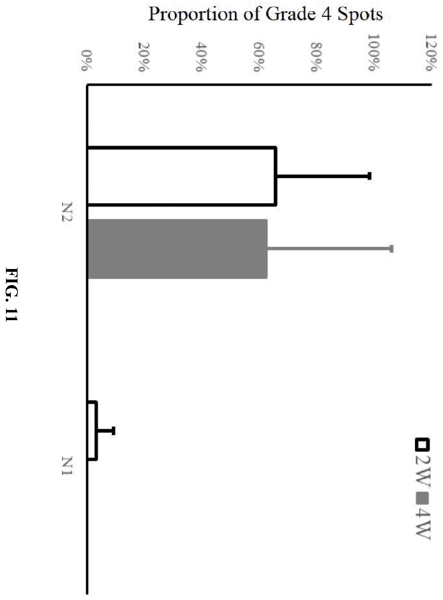

FIG. 11: Proportion of grade 4 fluorescent spots at Day 14 (2w) and Day 28 (4w) after CNV modeling in cynomolgus monkeys treated with virus and control drug.
   At Day 14 and Day 28 after CNV modeling in the cynomolgus monkeys in Groups N1 and N2 (FIG. 10), the area and intensity of fluorescent spots are scored based on the FFA images. Three technicians with expertise in ophthalmology are invited to score the neovascular leakage (fluorescent spot area and intensity) in a randomized, double-blind manner. The proportion of grade 4 fluorescent spots is as shown in FIG. 11.

### DETAILED DESCRIPTION

To render the present disclosure more understandable, some technical and scientific terms are specifically defined below. Unless otherwise explicitly defined herein, all of the other technical and scientific terms used herein have the same meanings as typically understood by one of ordinary skill in the art to which the present disclosure belongs.

According to the present disclosure, the numerical range represented by "numerical value A to numerical value B" refers to the range including the endpoint values A and B.

According to the present disclosure, the term "basically" or "substantially" means that the standard deviation from the theoretical model or theoretical data is within a range of 5%, preferably 3%, more preferably 1%.

According to the present disclosure, the term "may" involves both the meaning of doing something and the meaning of not doing something.

According to the present disclosure, the term "optional" or "optionally" means that the event or case described below may or may not occur, and this description includes the case where this event occurs and the case where this event does not occur.

According to the present disclosure, the terms "comprising" and "having" and any variations thereof are intended to encompass non-exclusive inclusions. For example, a process, method, apparatus, product or device including a series of steps or modules is not confined to including the listed steps or modules, but may optionally include those unlisted, or may optionally include other steps or modules innate in the process, method, product or device.

According to the present disclosure, the term "a plurality of" referred to in the present disclosure means two or more. The term "and/or" describes an association between the associated objects, and represents three possible relationships. For example, A and/or B may represent the following three cases: A exists alone, both A and B exist, and B exists alone. The character "/" typically denotes an alternative (*i.e.* "or") relationship between the associated objects it connects.

According to the present disclosure, phrases such as "some specific/preferred embodiments", "other specific/preferred embodiments", and "embodiments" referred to herein mean that particular elements (for example, features, structures, properties and/or characteristics) described in relation to this embodiment are included in at least one of the embodiments described herein, and may or may not exist in other embodiments. Additionally, it should be appreciated that the elements may be combined in any suitable manner into various embodiments.

According to the present disclosure, "Vg" refers to Viral Genome and is equivalent to Genome Copy.

According to the present disclosure, the terms "polypeptide", "protein", and "peptide" are used interchangeably herein to refer to a polymeric form of amino acids of any length, which may include coded and non-coded amino acids, chemically or biochemically modified or derived amino acids, and polypeptides having similar peptide backbones.

According to the present disclosure, the terms "nucleic acid molecule", "polynucleotide", "polynucleic acid", and "nucleic acid" are used interchangeably to refer to a polymeric form of nucleotides of any length, regardless of deoxyribonucleotides or ribonucleotides, or analogs thereof.

According to the present disclosure, the term "isolated" means altered or removed from its natural state. For example, a nucleic acid or peptide that naturally occurs in a living animal is not considered "isolated", but the same nucleic acid or peptide becomes "isolated" if it is partially or completely separated from the materials that co-exist with it in its natural state. An isolated nucleic acid or protein may exist in a substantially purified form or may exist in a non-natural environment, e.g., in a host cell.

According to the present disclosure, the term "upstream" and "downstream" are relative terms that define the linear positions of at least two elements in a nucleic acid molecule (either single-stranded or double-stranded) oriented in the 5' to 3' direction.

According to the present disclosure, the term "fusion protein" or "fusion polypeptide" refers to a hybrid polypeptide containing a protein domain derived from at least two different proteins. Any protein provided herein may be produced by any method known in the related art. For example, the proteins provided herein may be produced via recombinant protein expression and purification, which is particularly suitable for fusion proteins containing a peptide linker. Methods for recombinant protein expression and purification are well-known and include those described in: Green and Sambrook, Molecular Cloning: A Laboratory Manual (4th ed., Cold Spring Harbor Laboratory Press, Cold Spring Harbor, N.Y. (2012)), the entire contents of which are incorporated herein by reference.

According to the present disclosure, the term "amino acid" may include natural amino acids, unnatural amino acids, amino acid analogs, and all of D and L stereoisomers thereof. According to the present disclosure, the three-letter and single-letter codes for amino acids used herein are as described in J. biol. chem, 243, p3558 (1968). In the present disclosure, the amino acids as well as their abbreviations and English codes are as follows: histidine (His, H); serine (Ser, S); glutamic acid (Glu, E); glutamine (Gln, Q); glycine (Gly, G); threonine (Thr, T); phenylalanine (Phe, F); aspartic acid (Asp, D); tyrosine (Tyr, Y); leucine (Leu, L); isoleucine (Ile, I); arginine (Arg, R); alanine (Ala, A); valine (Val, V); tryptophan (Trp, W); methionine (Met, M); asparagine (Asn, N); cysteine (Cys, C); lysine (Lys, K); proline (Pro, P).

According to the present disclosure, the "addition" of an amino acid means that an amino acid is added at the C-terminus or N-terminus of an amino acid sequence. According to the present disclosure, the "deletion" of an amino acid means that 1, 2 or 3 or more amino acids can be deleted from an amino acid sequence. According to the present disclosure, the "insertion" of an amino acid means that an amino acid residue is inserted at an appropriate position in an amino acid sequence, and the inserted amino acid residues may also be all or partially adjacent to one another, or the inserted amino acids are not adjacent to one another. According to the present disclosure, the "substitution" of an amino acid means that an amino acid residue at a position in an amino acid sequence is substituted with an additional amino acid residue, where the "substitution" may be a conservative substitution of an amino acid.

According to the present disclosure, the term "conservative modification", "conservative substitution" or "conservative replacement" refers to replacement of an amino acid in a protein with an additional amino acid having similar characteristics (e.g. charge, size of side chain, hydrophobicity/hydrophilicity, backbone conformation, and rigidity), such that the protein can be changed frequently without altering the biological activity of the protein. A person skilled in the art knows that in general, replacement of a single amino acid in the non-essential region of a polypeptide does not substantially alter the biological activity (see, for example, Watson et al. (1987) Molecular Biology of the Gene, The Benjamin/Cummings Pub. Co., page 224, (4th ed.)). Furthermore, replacement with structurally or functionally similar amino acids is unlikely to disrupt the biological activity. Exemplary conservative substitutions are set forth in the following "Exemplary Amino Acid Conservative Substitutions":

Exemplary Amino Acid Conservative Substitutions

| Original Residue | Conservative Substitution |
|---|---|
| Ala (A) | Gly; Ser |
| Arg (R) | Lys; His |
| Asn (N) | Gln; His; Asp |
| Asp (D) | Glu; Asn |
| Cys (C) | Ser; Ala; Val |
| Gln (Q) | Asn; Glu |
| Glu (E) | Asp; Gln |
| Gly (G) | Ala |
| His (H) | Asn; Gln |
| Ile (I) | Leu; Val |
| Leu (L) | Ile; Val |
| Lys (K) | Arg; His |
| Met (M) | Leu; Ile; Tyr |
| Phe (F) | Tyr; Met; Leu |
| Pro (P) | Ala |
| Ser (S) | Thr |
| Thr (T) | Ser |
| Trp (W) | Tyr; Phe |
| Tyr (Y) | Trp; Phe |
| Val (V) | Ile; Leu |

According to the present disclosure, "moderate to very high stringency conditions" include "moderate stringency conditions", "moderate-high stringency conditions", "high stringency conditions" or "very high stringency conditions", which describe the conditions for hybridization and washing of nucleic acids. Guidance for conducting hybridization reactions can be found in Current Protocols in Molecular Biology, John Wiley & Sons, N.Y. (1989), 6.3.1-6.3.6, which is incorporated herein by reference. This literature describes both aqueous and non-aqueous processes and either can be used. For example, specific hybridization conditions are as follows: (1) the low stringency hybridization conditions refer to hybridizing in 6× sodium chloride/sodium citrate (SSC) at about 45°C, and then washing twice in 0.2× SSC and 0.1% SDS at 50°C or higher (the washing temperature can be raised to 55°C in the case of the low stringency conditions); (2) the moderate stringency hybridization conditions refer to hybridizing in 6× SSC at about 45°C, and then washing one or more times in 0.2× SSC and 0.1% SDS at 60°C; (3) the high stringency hybridization conditions refer to hybridizing in 6× SSC at about 45°C, and then washing one or more times in 0.2× SSC and 0.1% SDS at 65°C, which is preferred; and (4) the very high stringency hybridization conditions refer to hybridizing in 0.5 M sodium phosphate and 7% SDS at 65°C, and then washing one or more times in 0.2× SSC and 1% SDS at 65°C.

According to the present disclosure, the term "identity" refers to the sequence similarity between two polynucleotide sequences or between two polypeptides. When the positions in two compared sequences are all occupied by the same base or amino acid monomer subunit, for example, if each of the positions in two DNA molecules is occupied by adenine, the molecules are homologous at this position. The percent identity between two sequences is a function of the number of matching or homologous positions shared by the two sequences / the number of positions to be compared × 100%. For example, in the case of optimal alignment of sequences, if six out of ten positions in two sequences are matched or homologous, the two sequences are 60% homologous. In general, comparison is made between two sequences when aligned to obtain the maximum percent identity.

According to the present disclosure, when applied to an animal, human, test subject, cell, tissue, organ or biological fluid, the terms "administration", "giving", and "treating" refer to contact of an exogenous drug, therapeutic agent, diagnostic agent, or composition with the animal, human, subject, cell, tissue, organ or biological fluid. The terms "administration", "giving", and "treating" may refer to, e.g., therapeutic, pharmacokinetics, diagnostic, research, and experimental methods. Treating of cells includes contact of a reagent with the cells and contact of a reagent with a fluid, where the fluid is in contact with the cells. The terms "administration", "giving", and "treating" also means *in vitro* and *ex vivo* treatment of, for example, a cell, by a reagent, a diagnostic agent, a binding composition or an additional cell. When applied to humans, veterinary, or research subjects, "treating" refers to therapeutic, prophylactic or precautionary measures, research and diagnostic applications.

According to the present disclosure, the term "treatment" means administering a therapeutic agent for internal or external use, such as a therapeutic agent comprising any of the antibodies described herein, to a subject having one or more symptoms of a disease, on which the therapeutic agent is known to have a therapeutic effect. Generally, the therapeutic agent is administered in an amount effective to alleviate one or more symptoms of the disease in the subject or population being treated by either inducing regression of such symptoms or suppressing progression of such symptoms to any clinically measurable degree. The amount of a therapeutic agent effective to alleviate any specific disease symptom (also referred to as a "therapeutically effective amount") may vary depending upon multiple factors such as the disease state, age, and body weight of the patient, and the ability of the drug to produce the desired therapeutic effect in the patient. Any clinical test method typically used by a physician or other health care professional to assess the severity or progression of a disease symptom may be adopted to evaluate whether this symptom has been alleviated.

According to the present disclosure, the term "prevention" refers to prophylactic treatment on a subject who does not have a disease now or in the past but is at risk of developing a disease or who had a disease in the past and does not have a disease now but is at risk of recurrence of the disease. In some embodiments, the subject runs a higher risk of developing a disease or a higher risk of recurrence of the disease as compared to the members at the average healthy level in the subject population.

According to the present disclosure, an "effective amount" includes an amount sufficient to ameliorate or prevent the symptoms of a medical condition or the condition. An effective amount also means an amount sufficient to allow for or facilitate diagnosis. An effective amount for a particular patient or veterinary subject may vary depending on factors such as the condition to be treated, the patient's overall health, the method, route, and dose of administration, and the severity of side effects. An effective amount may be the maximum dose or dosage regimen that avoids significant side effects or toxic effects.

According to the present disclosure, the "therapeutically effective amount" is an amount sufficient to provide a therapeutic benefit in the course of treating a condition or sufficient to delay or minimize one or more symptoms associated with the condition. A therapeutically effective amount refers to an amount of a therapeutic agent alone or in combination with other therapies, which provides therapeutic benefits in the course of treating a condition. The term "therapeutically effective amount" may include an amount that improves overall therapy; reduces or avoids symptoms, signs, or causes of a condition; and/or enhances the therapeutic efficacy of an additional therapeutic agent.

According to the present disclosure, the "prophylactically effective amount" is an amount sufficient to prevent a condition or one or more symptoms associated with the condition or to prevent its recurrence. A prophylactically effective amount refers to an amount of a therapeutic agent alone or in combination with other drugs, which provides therapeutic benefits in the course of preventing a condition. The term "prophylactically effective amount" may include an amount that improves overall prevention or enhances the prophylactic efficacy of an additional prophylactic agent.

According to the present disclosure, the term "subject" refers to a human (*i.e.,* a male or female of any age, *e.g.,* a pediatric subject (*e.g.,* an infant, child, or adolescent) or an adult subject (*e.g.,* a young, middle-aged, or elderly person)) or a non-human animal. In some embodiments, the non-human animal is a mammal (*e.g.,* a primate (such as *Macaca fascicularis* or *Macaca mulatta),* a commercially relevant mammal (such as cow, pig, horse, sheep, goat, cat, or dog), or a bird. The non-human animal may be male or female at any stage of development. The non-human animal may be a transgenic animal or a genetically engineered animal.

According to the present disclosure, the term "vector" refers to a polymer or an association of polymers that includes or is associated with a polynucleotide and can be used to mediate the delivery of the polynucleotide to a cell. Illustrative vectors include, for example, plasmids, viral vectors (i.e., viruses, such as adeno-associated virus), liposomes, and other gene delivery vehicles.

According to the present disclosure, the term "expression vector" encompasses vectors that include a gene expression cassette encoding the gene product of interest, such as plasmids, minicircles, viral vectors, and liposomes, and are used to deliver the gene expression cassette to a target cell as desired.

According to the present disclosure, the term "AAV" is an abbreviation for adeno-associated virus and may be used to refer to the virus itself or a derivative thereof. This term covers all subtypes as well as the naturally occurring and recombinant forms, unless otherwise required. The term "AAV" includes AAV type 1 (AAV-1), AAV type 2 (AAV-2), AAV type 3 (AAV-3), AAV type 4 (AAV-4), AAV type 5 (AAV-5), AAV type 6 (AAV-6), AAV type 7 (AAV-7), AAV type 8 (AAV-8), AAV type 9 (AAV-9), avian AAV, bovine AAV, canine AAV, equine AAV, primate AAV, non-primate AAV, and ovine AAV. The "primate AAV" refers to an AAV that infects a primate; the "non-primate AAV" refers to an AAV that infects a non-primate mammal; the "bovine AAV" refers to an AAV that infects a bovine mammal, and so forth.

According to the present disclosure, the term "AAV virus" or "AAV viral particle" or "rAAV vector particle" refers to a viral particle composed of at least one AAV capsid protein (typically consisting of all capsid proteins of the wild-type AAV) and an encapsidated polynucleotide. If the particle includes a heterologous polynucleotide (i.e., a polynucleotide other than the wild-type AAV genome, such as a transgene to be delivered to a mammalian cell), it is generally referred to as a recombinant AAV vector or rAAV. Typically, heterologous polynucleotides are flanked by an AAV inverted terminal repeat (ITR).

According to the present disclosure, the term "replication-deficient" in relation to the AAV viral vector of the present disclosure means that the AAV vector cannot replicate and package its genome independently. For example, when the cells of a subject are infected with rAAV viral particles, a heterologous gene is expressed in the infected cells, but rAAV cannot further replicate due to the fact that the infected cells lack the AAV *rep* and *cap* genes and the helper function gene(s), rAAV cannot replicate further.

According to the present disclosure, the term "AAV variant" or "AAV mutant" refers to a viral particle composed of a variant AAV capsid protein that includes at least one amino acid difference (e.g., amino acid substitution, amino acid insertion, or amino acid deletion) relative to a corresponding parental AAV capsid protein and confers increased infectivity to retinal cells as compared to the AAV viral particles comprising the corresponding parental AAV capsid protein, where the AAV capsid protein does not include the amino acid sequence present in the naturally occurring AAV capsid protein. The polynucleotide expression cassette of the present disclosure can be packaged in the variant AAV particles to facilitate delivery of the cassette to a particular cell type (such as retinal cells) in the target tissue.

According to the present disclosure, the term "gene", "coding sequence" or "coding gene" refers to a nucleotide sequence encoding an *in vitro* or *in vivo* gene product. The term "transgene" refers to a coding sequence or gene delivered into a cell via a vector. A coding sequence or gene can encode a peptide or polypeptide molecule.

According to the present disclosure, the term "operably linked" refers to juxtaposition of genetic elements (e.g., a promoter, an enhancer, a termination signal sequence, or a polyadenylation sequence), where the elements are in a relationship that allows them to operate in the intended manner. For example, if a promoter helps initiate transcription of a coding sequence, the promoter is operably linked to the coding region. As long as this functional relationship is maintained, intervening residues may exist between the promoter and the coding region.

According to the present disclosure, the term "heterologous" refers to an entity having a genotype different from that of the remainder of an entity with which it is compared. For example, a polynucleotide introduced into a plasmid or vector derived from a different species by a genetic engineering technique is a heterologous polynucleotide. As another example, a promoter that is removed from its natural coding sequence and operably linked to a coding sequence to which a native linkage is not found is a heterologous promoter. Therefore, for example, an rAAV containing a heterologous nucleic acid encoding a heterologous gene product is an rAAV containing a nucleic acid that is typically not contained in a naturally occurring wild-type AAV, and the encoded heterologous gene product is a gene product that is typically not encoded by a naturally occurring wild-type AAV.

According to the present disclosure, the term "endogenous" in relation to a nucleotide molecule or gene product refers to a nucleic acid sequence (e.g., a gene or a genetic element) or a gene product (e.g., RNA or protein) naturally occurring in or associated with a host virus or cell.

### Detailed Description of the Invention

### <Fusion Polypeptide>

In some aspects of the present disclosure, there is provided a fusion polypeptide, comprising a secretory signal peptide and an aflibercept polypeptide, wherein the secretory signal peptide is any one selected from a human albumin secretory signal peptide, a human Opticin protein secretory signal peptide, a human interleukin-2 secretory signal peptide, and a mouse receptor tyrosine kinase-like orphan receptor 1 secretory signal peptide.

According to the present disclosure, the term "signal peptide", "signal sequence", "signal peptide sequence" or "secretory signal peptide" refers to such a short peptide that, when fused to a protein of interest (such as aflibercept), is capable of promoting secretion of the protein of interest expressed by a cell onto the cell membrane or outside the cell. The signal peptide is typically located at the N-terminus of a protein of interest. Exemplarily, the secretory signal peptide is selected from a hemagglutinin signal sequence, a human insulin signal sequence, a human albumin secretory signal peptide sequence, a human Opticin protein secretory signal peptide sequence, a human interleukin-2 secretory signal peptide sequence, a mouse receptor tyrosine kinase-like orphan receptor 1 secretory signal peptide sequence, etc. However, different secretory signal peptides are used for different proteins of interest, and their effects in different gene expression cassettes and gene delivery vectors are unknown.

In some optional embodiments, the amino acid sequence of the human albumin secretory signal peptide comprises:
MKWVTFISLLFLFSSAYS (SEQ ID NO: 44);

the amino acid sequence of the human Opticin protein secretory signal peptide comprises:
MRLLAFLSLLALVLQETGT (SEQ ID NO: 45);

the amino acid sequence of the human interleukin-2 secretory signal peptide comprises:
MYRMQLLSCIALSLALVTNS (SEQ ID NO: 46);

the amino acid sequence of the mouse receptor tyrosine kinase-like orphan receptor 1 secretory signal peptide is:
MHRPRRRGTRPPPLALLAALLLAARGADA (SEQ ID NO: 47).

According to the present disclosure, aflibercept is a 115 kDa fusion protein, which can be glycosylated. Aflibercept comprises an IgG backbone fused to the extracellular VEGF receptor sequences of human VEGFR-1 and VEGFR-2, and functions similarly to a soluble decoy receptor by binding to VEGF-A with higher affinity than its native or endogenous receptor. See, for example, Stewart MW. Aflibercept (VEGF Trap-eye): the newest anti-VEGF drug. Br. J. Ophthalmol. 2012 Sep; 96(9):1157-8. The high affinity of aflibercept for VEGF interferes with or disrupts subsequent binding and activation of native or endogenous VEGF receptors. The reduced VEGF activity can lead to decreased angiogenesis and decreased vascular permeability. The inhibition of aflibercept against the placental growth factor PIGF and VEGF-B may also contribute to the treatment of angiogenic conditions. PIGF has been associated with angiogenesis and can be elevated in various conditions, such as wet AMD. VEGF-B overexpression can be associated with disruption of the blood-retinal barrier and retinal angiogenesis. Consequently, inhibition of VEGF-A, VEGF-B, and PIGF can all contribute to the efficacy of aflibercept.

In some specific embodiments, the amino acid sequence of the aflibercept polypeptide comprises:

SDTGRPFVEMYSEIPEIIHMTEGRELVIPCRVTSPNITVTLKKFPLDTLIPDGKRIIWD
SRKGFIISNATYKEIGLLTCEATVNGHLYKTNYLTHRQTNTIIDVVLSPSHGIELSVGEKL
VLNCTARTELNVGIDFNWEYPSSKHQHKKLVNRDLKTQSGSEMKKFLSTLTIDGVTRS
DQGLYTCAASSGLMTKKNSTFVRVHEKDKTHTCPPCPAPELLGGPSVFLFPPKPKDTL
MISRTPEVTCVVVDVSHEDPEVKFNWYVDGVEVHNAKTKPREEQYNSTYRVVSVLTV
LHQDWLNGKEYKCKVSNKALPAPIEKTISKAKGQPREPQVYTLPPSRDELTKNQVSLT
CLVKGFYPSDIAVEWESNGQPENNYKTTPPVLDSDGSFFLYSKLTVDKSRWQQGNVFS
CSVMHEALHNHYTQKSLSLSPGK (SEQ ID NO: 48)

In some specific embodiments, the amino acid sequence of the fusion polypeptide comprises any one selected from the following sequences:
(i) an amino acid sequence as set forth in any one of SEQ ID NO: 1, 30, 32 or 34;
(ii) an amino acid sequence having at least 80%, 82%, 85%, 87%, 90%, 92%, 95%, 96%, 97%, 98% or 99% identity to the amino acid sequence as set forth in any one of SEQ ID NO: 1, 30, 32 or 34, and retaining a function of the amino acid sequence as set forth in any one of SEQ ID NO: 1, 30, 32 or 34;
(iii) an amino acid sequence obtained by adding, substituting, deleting or inserting one or more amino acid residues in the amino acid sequence as set forth in any one of SEQ ID NO: 1, 30, 32 or 34, and retaining a function of the amino acid sequence as set forth in any one of SEQ ID NO: 1, 30, 32 or 34; or
(iv) an amino acid sequence encoded by a nucleotide sequence that hybridizes under stringency conditions with a polynucleotide sequence encoding the amino acid sequence as set forth in any one of SEQ ID NO: 1, 30, 32 or 34, and the amino acid sequence retaining a function of the amino acid sequence as set forth in any one of SEQ ID NO: 1, 30, 32 or 34, wherein the stringency conditions are moderate stringency conditions, moderate-high stringency conditions, high stringency conditions, or very high stringency conditions.

Exemplarily, the amino acid sequence of a fusion polypeptide containing a human interleukin-2 secretory signal peptide and an aflibercept polypeptide (referred to as hIL2sp-Aflibercept opt3) (SEQ ID NO: 1) is: 
MYRMQLLSCIALSLALVTNSSDTGRPFVEMYSEIPEIIHMTEGRELVIPCRVTSPNIT
VTLKKFPLDTLIPDGKRIIWDSRKGFIISNATYKEIGLLTCEATVNGHLYKTNYLTHRQT
NTIIDVVLSPSHGIELSVGEKLVLNCTARTELNVGIDFNWEYPSSKHQHKKLVNRDLKT
QSGSEMKKFLSTLTIDGVTRSDQGLYTCAASSGLMTKKNSTFVRVHEKDKTHTCPPCP
APELLGGPSVFLFPPKPKDTLMISRTPEVTCVVVDVSHEDPEVKFNWYVDGVEVHNAK
 
TKPREEQYNSTYRVVSVLTVLHQDWLNGKEYKCKVSNKALPAPIEKTISKAKGQPREP
QVYTLPPSRDELTKNQVSLTCLVKGFYPSDIAVEWESNGQPENNYKTTPPVLDSDGSFF
LYSKLTVDKSRWQQGNVFSCSVMHEALHNHYTQKSLSLSPGK*
 where the single underlined portion is the human interleukin-2 secretory signal peptide (also referred to as hlL2sp in the present specification); and the double underlined portion is aflibercept.

### <Polynucleotide>

In some aspects of the present disclosure, there is provided an isolated polynucleotide encoding the fusion polypeptide provided in the present disclosure.

In some embodiments of the present disclosure, the polynucleotide encoding the fusion polypeptide provided in the present disclosure is modified or "codon-optimized" to enhance expression by replacing infrequently represented codons with more frequently represented codons. The coding sequence is a portion of the mRNA sequence that encodes the amino acids for translation. During translation, each of the 61 trinucleotide codons is translated into one of the 20 amino acids, resulting in degeneracy or redundancy in the genetic code. However, different cell types and different animal species will utilize tRNAs (each carrying an anticodon) that encode the same amino acid at different frequencies. When a gene sequence contains a codon that is infrequently represented by the corresponding tRNA, the ribosomal translation mechanism may be slowed, thus hindering efficient translation. Expression can be improved by species-specific "codon optimization", in which the coding sequence is altered to encode the same protein sequence while utilizing codons that are highly represented and/or utilized by highly represented human proteins (Cid-Arregui et al., 2003; Journal of Virology, 77:4928).

In some preferred embodiments of the present disclosure, the polynucleotide comprises a nucleotide sequence as set forth in any one of SEQ ID NO: 2, 29, 31 or 33, or a nucleotide sequence having at least 80%, 82%, 85%, 87%, 90%, 92%, 95%, 96%, 97%, 98% or 99% identity to the nucleotide sequence as set forth in any one of SEQ ID NO: 2, 29, 31 or 33.

Exemplarily, the polynucleotide encoding the fusion polypeptide containing a human interleukin-2 secretory signal peptide and an aflibercept polypeptide provided in the present disclosure (SEQ ID NO: 2) is:

5'ATGTACAGAATGCAGCTGCTGAGCTGCATAGCCCTGTCTCTGGCCCTGGTGA
CCAACAGCTCTGACACAGGCAGACCCTTTGTGGAAATGTACTCTGAGATCCCTGAG
ATCATCCACATGACAGAGGGCAGAGAGCTGGTCATCCCCTGCAGAGTGACAAGCCC

CAACATCACAGTGACCCTGAAGAAGTTCCCTCTGGACACACTGATCCCTGATGGCA
AGAGAATCATCTGGGACAGCAGAAAGGGCTTCATCATCAGCAATGCCACCTACAAA
GAGATTGGCCTGCTGACCTGTGAAGCCACAGTGAATGGCCACCTGTACAAGACCAA
CTACCTGACACACAGACAGACCAACACCATCATTGATGTGGTGCTGAGCCCCAGCC
ATGGCATTGAGCTGTCTGTGGGAGAGAAGCTGGTGCTGAACTGCACAGCCAGAAC
AGAGCTGAATGTGGGCATTGACTTCAACTGGGAGTACCCCAGCAGCAAGCACCAG
CACAAGAAACTGGTCAACAGGGACCTGAAAACCCAGAGTGGCTCTGAGATGAAGA
AATTCCTGAGCACCCTGACCATTGATGGGGTCACCAGATCTGACCAGGGCCTGTAC
ACATGTGCTGCCAGCTCTGGACTGATGACCAAGAAAAACAGCACCTTTGTCAGAGT
GCATGAGAAGGACAAGACCCACACCTGTCCTCCATGTCCTGCACCTGAGCTGCTTG
GAGGCCCCTCTGTGTTCCTGTTTCCTCCAAAGCCTAAGGACACCCTGATGATCAGCA
GAACACCTGAAGTGACCTGTGTGGTGGTGGATGTGTCCCATGAGGACCCAGAAGT
GAAGTTCAATTGGTATGTGGATGGGGTTGAAGTGCACAATGCCAAGACCAAGCCTA
GAGAGGAACAGTACAACTCCACCTACAGAGTGGTGTCAGTGCTGACAGTGCTGCA
CCAGGACTGGCTGAATGGCAAAGAGTACAAGTGCAAGGTGTCCAACAAGGCCCTG
CCTGCTCCTATTGAGAAAACCATCTCCAAGGCCAAGGGCCAGCCTAGGGAACCCCA
GGTTTACACACTGCCACCTAGCAGGGATGAGCTGACAAAGAACCAGGTGTCCCTGA
CCTGCCTGGTCAAGGGCTTCTACCCCTCTGACATTGCTGTGGAATGGGAGAGCAAT
GGCCAGCCTGAGAACAACTACAAGACAACCCCTCCTGTGCTGGACTCTGATGGCTC
ATTCTTCCTGTACAGCAAGCTGACTGTGGACAAGAGCAGATGGCAGCAGGGAAATG
TGTTCAGCTGCTCTGTGATGCATGAGGCCCTGCACAACCACTACACCCAGAAAAGC
CTGAGCCTGTCTCCTGGCAAGTGA3’

### <Gene Expression Cassette>

In some aspects of the present disclosure, there is provided a gene expression cassette, comprising the polynucleotide provided in the present disclosure, namely, comprising the polynucleotide encoding the fusion polypeptide provided in the present disclosure.

In some optional embodiments of the present disclosure, the gene expression cassette further comprises, upstream of the polynucleotide encoding the fusion polypeptide provided in the present disclosure, one or more of a promoter, an enhancer, an intron, and a Kozak sequence.

According to the present disclosure, the "promoter" encompasses a DNA sequence that directs the binding of an RNA polymerase and thereby promotes RNA synthesis. The promoter and the expression of the corresponding protein or polypeptide may be ubiquitous (meaning strongly active in a wide range of cells, tissue, and species) or cell type-specific, tissue-specific or species-specific. The promoter may be a "constitutive" (meaning persistently active) or "inducible" (meaning that the promoter may be activated or inactivated depending on the presence or absence of biotic or abiotic factors).

According to the present disclosure, the "enhancer" encompasses a cis-acting element that stimulates or inhibits transcription of adjacent genes. The enhancer that inhibits transcription is also referred to as a "silencer". The enhancer can function at a distance of several thousand base pairs (kb) from a position that is downstream of the coding sequence and the transcribed region in either orientation (i.e., it can be associated with the coding sequence). Exemplarily, an example of a suitable enhancer is a CMV enhancer. Other suitable enhancers include enhancers appropriate for the desired indications of target tissue.

According to the present disclosure, the "intron" includes a splice donor/acceptor region. The intron is located downstream of the promoter region and upstream of the translation initiation sequence of the gene. The intron is a DNA polynucleotide, which is transcribed into RNA and removed by splicing the intron during mRNA processing. The expression of intron-containing gene expression cassettes is generally higher than that of intron-free gene expression cassettes. In some optional embodiments of the present disclosure, the intron may be, for example, a chicken β-actin intron or a human beta globin intron. In other optional embodiments of the present disclosure, the intron is a chimeric intron (CI) - a hybrid intron consisting of a human β-globin splice donor and an immunoglobulin G (IgG) splice acceptor element. Other suitable introns include those known in the art, for example, the introns as described in WO 2011/126808.

According to the present disclosure, the "Kozak sequence" is a nucleic acid sequence located behind the 5' cap structure in the eukaryotic mRNA. It can bind to a translation initiation factor to mediate the translation initiation of mRNA containing the 5' cap structure. In some specific embodiments, the specific Kozak sequence is 5'-GCCACC-3'.

In some optional embodiments of the present disclosure, the gene expression cassette comprises a promoter located upstream of the polynucleotide encoding the fusion polypeptide provided in the present disclosure, and the promoter comprises any one of a CBh promoter, a CB7 promoter, and a CMV promoter.

The CBh promoter is an artificially designed promoter composed of a human cytomegalovirus enhancer, a chicken β-actin promoter, and an intron derived partially from chicken β-actin and partially from minute virus of mice (mvm).

In some specific embodiments, the CBh promoter comprises a sequence as set forth in SEQ ID NO: 3 or a sequence having at least 85% identity thereto.

The nucleotide sequence of the CBh promoter (SEQ ID NO: 3):

5’ACGCGTCTAGTTATTAATAGTAATCAATTACGGGGTCATTAGTTCATAGCCCAT
ATATGGAGTTCCGCGTTACATAACTTACGGTAAATGGCCCGCCTGGCTGACCGCCCA
ACGACCCCCGCCCATTGACGTCAATAATGACGTATGTTCCCATAGTAACGCCAATAG
GGACTTTCCATTGACGTCAATGGGTGGAGTATTTACGGTAAACTGCCCACTTGGCAG
TACATCAAGTGTATCATATGCCAAGTACGCCCCCTATTGACGTCAATGACGGTAAATG
GCCCGCCTGGCATTATGCCCAGTACATGACCTTACGGGACTTTCCTACTTGGCAGTA
CATCTACGTATTAGTCATCGCTATTACCATGGTCGAGGTGAGCCCCACGTTCTGCTTC
ACTCTCCCCATCTCCCCCCCCTCCCCACCCCCAATTTTGTATTTATTTATTTTTTAATTA
TTTTGTGCAGCGATGGGGGCGGGGGGGGGGGGGGCGCGCGCCAGGCGGGGCGGG
GCGGGGCGAGGGGCGGGGCGGGGCGAGGCGGAGAGGTGCGGCGGCAGCCAATCA
GAGCGGCGCGCTCCGAAAGTTTCCTTTTATGGCGAGGCGGCGGCGGCGGCGGCCCT
ATAAAAAGCGAAGCGCGCGGCGGGCGGGGAGTCGCTGCGTTGCCTTCGCCCCGTG
CCCCGCTCCGCGCCGCCTCGCGCCGCCCGCCCCGGCTCTGACTGACCGCGTTACTC
CCACAGGTGAGCGGGCGGGACGGCCCTTCTCCTCCGGGCTGTAATTAGCAAGAGGT
AAGGGTTTAAGGGATGGTTGGTTGGTGGGGTATTAATGTTTAATTACCTGTTTTACAG
GCCTGAAATCACTTGGTTTTAGGTTGG3’

The CB7 is an artificially designed promoter composed of a human cytomegalovirus enhancer, a chicken β-actin promoter, an intron and the like.

In some specific embodiments, the CB7 promoter comprises a sequence as set forth in SEQ ID NO: 4 or a sequence having at least 85% identity thereto.

The nucleotide sequence of the CB7 promoter (SEQ ID NO: 4):

5'ACGCGTCTAGTTATTAATAGTAATCAATTACGGGGTCATTAGTTCATAGCCCAT
ATATGGAGTTCCGCGTTACATAACTTACGGTAAATGGCCCGCCTGGCTGACCGCCCA
ACGACCCCCGCCCATTGACGTCAATAATGACGTATGTTCCCATAGTAACGCCAATAG
GGACTTTCCATTGACGTCAATGGGTGGAGTATTTACGGTAAACTGCCCACTTGGCAG
TACATCAAGTGTATCATATGCCAAGTACGCCCCCTATTGACGTCAATGACGGTAAATG
GCCCGCCTGGCATTATGCCCAGTACATGACCTTACGGGACTTTCCTACTTGGCAGTA
CATCTACGTATTAGTCATCGCTATTACCATGGTCGAGGTGAGCCCCACGTTCTGCTTC
ACTCTCCCCATCTCCCCCCCCTCCCCACCCCCAATTTTGTATTTATTTATTTTTTAATTA
TTTTGTGCAGCGATGGGGGCGGGGGGGGGGGGGGCGCGCGCCAGGCGGGGCGGG
GCGGGGCGAGGGGCGGGGCGGGGCGAGGCGGAGAGGTGCGGCGGCAGCCAATCA
GAGCGGCGCGCTCCGAAAGTTTCCTTTTATGGCGAGGCGGCGGCGGCGGCGGCCCT
ATAAAAAGCGAAGCGCGCGGCGGGCGGGAGTCGCTGCGCGCTGCCTTCGCCCCGT
GCCCCGCTCCGCCGCCGCCTCGCGCCGCCCGCCCCGGCTCTGACTGACCGCGTTAC
TCCCACAGGTGAGCGGGCGGGACGGCCCTTCTCCTCCGGGCTGTAATTAGCGCTTG

GTTTAATGACGGCTTGTTTCTTTTCTGTGGCTGCGTGAAAGCCTTGAGGGGCTCCGG
GAGGGCCCTTTGTGCGGGGGGAGCGGCTCGGGGGGTGCGTGCGTGTGTGTGTGCG
TGGGGAGCGCCGCGTGCGGCTCCGCGCTGCCCGGCGGCTGTGAGCGCTGCGGGCG
CGGCGCGGGGCTTTGTGCGCTCCGCAGTGTGCGCGAGGGGAGCGCGGCCGGGGGC
GGTGCCCCGCGGTGCGGGGGGGGCTGCGAGGGGAACAAAGGCTGCGTGCGGGGT
GTGTGCGTGGGGGGGTGAGCAGGGGGTGTGGGCGCGTCGGTCGGGCTGCAACCCC
CCCTGCACCCCCCTCCCCGAGTTGCTGAGCACGGCCCGGCTTCGGGTGCGGGGCTC
CGTACGGGGCGTGGCGCGGGGCTCGCCGTGCCGGGCGGGGGGTGGCGGCAGGTGG
GGGTGCCGGGCGGGGCGGGGCCGCCTCGGGCCGGGGAGGGCTCGGGGGAGGGGC
GCGGCGGCCCCCGGAGCGCCGGCGGCTGTCGAGGCGCGGCGAGCCGCAGCCATTG
CCTTTTATGGTAATCGTGCGAGAGGGCGCAGGGACTTCCTTTGTCCCAAATCTGTGC
GGAGCCGAAATCTGGGAGGCGCCGCCGCACCCCCTCTAGCGGGCGCGGGGCGAAG
CGGTGCGGCGCCGGCAGGAAGGAAATGGGCGGGGAGGGCCTTCGTGCGTCGCCGC
GCCGCCGTCCCCTTCTCCCTCTCCAGCCTCGGGGCTGTCCGCGGGGGGACGGCTGC
CTTCGGGGGGGACGGGGCAGGGCGGGGTTCGGCTTCTGGCGTGTGACCGGCGGCT
CTAGAGCCTCTGCTAACCATGTTCATGCCTTCTTCTTTTTCCTACAGCTCCTGGGCAA
CGTGCTGGTTATTGTGCTGTCTCATCATTTTGGCAAAG3’

In some optional embodiments of the present disclosure, the gene expression cassette comprises a promoter and an intron located upstream of the polynucleotide encoding the fusion polypeptide provided in the present disclosure. The promoter is a CMV promoter, and the intron is a human β-globin intron.

In some specific embodiments, the CMV promoter comprises a sequence as set forth in SEQ ID NO: 5 or a sequence having at least 85% identity thereto.

The nucleotide sequence of the CMV promoter (SEQ ID NO: 5):

5’ACGCGTCTAGTTATTAATAGTAATCAATTACGGGGTCATTAGTTCATAGCCCAT
ATATGGAGTTCCGCGTTACATAACTTACGGTAAATGGCCCGCCTGGCTGACCGCCCA
ACGACCCCCGCCCATTGACGTCAATAATGACGTATGTTCCCATAGTAACGCCAATAG
GGACTTTCCATTGACGTCAATGGGTGGAGTATTTACGGTAAACTGCCCACTTGGCAG
TACATCAAGTGTATCATATGCCAAGTACGCCCCCTATTGACGTCAATGACGGTAAATG
GCCCGCCTGGCATTATGCCCAGTACATGACCTTATGGGACTTTCCTACTTGGCAGTA
CATCTACGTATTAGTCATCGCTATTACCATGGTGATGCGGTTTTGGCAGTACATCAAT
GGGCGTGGATAGCGGTTTGACTCACGGGGATTTCCAAGTCTCCACCCCATTGACGT
CAATGGGAGTTTGTTTTGGCACCAAAATCAACGGGACTTTCCAAAATGTCGTAACA
ACTCCGCCCCATTGACGCAAATGGGCGGTAGGCGTGTACGGTGGGAGGTCTATATAA
GCAGAGCTCGTTTAGTGAACCGTCAGATCGCCTGGAGACGCCATCCACGCTGTTTT

GACCTCCATAGAAGACACCGGGACCGATCCAGCCTCC3'

In some specific embodiments, the human β-globin intron comprises a sequence as set forth in SEQ ID NO: 6 or a sequence having at least 85% identity thereto.

The nucleotide sequence of the human β-globin intron (SEQ ID NO: 6):

5’CGAATCCCGGCCGGGAACGGTGCATTGGAACGCGGATTCCCCGTGCCAAGA
GTGACGTAAGTACCGCCTATAGAGTCTATAGGCCCACAAAAAATGCTTTCTTCTTTT
AATATACTTTTTTGTTTATCTTATTTCTAATACTTTCCCTAATCTCTTTCTTTCAGGGCA
ATAATGATACAATGTATCATGCCTCTTTGCACCATTCTAAAGAATAACAGTGATAATTT
CTGGGTTAAGGCAATAGCAATATTTCTGCATATAAATATTTCTGCATATAAATTGTAAC
TGATGTAAGAGGTTTCATATTGCTAATAGCAGCTACAATCCAGCTACCATTCTGCTTT
TATTTTATGGTTGGGATAAGGCTGGATTATTCTGAGTCCAAGCTAGGCCCTTTTGCTA
ATCATGTTCATACCTCTTATCTTCCTCCCACAGCTCCTGGGCAACGTGCTGGTCTGTG
TGCTGGCCCATCACTTTGGCAAAGAATTGGGAT3’

In some optional embodiments of the present disclosure, the gene expression cassette comprises a polyadenylation region (or referred to as a polyadenylation signal) located downstream of the polynucleotide encoding the fusion polypeptide provided in the present disclosure.

As understood in the related art, the RNA polymerase II transcript is terminated through cleavage and the addition of a polyadenylation region. The polyadenylation region may also be referred to as a polyadenylation signal, a polyA signal, a polyA region or a polyA tail. The polyA region contains a plurality of consecutive adenosine monophosphates, which typically have repeats of the motif AAUAAA. Several efficient polyadenylation sites have been identified, including those from SV40, bovine growth hormone, human growth hormone, and rabbit beta globin. The most efficient polyA signal for expressing a transgene in mammalian cells may depend on the cell type, the species of interest, and the specific vector used. In some examples of the present disclosure, the gene expression cassette comprises a polyA region selected from the group consisting of: a bovine growth hormone (bGH), a human growth hormone (hGH), and a β-globin (beta globin).

In some preferred embodiments, the polyA region is a human growth hormone polyA region. In some specific embodiments, the human growth hormone polyA region comprises a sequence as set forth in SEQ ID NO: 7 or a sequence having at least 85% identity thereto.

The nucleotide sequence of the human growth hormone polyA region (SEQ ID NO: 7):

5’CGGGTGGCATCCCTGTGACCCCTCCCCAGTGCCTCTCCTGGCCCTGGAAGTT
GCCACTCCAGTGCCCACCAGCCTTGTCCTAATAAAATTAAGTTGCATCATTTTGTCTG

ACTAGGTGTCCTTCTATAATATTATGGGGTGGAGGGGGGTGGTATGGAGCAAGGGGC
AAGTTGGGAAGACAACCTGTAGGGCCTGCGGGGTCTATTGGGAACCAAGCTGGAG
TGCAGTGGCACAATCTTGGCTCACTGCAATCTCCGCCTCCTGGGTTCAAGCGATTCT
CCTGCCTCAGCCTCCCGAGTTGTTGGGATTCCAGGCATGCATGACCAGGCTCAGCTA
ATTTTTGTTTTTTTGGTAGAGACGGGGTTTCACCATATTGGCCAGGCTGGTCTCCAA
CTCCTAATCTCAGGTGATCTACCCACCTTGGCCTCCCAAATTGCTGGGATTACAGGC
GTGAACCACTGCTCCCTTCCCTGTCCTTCGG3’

As will be appreciated by one of ordinary skill in the art, the gene expression cassette may optionally contain other elements including, but not limited to, restriction sites that facilitate cloning and regulatory elements used for particular gene expression vectors. Examples of the regulatory sequence include ITR of an AAV vector, a bacterial sequence of a plasmid vector, an attP or attB site of a phage integrase vector, and transposable elements of a transposon.

In some more preferred embodiments, the gene expression cassette comprises a sequence as set forth in any one of SEQ ID NOs: 8 to 10 and 35 to 43 or a sequence having at least 85% identity to any one of SEQ ID NOs: 8 to 10 and 35 to 43.

### Exemplarily:

SEQ ID NO: 8 (CMV-hIL2sp-Aflibercept opt3-hGH pA) 
5'ACGCGTCTAGTTATTAATAGTAATCAATTACGGGGTCATTAGTTCATAGCCCAT
ATATGGAGTTCCGCGTTACATAACTTACGGTAAATGGCCCGCCTGGCTGACCGCCCA
ACGACCCCCGCCCATTGACGTCAATAATGACGTATGTTCCCATAGTAACGCCAATAG
GGACTTTCCATTGACGTCAATGGGTGGAGTATTTACGGTAAACTGCCCACTTGGCAG
TACATCAAGTGTATCATATGCCAAGTACGCCCCCTATTGACGTCAATGACGGTAAATG
GCCCGCCTGGCATTATGCCCAGTACATGACCTTATGGGACTTTCCTACTTGGCAGTA
CATCTACGTATTAGTCATCGCTATTACCATGGTGATGCGGTTTTGGCAGTACATCAAT
GGGCGTGGATAGCGGTTTGACTCACGGGGATTTCCAAGTCTCCACCCCATTGACGT
CAATGGGAGTTTGTTTTGGCACCAAAATCAACGGGACTTTCCAAAATGTCGTAACA
ACTCCGCCCCATTGACGCAAATGGGCGGTAGGCGTGTACGGTGGGAGGTCTATATAA
GCAGAGCTCGTTTAGTGAACCGTCAGATCGCCTGGAGACGCCATCCACGCTGTTTT
GACCTCCATAGAAGACACCGGGACCGATCCAGCCTCCGCGGATTCGAATCCCGGCC
GGGAACGGTGCATTGGAACGCGGATTCCCCGTGCCAAGAGTGACGTAAGTACCGCC
TATAGAGTCTATAGGCCCACAAAAAATGCTTTCTTCTTTTAATATACTTTTTTGTTTAT
CTTATTTCTAATACTTTCCCTAATCTCTTTCTTTCAGGGCAATAATGATACAATGTATC
ATGCCTCTTTGCACCATTCTAAAGAATAACAGTGATAATTTCTGGGTTAAGGCAATAG
CAATATTTCTGCATATAAATATTTCTGCATATAAATTGTAACTGATGTAAGAGGTTTCA
 
TATTGCTAATAGCAGCTACAATCCAGCTACCATTCTGCTTTTATTTTATGGTTGGGATA
AGGCTGGATTATTCTGAGTCCAAGCTAGGCCCTTTTGCTAATCATGTTCATACCTCTT
ATCTTCCTCCCACAGCTCCTGGGCAACGTGCTGGTCTGTGTGCTGGCCCATCACTTT
GGCAAAGAATTGGGATAATTACTAGATCGCCACCATGTACAGAATGCAGCTGCTGAG
CTGCATAGCCCTGTCTCTGGCCCTGGTGACCAACAGCTCTGACACAGGCAGACCCT
TTGTGGAAATGTACTCTGAGATCCCTGAGATCATCCACATGACAGAGGGCAGAGAG
CTGGTCATCCCCTGCAGAGTGACAAGCCCCAACATCACAGTGACCCTGAAGAAGTT
CCCTCTGGACACACTGATCCCTGATGGCAAGAGAATCATCTGGGACAGCAGAAAGG
GCTTCATCATCAGCAATGCCACCTACAAAGAGATTGGCCTGCTGACCTGTGAAGCC
ACAGTGAATGGCCACCTGTACAAGACCAACTACCTGACACACAGACAGACCAACA
CCATCATTGATGTGGTGCTGAGCCCCAGCCATGGCATTGAGCTGTCTGTGGGAGAG
AAGCTGGTGCTGAACTGCACAGCCAGAACAGAGCTGAATGTGGGCATTGACTTCA
ACTGGGAGTACCCCAGCAGCAAGCACCAGCACAAGAAACTGGTCAACAGGGACCT
GAAAACCCAGAGTGGCTCTGAGATGAAGAAATTCCTGAGCACCCTGACCATTGATG
GGGTCACCAGATCTGACCAGGGCCTGTACACATGTGCTGCCAGCTCTGGACTGATG
ACCAAGAAAAACAGCACCTTTGTCAGAGTGCATGAGAAGGACAAGACCCACACCT
GTCCTCCATGTCCTGCACCTGAGCTGCTTGGAGGCCCCTCTGTGTTCCTGTTTCCTC
CAAAGCCTAAGGACACCCTGATGATCAGCAGAACACCTGAAGTGACCTGTGTGGTG
GTGGATGTGTCCCATGAGGACCCAGAAGTGAAGTTCAATTGGTATGTGGATGGGGT
TGAAGTGCACAATGCCAAGACCAAGCCTAGAGAGGAACAGTACAACTCCACCTAC
AGAGTGGTGTCAGTGCTGACAGTGCTGCACCAGGACTGGCTGAATGGCAAAGAGT
ACAAGTGCAAGGTGTCCAACAAGGCCCTGCCTGCTCCTATTGAGAAAACCATCTCC
AAGGCCAAGGGCCAGCCTAGGGAACCCCAGGTTTACACACTGCCACCTAGCAGGG
ATGAGCTGACAAAGAACCAGGTGTCCCTGACCTGCCTGGTCAAGGGCTTCTACCCC
TCTGACATTGCTGTGGAATGGGAGAGCAATGGCCAGCCTGAGAACAACTACAAGAC
AACCCCTCCTGTGCTGGACTCTGATGGCTCATTCTTCCTGTACAGCAAGCTGACTGT
GGACAAGAGCAGATGGCAGCAGGGAAATGTGTTCAGCTGCTCTGTGATGCATGAG
GCCCTGCACAACCACTACACCCAGAAAAGCCTGAGCCTGTCTCCTGGCAAGTGAG
CGGCCGCAAGCTTCGGGTGGCATCCCTGTGACCCCTCCCCAGTGCCTCTCCTGGCC
CTGGAAGTTGCCACTCCAGTGCCCACCAGCCTTGTCCTAATAAAATTAAGTTGCATC
ATTTTGTCTGACTAGGTGTCCTTCTATAATATTATGGGGTGGAGGGGGGTGGTATGGA
GCAAGGGGCAAGTTGGGAAGACAACCTGTAGGGCCTGCGGGGTCTATTGGGAACC
AAGCTGGAGTGCAGTGGCACAATCTTGGCTCACTGCAATCTCCGCCTCCTGGGTTC
AAGCGATTCTCCTGCCTCAGCCTCCCGAGTTGTTGGGATTCCAGGCATGCATGACCA
GGCTCAGCTAATTTTTGTTTTTTTGGTAGAGACGGGGTTTCACCATATTGGCCAGGC
 
TGGTCTCCAACTCCTAATCTCAGGTGATCTACCCACCTTGGCCTCCCAAATTGCTGG
GATTACAGGCGTGAACCACTGCTCCCTTCCCTGTCCTTCGG3'
SEQ ID NO: 9 (CBh-hIL2sp-Aflibercept opt3-hGH pA) 
5'ACGCGTCTAGTTATTAATAGTAATCAATTACGGGGTCATTAGTTCATAGCCCAT
ATATGGAGTTCCGCGTTACATAACTTACGGTAAATGGCCCGCCTGGCTGACCGCCCA
ACGACCCCCGCCCATTGACGTCAATAATGACGTATGTTCCCATAGTAACGCCAATAG
GGACTTTCCATTGACGTCAATGGGTGGAGTATTTACGGTAAACTGCCCACTTGGCAG
TACATCAAGTGTATCATATGCCAAGTACGCCCCCTATTGACGTCAATGACGGTAAATG
GCCCGCCTGGCATTATGCCCAGTACATGACCTTACGGGACTTTCCTACTTGGCAGTA
CATCTACGTATTAGTCATCGCTATTACCATGGTCGAGGTGAGCCCCACGTTCTGCTTC
ACTCTCCCCATCTCCCCCCCCTCCCCACCCCCAATTTTGTATTTATTTATTTTTTAATTA
TTTTGTGCAGCGATGGGGGCGGGGGGGGGGGGGGCGCGCGCCAGGCGGGGCGGG
GCGGGGCGAGGGGCGGGGCGGGGCGAGGCGGAGAGGTGCGGCGGCAGCCAATCA
GAGCGGCGCGCTCCGAAAGTTTCCTTTTATGGCGAGGCGGCGGCGGCGGCGGCCCT
ATAAAAAGCGAAGCGCGCGGCGGGCGGGGAGTCGCTGCGTTGCCTTCGCCCCGTG
CCCCGCTCCGCGCCGCCTCGCGCCGCCCGCCCCGGCTCTGACTGACCGCGTTACTC
CCACAGGTGAGCGGGCGGGACGGCCCTTCTCCTCCGGGCTGTAATTAGCAAGAGGT
AAGGGTTTAAGGGATGGTTGGTTGGTGGGGTATTAATGTTTAATTACCTGTTTTACAG
GCCTGAAATCACTTGGTTTTAGGTTGGAATTACTAGATCGCCACCATGTACAGAATG
CAGCTGCTGAGCTGCATAGCCCTGTCTCTGGCCCTGGTGACCAACAGCTCTGACAC
AGGCAGACCCTTTGTGGAAATGTACTCTGAGATCCCTGAGATCATCCACATGACAG
AGGGCAGAGAGCTGGTCATCCCCTGCAGAGTGACAAGCCCCAACATCACAGTGAC
CCTGAAGAAGTTCCCTCTGGACACACTGATCCCTGATGGCAAGAGAATCATCTGGG
ACAGCAGAAAGGGCTTCATCATCAGCAATGCCACCTACAAAGAGATTGGCCTGCTG
ACCTGTGAAGCCACAGTGAATGGCCACCTGTACAAGACCAACTACCTGACACACA
GACAGACCAACACCATCATTGATGTGGTGCTGAGCCCCAGCCATGGCATTGAGCTG
TCTGTGGGAGAGAAGCTGGTGCTGAACTGCACAGCCAGAACAGAGCTGAATGTGG
GCATTGACTTCAACTGGGAGTACCCCAGCAGCAAGCACCAGCACAAGAAACTGGT
CAACAGGGACCTGAAAACCCAGAGTGGCTCTGAGATGAAGAAATTCCTGAGCACC
CTGACCATTGATGGGGTCACCAGATCTGACCAGGGCCTGTACACATGTGCTGCCAG
CTCTGGACTGATGACCAAGAAAAACAGCACCTTTGTCAGAGTGCATGAGAAGGAC
AAGACCCACACCTGTCCTCCATGTCCTGCACCTGAGCTGCTTGGAGGCCCCTCTGT
GTTCCTGTTTCCTCCAAAGCCTAAGGACACCCTGATGATCAGCAGAACACCTGAAG
TGACCTGTGTGGTGGTGGATGTGTCCCATGAGGACCCAGAAGTGAAGTTCAATTGG
 
TATGTGGATGGGGTTGAAGTGCACAATGCCAAGACCAAGCCTAGAGAGGAACAGTA
CAACTCCACCTACAGAGTGGTGTCAGTGCTGACAGTGCTGCACCAGGACTGGCTGA
ATGGCAAAGAGTACAAGTGCAAGGTGTCCAACAAGGCCCTGCCTGCTCCTATTGAG
AAAACCATCTCCAAGGCCAAGGGCCAGCCTAGGGAACCCCAGGTTTACACACTGC
CACCTAGCAGGGATGAGCTGACAAAGAACCAGGTGTCCCTGACCTGCCTGGTCAA
GGGCTTCTACCCCTCTGACATTGCTGTGGAATGGGAGAGCAATGGCCAGCCTGAGA
ACAACTACAAGACAACCCCTCCTGTGCTGGACTCTGATGGCTCATTCTTCCTGTACA
GCAAGCTGACTGTGGACAAGAGCAGATGGCAGCAGGGAAATGTGTTCAGCTGCTC
TGTGATGCATGAGGCCCTGCACAACCACTACACCCAGAAAAGCCTGAGCCTGTCTC
CTGGCAAGTGAGCGGCCGCAAGCTTCGGGTGGCATCCCTGTGACCCCTCCCCAGTG
CCTCTCCTGGCCCTGGAAGTTGCCACTCCAGTGCCCACCAGCCTTGTCCTAATAAAA
TTAAGTTGCATCATTTTGTCTGACTAGGTGTCCTTCTATAATATTATGGGGTGGAGGG
GGGTGGTATGGAGCAAGGGGCAAGTTGGGAAGACAACCTGTAGGGCCTGCGGGGT
CTATTGGGAACCAAGCTGGAGTGCAGTGGCACAATCTTGGCTCACTGCAATCTCCG
CCTCCTGGGTTCAAGCGATTCTCCTGCCTCAGCCTCCCGAGTTGTTGGGATTCCAGG
CATGCATGACCAGGCTCAGCTAATTTTTGTTTTTTTGGTAGAGACGGGGTTTCACCAT
ATTGGCCAGGCTGGTCTCCAACTCCTAATCTCAGGTGATCTACCCACCTTGGCCTCC
CAAATTGCTGGGATTACAGGCGTGAACCACTGCTCCCTTCCCTGTCCTTCGG3’
SEQ ID NO: 10 (CB7-hIL2sp-Aflibercept opt3-hGH pA)

5'ACGCGTCTAGTTATTAATAGTAATCAATTACGGGGTCATTAGTTCATAGCCCAT
ATATGGAGTTCCGCGTTACATAACTTACGGTAAATGGCCCGCCTGGCTGACCGCCCA
ACGACCCCCGCCCATTGACGTCAATAATGACGTATGTTCCCATAGTAACGCCAATAG
GGACTTTCCATTGACGTCAATGGGTGGAGTATTTACGGTAAACTGCCCACTTGGCAG
TACATCAAGTGTATCATATGCCAAGTACGCCCCCTATTGACGTCAATGACGGTAAATG
GCCCGCCTGGCATTATGCCCAGTACATGACCTTACGGGACTTTCCTACTTGGCAGTA
CATCTACGTATTAGTCATCGCTATTACCATGGTCGAGGTGAGCCCCACGTTCTGCTTC
ACTCTCCCCATCTCCCCCCCCTCCCCACCCCCAATTTTGTATTTATTTATTTTTTAATTA
TTTTGTGCAGCGATGGGGGCGGGGGGGGGGGGGGCGCGCGCCAGGCGGGGCGGG
GCGGGGCGAGGGGCGGGGCGGGGCGAGGCGGAGAGGTGCGGCGGCAGCCAATCA
GAGCGGCGCGCTCCGAAAGTTTCCTTTTATGGCGAGGCGGCGGCGGCGGCGGCCCT
ATAAAAAGCGAAGCGCGCGGCGGGCGGGAGTCGCTGCGCGCTGCCTTCGCCCCGT
GCCCCGCTCCGCCGCCGCCTCGCGCCGCCCGCCCCGGCTCTGACTGACCGCGTTAC
TCCCACAGGTGAGCGGGCGGGACGGCCCTTCTCCTCCGGGCTGTAATTAGCGCTTG
GTTTAATGACGGCTTGTTTCTTTTCTGTGGCTGCGTGAAAGCCTTGAGGGGCTCCGG

GAGGGCCCTTTGTGCGGGGGGAGCGGCTCGGGGGGTGCGTGCGTGTGTGTGTGCG
TGGGGAGCGCCGCGTGCGGCTCCGCGCTGCCCGGCGGCTGTGAGCGCTGCGGGCG
CGGCGCGGGGCTTTGTGCGCTCCGCAGTGTGCGCGAGGGGAGCGCGGCCGGGGGC
GGTGCCCCGCGGTGCGGGGGGGGCTGCGAGGGGAACAAAGGCTGCGTGCGGGGT
GTGTGCGTGGGGGGGTGAGCAGGGGGTGTGGGCGCGTCGGTCGGGCTGCAACCCC
CCCTGCACCCCCCTCCCCGAGTTGCTGAGCACGGCCCGGCTTCGGGTGCGGGGCTC
CGTACGGGGCGTGGCGCGGGGCTCGCCGTGCCGGGCGGGGGGTGGCGGCAGGTGG
GGGTGCCGGGCGGGGCGGGGCCGCCTCGGGCCGGGGAGGGCTCGGGGGAGGGGC
GCGGCGGCCCCCGGAGCGCCGGCGGCTGTCGAGGCGCGGCGAGCCGCAGCCATTG
CCTTTTATGGTAATCGTGCGAGAGGGCGCAGGGACTTCCTTTGTCCCAAATCTGTGC
GGAGCCGAAATCTGGGAGGCGCCGCCGCACCCCCTCTAGCGGGCGCGGGGCGAAG
CGGTGCGGCGCCGGCAGGAAGGAAATGGGCGGGGAGGGCCTTCGTGCGTCGCCGC
GCCGCCGTCCCCTTCTCCCTCTCCAGCCTCGGGGCTGTCCGCGGGGGGACGGCTGC
CTTCGGGGGGGACGGGGCAGGGCGGGGTTCGGCTTCTGGCGTGTGACCGGCGGCT
CTAGAGCCTCTGCTAACCATGTTCATGCCTTCTTCTTTTTCCTACAGCTCCTGGGCAA
CGTGCTGGTTATTGTGCTGTCTCATCATTTTGGCAAAGAATTACTAGATCGCCACCAT
GTACAGAATGCAGCTGCTGAGCTGCATAGCCCTGTCTCTGGCCCTGGTGACCAACA
GCTCTGACACAGGCAGACCCTTTGTGGAAATGTACTCTGAGATCCCTGAGATCATCC
ACATGACAGAGGGCAGAGAGCTGGTCATCCCCTGCAGAGTGACAAGCCCCAACAT
CACAGTGACCCTGAAGAAGTTCCCTCTGGACACACTGATCCCTGATGGCAAGAGAA
TCATCTGGGACAGCAGAAAGGGCTTCATCATCAGCAATGCCACCTACAAAGAGATT
GGCCTGCTGACCTGTGAAGCCACAGTGAATGGCCACCTGTACAAGACCAACTACCT
GACACACAGACAGACCAACACCATCATTGATGTGGTGCTGAGCCCCAGCCATGGCA
TTGAGCTGTCTGTGGGAGAGAAGCTGGTGCTGAACTGCACAGCCAGAACAGAGCT
GAATGTGGGCATTGACTTCAACTGGGAGTACCCCAGCAGCAAGCACCAGCACAAG
AAACTGGTCAACAGGGACCTGAAAACCCAGAGTGGCTCTGAGATGAAGAAATTCC
TGAGCACCCTGACCATTGATGGGGTCACCAGATCTGACCAGGGCCTGTACACATGT
GCTGCCAGCTCTGGACTGATGACCAAGAAAAACAGCACCTTTGTCAGAGTGCATGA
GAAGGACAAGACCCACACCTGTCCTCCATGTCCTGCACCTGAGCTGCTTGGAGGCC
CCTCTGTGTTCCTGTTTCCTCCAAAGCCTAAGGACACCCTGATGATCAGCAGAACAC
CTGAAGTGACCTGTGTGGTGGTGGATGTGTCCCATGAGGACCCAGAAGTGAAGTTC
AATTGGTATGTGGATGGGGTTGAAGTGCACAATGCCAAGACCAAGCCTAGAGAGGA
ACAGTACAACTCCACCTACAGAGTGGTGTCAGTGCTGACAGTGCTGCACCAGGACT
GGCTGAATGGCAAAGAGTACAAGTGCAAGGTGTCCAACAAGGCCCTGCCTGCTCCT
ATTGAGAAAACCATCTCCAAGGCCAAGGGCCAGCCTAGGGAACCCCAGGTTTACAC

ACTGCCACCTAGCAGGGATGAGCTGACAAAGAACCAGGTGTCCCTGACCTGCCTG
GTCAAGGGCTTCTACCCCTCTGACATTGCTGTGGAATGGGAGAGCAATGGCCAGCC
TGAGAACAACTACAAGACAACCCCTCCTGTGCTGGACTCTGATGGCTCATTCTTCCT
GTACAGCAAGCTGACTGTGGACAAGAGCAGATGGCAGCAGGGAAATGTGTTCAGC
TGCTCTGTGATGCATGAGGCCCTGCACAACCACTACACCCAGAAAAGCCTGAGCCT
GTCTCCTGGCAAGTGAGCGGCCGCAAGCTTCGGGTGGCATCCCTGTGACCCCTCCC
CAGTGCCTCTCCTGGCCCTGGAAGTTGCCACTCCAGTGCCCACCAGCCTTGTCCTAA
TAAAATTAAGTTGCATCATTTTGTCTGACTAGGTGTCCTTCTATAATATTATGGGGTGG
AGGGGGGTGGTATGGAGCAAGGGGCAAGTTGGGAAGACAACCTGTAGGGCCTGCG
GGGTCTATTGGGAACCAAGCTGGAGTGCAGTGGCACAATCTTGGCTCACTGCAATC
TCCGCCTCCTGGGTTCAAGCGATTCTCCTGCCTCAGCCTCCCGAGTTGTTGGGATTC
CAGGCATGCATGACCAGGCTCAGCTAATTTTTGTTTTTTTGGTAGAGACGGGGTTTC
ACCATATTGGCCAGGCTGGTCTCCAACTCCTAATCTCAGGTGATCTACCCACCTTGG
CCTCCCAAATTGCTGGGATTACAGGCGTGAACCACTGCTCCCTTCCCTGTCCTTCGG
3’

### <Gene Delivery Vector>

In some aspects of the present disclosure, the gene expression cassette of the present disclosure is used for delivering a gene (encoding a fusion polypeptide) to an animal cell, for example, to determine the effect of the gene on the cell viability and/or functions, to treat cellular disorders, etc. Therefore, in some aspects of the present disclosure, there is provided a gene delivery vector, comprising the gene expression cassette of the present disclosure. In some preferred embodiments, the gene delivery vector is used to express a transgene (encoding a fusion polypeptide) in a mammalian cell.

The gene delivery vector of the present disclosure encompasses any suitable gene delivery vector used for delivering a polynucleotide sequence to a mammalian cell. For example, the vector may include a single-stranded or double-stranded nucleic acid, e.g., a single-stranded or double-stranded DNA. For example, the gene delivery vector may be DNA, such as a naked DNA, e.g., plasmids or minicircles. The vector may include a single-stranded or double-stranded RNA, including a modified form of RNA. In another example, the gene delivery vector may be RNA, e.g., mRNA or modified mRNA.

As another example, the gene delivery vector may be a viral vector derived from a virus, such as an adenovirus, an adeno-associated virus (AAV), a lentivirus, a herpes virus, an alphavirus or a retrovirus, e.g., Moloney murine leukemia virus (M-MuLV), Moloney murine sarcoma virus (MoMSV), Harvey murine sarcoma virus (HaMuSV), murine mammary tumor virus (MuMTV), gibbon ape leukemia virus (GaLV), feline leukemia virus (FLV), foam virus, Friend murine leukemia virus, murine stem cell virus (MSCV), and Rous sarcoma virus (RSV) or lentivirus. While examples covering the use of adeno-associated viruses are described in greater detail below, it is expected that one of ordinary skill in the art will recognize that similar knowledge and skills in the related art are also applicable to non-AAV gene delivery vectors.

In some embodiments, the gene delivery vector is a recombinant adeno-associated virus (rAAV). In such an embodiment, the gene expression cassette is flanked by a functional AAV inverted terminal repeat (ITR) sequence at the 5' and 3' ends. The "functional AAV ITR sequence" refers to an ITR sequence for rescuing, replicating, and packaging AAV viral particles as desired. Accordingly, the AAV ITR used in the gene delivery vector of the present disclosure does not require a wild-type nucleotide sequence and may be altered by nucleotide insertion, deletion or substitution, or may be derived from any of several AAV serotypes, e.g., AAV1, AAV2, AAV3, AAV4, AAV5, AAV6, AAV7, AAV8, AAV9, and AAV10. A preferred AAV vector has the wild-type *Rep* gene and *Cap* gene deleted in whole or in part, while retaining the functional flanking ITR sequences. In a particular embodiment, the AAV viral vector is an AAV variant. In some embodiments, the AAV variant is an AAV viral vector containing a variant AAV capsid (or referred to as an AAV capsid protein variant).

In some embodiments, the gene expression cassette is encapsidated in the AAV capsid. The AAV capsid may be derived from any of adeno-associated virus serotypes including, but not limited to, AAV1, AAV2, AAV3, AAV4, AAV5, AAV6, AAV7, AAV8, AAV9, AAV10, etc. Any of the adeno-associated virus serotypes can act as a gene delivery vector. For example, the AAV capsid may be a wild-type capsid or a natural capsid. However, like ITRs, the capsid does not require a wild-type nucleotide sequence. As long as the capsid is capable of transducing mammalian cells, its sequence can be altered by nucleotide insertion, deletion or substitution in the VP1, VP2 or VP3 sequence relative to the wild-type sequence. In other words, the AAV capsid may be a variant AAV capsid that includes one or more amino acid substitutions, deletions or insertions relative to the parental capsid protein or the AAV capsid protein.

AAV capsid is an icosahedron composed of 60 VP capsid protein monomers including 5 VP1 monomers, 5 VP2 monomers, and 50 VP3 monomers. VP1, VP2, and VP3 monomers are all transcribed and translated by the *Cap* gene of AAV. VP1 is the longest and includes approximate 735 amino acids. VP2 and VP3 are "truncated forms" of VP1 and do not contain part of amino acids at the N-terminus of the VP1 protein. Conventionally, the modification sites in the capsid protein are designated based on the amino acid sequence of the VP1 protein.

The AAV capsid protein variant may be an AAV capsid protein variant containing a targeting peptide having a targeting ability to a specific tissue. In some specific embodiments, the targeting peptide is a targeting peptide having a good targeting ability towards the eye. Selectable targeting peptides are as shown in Table 1 below.

**Table 1:**

| Targeting Peptide | SEQ ID NO: | Sequence |
|---|---|---|
| Targeting peptide 1 | 57 | AAARGDLATIAA |
| Targeting peptide 2 | 58 | AAARGDLGRLAA |
| Targeting peptide 3 | 59 | AAARGDLQNIAA |
| Targeting peptide 4 | 60 | AAARGDLQNLAA |
| Targeting peptide 5 | 61 | AAARGDLAAIAA |
| Targeting peptide 6 | 62 | AAARGDLATLAA |
| Targeting peptide 7 | 63 | AAARGDLATPAA |
| Targeting peptide 8 | 64 | AAARGDLGRPAA |
| Targeting peptide 9 | 65 | AAARGDLGTLAA |
| Targeting peptide 10 | 66 | AAARGDLARIAA |
| Targeting peptide 11 | 67 | AAARGSLQNIAA |
| Targeting peptide 12 | 68 | AAARGSLQNLAA |
| Targeting peptide 13 | 69 | AAARGSLAA |
| Targeting peptide 14 | / | NG |
| Targeting peptide 15 | 11 | AAAGNGRAHAAA |

In some more specific embodiments, the AAV capsid protein variant is an AAV2 capsid protein variant.

In some preferred embodiments, the AAV2 capsid protein variant is any one of AAV2 capsid protein variants IVT1 to IVT15.

The above-mentioned targeting peptides 1 to 15 and IVT1 to IVT15 are described in the PCT International Application No.: PCT/CN2023/095837, which is incorporated herein by reference. The sequences and specific construction methods of the targeting peptides 1 to 15 and IVT1 to IVT15 are described in Example 5 below.

In some preferred embodiments, the AAV2 capsid protein variant is an AAV2 capsid protein variant IVT15, which is obtained by inserting the targeting peptide 15 (AAAGNGRAHAAA (SEQ ID NO: 11)) having a good targeting ability to the eye into the position at the 587^{th} amino acid in the AAV2 VP1. That is, the targeting peptide is located between the 587^{th} amino acid and the 588^{th} amino acid in the AAV2 VP1 to obtain the AAV2 capsid protein/AAV2 containing the capsid protein.

In other preferred embodiments, the AAV2 capsid protein variant is an AAV2 capsid protein variant IVT13, which is obtained by inserting the targeting peptide 13 (AAARGSLAA (SEQ ID NO: 69)) having a good targeting ability to the eye into the position at the 587^{th} amino acid in the AAV2 VP1. That is, the targeting peptide is located between the 587^{th} amino acid and the 588^{th} amino acid in the AAV2 VP1 to obtain the AAV2 capsid protein/AAV2 containing the capsid protein.

Preferably, rAAV is replication-deficient because an AAV vector cannot replicate and package its genome independently. For example, when cone cells are transduced with rAAV viral particles, the gene is expressed in the transduced cone cells. However, rAAV cannot replicate due to the fact of lack of the AAV *rep* gene and *cap* gene and the helper function gene(s) in the transduced cone cells.

A gene delivery vector (e.g., a rAAV viral particle) that encapsidates the gene expression cassette of the present disclosure may be generated by a standard method. For example, in the case of rAAV viral particles, the AAV expression vector according to the present disclosure can be introduced into a producer cell, followed by the introduction of an AAV helper construct including an AAV coding region that is capable of expressing in the producer cell and may complement the AAV helper functions absent in the AAV vector. Subsequently, a helper virus and/or an additional vector are introduced into a producer cell, wherein the helper virus and/or an additional vector provide helper functions capable of supporting production of efficient rAAV viruses. Afterwards, the producer cell is cultured to produce rAAVs. These steps are carried out according to standard methods. Replication-deficient AAV viral particles that encapsidate the recombinant AAV vectors of the present disclosure are prepared by standard techniques known in the related art using AAV packaging cells and packaging techniques.

Any concentration of viral particles suitable for efficient transduction of mammalian cells may be prepared for contact with mammalian cells *in vitro* or *in vivo.* Similarly, any total number of viral particles suitable for providing appropriate cell transduction to impart a desired effect or treat diseases may be administered to a mammal. Any suitable number of vectors may be administered to an eye of a mammal or primate.

A viral vector may be formulated into a pharmaceutical composition comprising any suitable unit dose of the vector. The pharmaceutical composition may be administered to a subject to cause a change in the subject or treat a disease in the subject.

In some cases, the multiplicity of infection (MOI) may be used as a metric for the unit dose of the pharmaceutical composition. MOI refers to the ratio or fold of the vector or viral genome to the cell to which the nucleic acid can be delivered.

In the preparation of a rAAV composition, any host cell used for generating rAAV viral particles may be used, which includes, but is not limited to, for example, mammalian cells (e.g., 293 cells), insect cells (e.g., SF9 cells), microorganisms, and yeast. The host cell may be a packaging cell or a producer cell. In the packaging cell, the AAV *rep* gene and *cap* gene are stably maintained in the host cell, and the AAV vector genome is stably maintained and packaged in the producer cell. Exemplary packaging and producer cells are derived from SF-9, 293, A549 or HeLa cells. AAV vectors are purified and formulated by the standard techniques known in the related art.

### <Pharmaceutical Composition>

As disclosed herein, in some aspects of the present disclosure, there is provided a pharmaceutical composition, comprising the fusion polypeptide, the polynucleotide, the gene expression cassette or the gene delivery vector provided in the present disclosure, and optionally a pharmaceutically acceptable carrier.

In some embodiments, the pharmaceutical composition according to the present disclosure contains, as an active ingredient, the fusion polypeptide, the polynucleotide or the gene expression cassette as described above or the gene delivery vector as described above.

In some specific embodiments, the pharmaceutical composition according to the present disclosure contains the gene delivery vector as an active ingredient, and the gene delivery vector is a recombinant adeno-associated virus. In these embodiments, the pharmaceutical composition comprises about 1×10⁸ to about 1×10¹⁵ viral genome (vg), about 1×10⁹ to about 1×10¹⁴ vg, about 1×10⁹ to about 1×10¹³ vg, e.g., 6.0 ×10⁹ vg.

A single dose is generally not less than a dose required to produce a measurable effect in a subject and can be determined based on the pharmacokinetics and pharmacology of the absorption, distribution, metabolism, and excretion ("ADME") of the pharmaceutical composition or byproducts thereof and consequently determined based on the disposition of the composition in the subject. This involves consideration of the route of administration and the dose. An effective dose and/or dosage regimen can be readily determined empirically, based on preclinical assays, safety and dose escalation and range trials, individual clinician-patient relationships, and *in vitro* and *in vivo* assays.

As used herein, the term "pharmaceutically acceptable carrier" includes saline, solvents, dispersion media, coatings, antibacterial and antifungal agents, isotonic and absorption delaying agents, and the like, compatible with pharmaceutical administration.

Pharmaceutical compositions are typically formulated to be compatible with its intended route of administration. Examples of routes of administration include parenteral, e.g., intravenous, intraarterial, subcutaneous, intraperitoneal, intrathecal, and intramuscular administration via either injection or infusion. Delivery can thus be systemic or localized. For example, in order to deliver to the retina, subretinal or intravitreal injection may be taken (see for example Ochakovski et al., Front Neurosci. 2017; 11: 174; Xue et al., Eye (Lond). 2017 Sep; 31(9):1308-1316).

In some specific embodiments, the pharmaceutical composition of the present disclosure is designed, engineered, or adapted for administration to primates (e.g., non-human primate and human subjects) via intravitreal or subretinal injection.

Methods of formulating suitable pharmaceutical compositions are known in the art, see, *e.g.,* Remington: The Science and Practice of Pharmacy, 21st ed., 2005; and the series Drugs and the Pharmaceutical Sciences: a Series of Textbooks and Monographs (Dekker, NY).

### <Use and Methods>

In some aspects of the present disclosure, the present disclosure provides a method for treating or preventing a disease (e.g., an eye disease) in a subject in need thereof, the method comprising administering, to the subject in need thereof, an effective amount of the fusion polypeptide, polynucleotide, gene expression cassette, gene delivery vector or pharmaceutical composition of the present disclosure.

In other aspects of the present disclosure, the present disclosure further provides use of the fusion polypeptide, polynucleotide, gene expression cassette or gene delivery vector of the present disclosure in the preparation of a medicament for treating a disease (e.g., an eye disease).

In some specific embodiments, the gene expression cassette, gene delivery vector or pharmaceutical composition of the present disclosure can at least partially ameliorate eye diseases associated with the ocular neovascularization or with the choroidal neovascularization (CNV). In some embodiments, the gene expression cassette, gene delivery vector or pharmaceutical composition (the coding sequence of the fusion polypeptide) of the present disclosure can be delivered to the eye of a human subject.

The approved indications for the aflibercept fusion protein includes neovascular (wet) age-related macular degeneration (AMD), macular edema secondary to retinal vein occlusion (RVO), diabetic macular edema (DME), and diabetic retinopathy (DR). In some cases, the method and the gene expression cassette, gene delivery vector or pharmaceutical composition of the present disclosure may be used for prevention or treatment of eye diseases for which aflibercept has been approved or is indicated.

In some specific embodiments, the eye disease may be choroidal neovascularization (also referred to as wet AMD). The choroidal neovascularization may involve the growth of new blood vessels that originate from the choroid, extend through a rupture in the Bruch membrane into the sub-retinal pigment epithelium (sub-RPE) or sub-retinal space, which can be a leading cause of vision loss. CNV can cause a sudden deterioration of central vision, which is obvious within several weeks. Other symptoms may include color disturbances and metamorphopsia (a visual distortion where straight lines look wavy). Bleeding from new blood vessels can accelerate the onset of CNV symptoms. CNV may also include a feeling of pressure behind the eye. In some embodiments, the method and the gene expression cassette, gene delivery vector or pharmaceutical composition according to the present disclosure are used for treatment of CNV or eye conditions associated with neovascularization.

The late-stage "wet" (neovascular or exudative) form of AMD, though less common, can frequently cause rapid and often substantial loss of central vision in patients. In a "wet" form of AMD, choroidal neovascularization forms and develops into a network of blood vessels that can grow beneath and across the retinal pigment epithelium. As this is accompanied by plasma leakage and/or hemorrhage into the sub-retinal space, severe sudden loss of central vision may occur if the macula is involved. The term "AMD", unless otherwise specified, may be dry AMD or wet AMD. The present disclosure contemplates treatment or prevention of AMD, wet AMD and/or dry AMD. In some embodiments, the method and the gene expression cassette, gene delivery vector or pharmaceutical composition according to the present disclosure are used for treatment of AMD.

In some embodiments, the method and the gene expression cassette, gene delivery vector or pharmaceutical composition according to the present disclosure are used for prevention or treatment of eye diseases or conditions responsive to aflibercept *in vivo.*

In some specific embodiments, the gene expression cassette, gene delivery vector or pharmaceutical composition may be parenterally administered via intravenous injection or oral infusion. In some more specific embodiments, the gene expression cassette, gene delivery vector or pharmaceutical composition is administered via injection to the eye, e.g., to the retina, sub-retinal space, or vitreous humor. In other more specific embodiments, the gene expression cassette, gene delivery vector or pharmaceutical composition is administered via retinal injection, sub-retinal injection, or intravitreal injection. In other more specific embodiments, the gene expression cassette, gene delivery vector or pharmaceutical composition is topically or directly administered to the tissue or organ of interest, for example, via injection into the liver. In some embodiments, the method comprises a single administration; in other embodiments, multiple administrations may be performed over time whenever an attending clinician considers it appropriate.

The subject may be a mammal including, for example, a human subject in need of treatment of a particular disease (e.g., an eye disease associated with ocular neovascularization or with choroidal neovascularization).

In some specific embodiments, if a gene delivery vector (a recombinant adeno-associated virus) is administered to a subject, the therapeutically effective amount used to achieve a change or produce a therapeutic effect in the subject may be about 1×10⁸ viral genome (vg) or more, and in some cases about 1×10⁹, 1×10¹⁰, 1×10¹¹, 1×10¹² or 1×10¹³ viral genome or more.

### Examples

The present disclosure will be described below in greater details with reference to the specific embodiments. The examples described below are merely intended to illustrate the present disclosure, rather than to limit its scope. The examples provided below may serve as guidance for further improvements by one of ordinary skill in the art and are not intended to limit the present disclosure in any way.

Unless otherwise specified, the experimental methods in the following examples are all conventional methods and are carried out according to the techniques or conditions described in the literatures in the related art or according to the product instructions. All the materials, reagents and the like used in the following examples are commercially available, unless otherwise specified.

### Materials and Methods

HEK 293T cell line (ATCC, CRL-3216);
HEK 293 cell line (purchased from Chinese Academy of Sciences);
Poly ethylenimine Linear (PEI) MW40000 (YEASEN, 40816ES03);
Serological Pipets (Jet Bio-Filtration);
Benzonase (Sigma-Aldrich, E8263-25KU);
c57 mice (purchased from Beijing Vital River Laboratory Animal Technology Co., Ltd.).

### Example 1: Construction of Plasmid Vectors for Codon-Optimized Aflibercept

To construct AAV vector plasmids (i.e., gene delivery vectors) carrying an aflibercept expression cassette (a gene expression cassette), an expression vector for aflibercept was first constructed in this example, followed by codon optimization.

The unoptimized aflibercept sequence (Aflibercept opt) and two artificially synthesized codon-optimized aflibercept coding sequences (Aflibercept opt2 and Aflibercept opt3) were respectively substituted for the Fluc sequence in the applicant's deposited AAV vector plasmid pssAAV-CMV-Fluc-hGH pA (FIG. 1A) carrying the Firefly Luciferase (Fluc) reporter gene expression cassette, to obtain pssAAV-CMV-Aflibercept (FIG. 1B), pssAAV-CMV-Aflibercept opt2 (FIG. 1C), and pssAAV-CMV-Aflibercept opt3 (FIG. 1D).

The specific construction procedures were as follows.

### (1) Codon-optimized aflibercept sequence

Based on the patent CN114375195A published by Adverum Biotechnologies, Inc., the aflibercept expression cassette and the coding sequence of aflibercept used by this corporation for construction of AAV vectors were obtained, and the coding sequence was used as an unoptimized sequence, i.e., the Aflibercept sequence (SEQ ID NO: 20). The sequence in the protein coding region was further codon-optimized to obtain two optimized aflibercept (Coding optimized Recombined Aflibercept, Aflibercept opt) sequences, namely, the Aflibercept opt 2 sequence (SEQ ID NO: 22) and the Aflibercept opt3 sequence (SEQ ID NO: 23).
SEQ ID NO: 20 (the nucleotide sequence of Aflibercept) 
5'ATGGTGTCATACTGGGATACTGGAGTCTTGCTTTGTGCCCTGCTGTCCTGCCT
CCTCCTGACTGGCTCCAGCTCGGGCTCAGATACCGGTCGCCCCTTCGTGGAGATGTA
CTCCGAGATCCCGGAAATTATCCACATGACTGAGGGGCGCGAACTTGTGATCCCCTG
CCGGGTCACCAGCCCGAACATTACTGTGACTTTGAAGAAGTTCCCCCTGGACACCC
TGATTCCGGATGGGAAGAGAATTATCTGGGATTCACGGAAGGGATTCATCATCAGCA
ACGCGACCTACAAGGAAATTGGCCTCCTCACTTGCGAAGCCACTGTGAACGGACAC
TTGTACAAGACCAACTACCTGACCCACCGCCAGACCAACACCATCATCGACGTCGT
CCTGTCCCCTTCGCACGGGATCGAGCTCTCGGTGGGAGAGAAGTTGGTGCTTAACT
GCACCGCCCGGACGGAACTGAATGTGGGAATCGACTTCAACTGGGAATACCCGTCC
AGCAAGCATCAGCATAAGAAGCTGGTGAACCGGGACCTCAAGACTCAGTCCGGCA
GCGAAATGAAGAAGTTCCTGTCGACCCTCACTATTGACGGAGTGACCAGATCCGAC
CAGGGCCTCTACACTTGCGCCGCTTCCAGCGGACTCATGACCAAGAAGAACAGCAC
TTTCGTGAGGGTGCATGAGAAGGACAAGACCCACACGTGTCCGCCGTGCCCAGCC
CCAGAGCTGCTGGGAGGCCCTTCCGTGTTCCTGTTTCCGCCCAAGCCAAAGGATAC
CCTGATGATCTCAAGGACCCCTGAGGTCACATGCGTCGTGGTGGATGTGTCGCACG
AGGACCCTGAAGTCAAATTCAATTGGTATGTGGACGGAGTGGAAGTCCACAACGCG
AAAACCAAGCCGAGAGAAGAACAGTACAATTCCACCTACCGGGTGGTGTCGGTGC
TGACTGTGCTGCACCAGGACTGGCTCAACGGAAAGGAGTACAAGTGCAAGGTGTC
CAACAAGGCTCTGCCCGCACCTATTGAAAAGACCATCTCCAAGGCCAAGGGTCAAC
CTCGCGAGCCTCAGGTGTACACTCTGCCTCCAAGCCGGGACGAACTGACTAAGAAC
CAAGTCTCTCTGACCTGTTTGGTGAAGGGCTTCTACCCGTCAGACATCGCAGTGGA
GTGGGAGTCAAACGGTCAGCCGGAGAACAACTACAAAACAACCCCCCCCGTGCTG
GACTCCGACGGCTCCTTCTTCCTGTACTCCAAGCTTACCGTGGATAAGAGCCGCTGG
CAACAGGGCAACGTGTTTTCCTGCTCCGTCATGCACGAAGCCCTGCACAACCATTAT
 ACCCAGAAGTCCCTGTCGCTGTCCCCCGGGAAATAG3'
SEQ ID NO: 21 (the amino acid sequence of Aflibercept) 
MVSYWDTGVLLCALLSCLLLTGSSSGSDTGRPFVEMYSEIPEIIHMTEGRELVIPCR
VTSPNITVTLKKFPLDTLIPDGKRIIWDSRKGFIISNATYKEIGLLTCEATVNGHLYKTNY
LTHRQTNTIIDVVLSPSHGIELSVGEKLVLNCTARTELNVGIDFNWEYPSSKHQHKKLV
NRDLKTQSGSEMKKFLSTLTIDGVTRSDQGLYTCAASSGLMTKKNSTFVRVHEKDKTH
TCPPCPAPELLGGPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPAPIEKTISKAK
GQPREPQVYTLPPSRDELTKNQVSLTCLVKGFYPSDIAVEWESNGQPENNYKTTPPVLD
SDGSFFLYSKLTVDKSRWQQGNVFSCSVMHEALHNHYTQKSLSLSPGK*
SEQ ID NO: 22 (the nucleotide sequence of Aflibercept opt2) 
5'ATGGTGTCATACTGGGATACTGGAGTCTTGCTTTGTGCCCTGCTGTCCTGCCT
CCTCCTGACTGGCTCCAGCTCGGGCTCTGATACAGGCAGACCCTTTGTGGAAATGT
ACTCTGAGATTCCTGAAATCATCCACATGACAGAGGGCAGAGAACTGGTGATCCCA
TGCAGAGTGACCAGCCCTAACATCACAGTGACACTGAAGAAGTTCCCACTGGACA
CCCTGATCCCTGATGGAAAAAGAATCATTTGGGACAGCAGGAAGGGCTTTATCATC
AGTAATGCCACCTACAAAGAGATAGGCCTGCTCACATGTGAAGCTACAGTGAATGG
CCACCTGTATAAAACAAACTACCTGACCCATAGACAGACCAATACCATCATAGATGT
GGTGCTGAGCCCCAGCCATGGAATAGAGCTGTCTGTGGGGGAGAAGCTGGTACTGA
ACTGCACTGCCAGAACTGAGCTTAATGTGGGCATAGACTTCAATTGGGAGTACCCC
AGCTCCAAGCACCAACACAAGAAGCTGGTCAACAGGGACCTGAAGACCCAGAGT
GGCTCTGAAATGAAAAAGTTTCTGAGCACCCTGACAATAGATGGTGTTACAAGGAG
TGATCAGGGCCTGTACACCTGTGCAGCTTCTAGTGGCCTGATGACCAAAAAGAACA
GCACCTTTGTGAGGGTGCATGAGAAGGACAAGACACATACCTGCCCTCCATGTCCT
GCCCCTGAACTGCTGGGAGGACCTAGTGTGTTTCTGTTCCCCCCCAAGCCTAAGGA
CACCCTGATGATCAGCAGGACCCCTGAGGTGACATGTGTGGTGGTGGATGTGTCTC
ATGAGGACCCAGAGGTGAAGTTCAACTGGTATGTGGATGGGGTGGAAGTGCACAAT
GCCAAAACCAAGCCCAGAGAGGAACAGTACAACAGCACATACAGGGTGGTGTCTG
TGCTGACAGTCCTGCACCAGGATTGGCTGAATGGAAAGGAATACAAGTGCAAGGTG
TCCAACAAGGCCCTGCCTGCTCCTATAGAGAAGACCATCAGCAAGGCCAAGGGCCA
GCCTAGAGAGCCTCAGGTGTACACCCTGCCTCCCTCCAGAGATGAGCTGACAAAGA
ACCAGGTGTCCCTGACTTGCCTGGTCAAGGGCTTCTACCCTAGTGACATAGCAGTG
GAATGGGAAAGCAATGGCCAACCTGAGAACAACTACAAGACAACCCCCCCAGTGT
 
TGGATTCAGATGGCAGCTTCTTCCTGTACAGCAAGCTGACAGTGGACAAAAGCAGG
TGGCAGCAGGGCAATGTGTTCAGCTGCAGTGTGATGCATGAGGCCCTGCACAATCA
CTACACACAGAAAAGCCTGAGCCTGTCTCCTGGCAAATGA3’
SEQ ID NO: 23 (the nucleotide sequence of Aflibercept opt3)

5’ATGGTGTCATACTGGGATACTGGAGTCTTGCTTTGTGCCCTGCTGTCCTGCCT
CCTCCTGACTGGCTCCAGCTCGGGCTCTGACACAGGCAGACCCTTTGTGGAAATGT
ACTCTGAGATCCCTGAGATCATCCACATGACAGAGGGCAGAGAGCTGGTCATCCCC
TGCAGAGTGACAAGCCCCAACATCACAGTGACCCTGAAGAAGTTCCCTCTGGACA
CACTGATCCCTGATGGCAAGAGAATCATCTGGGACAGCAGAAAGGGCTTCATCATC
AGCAATGCCACCTACAAAGAGATTGGCCTGCTGACCTGTGAAGCCACAGTGAATGG
CCACCTGTACAAGACCAACTACCTGACACACAGACAGACCAACACCATCATTGATG
TGGTGCTGAGCCCCAGCCATGGCATTGAGCTGTCTGTGGGAGAGAAGCTGGTGCTG
AACTGCACAGCCAGAACAGAGCTGAATGTGGGCATTGACTTCAACTGGGAGTACCC
CAGCAGCAAGCACCAGCACAAGAAACTGGTCAACAGGGACCTGAAAACCCAGAG
TGGCTCTGAGATGAAGAAATTCCTGAGCACCCTGACCATTGATGGGGTCACCAGAT
CTGACCAGGGCCTGTACACATGTGCTGCCAGCTCTGGACTGATGACCAAGAAAAAC
AGCACCTTTGTCAGAGTGCATGAGAAGGACAAGACCCACACCTGTCCTCCATGTCC
TGCACCTGAGCTGCTTGGAGGCCCCTCTGTGTTCCTGTTTCCTCCAAAGCCTAAGG
ACACCCTGATGATCAGCAGAACACCTGAAGTGACCTGTGTGGTGGTGGATGTGTCC
CATGAGGACCCAGAAGTGAAGTTCAATTGGTATGTGGATGGGGTTGAAGTGCACAA
TGCCAAGACCAAGCCTAGAGAGGAACAGTACAACTCCACCTACAGAGTGGTGTCA
GTGCTGACAGTGCTGCACCAGGACTGGCTGAATGGCAAAGAGTACAAGTGCAAGG
TGTCCAACAAGGCCCTGCCTGCTCCTATTGAGAAAACCATCTCCAAGGCCAAGGGC
CAGCCTAGGGAACCCCAGGTTTACACACTGCCACCTAGCAGGGATGAGCTGACAAA
GAACCAGGTGTCCCTGACCTGCCTGGTCAAGGGCTTCTACCCCTCTGACATTGCTGT
GGAATGGGAGAGCAATGGCCAGCCTGAGAACAACTACAAGACAACCCCTCCTGTG
CTGGACTCTGATGGCTCATTCTTCCTGTACAGCAAGCTGACTGTGGACAAGAGCAG
ATGGCAGCAGGGAAATGTGTTCAGCTGCTCTGTGATGCATGAGGCCCTGCACAACC
ACTACACCCAGAAAAGCCTGAGCCTGTCTCCTGGCAAGTGA3’

### (2) Construction of AAV vectors carrying the Aflibercept/Aflibercept opt2/Aflibercept opt3 expression cassette

The "5'-GCCACC-3'" sequence (Kozak sequence) was added to the 5' end of Aflibercept (SEQ ID NO: 20), Aflibercept opt2 (SEQ ID NO: 22), and Aflibercept opt3 (SEQ ID NO: 23). Subsequently, these sequences were provided to General Biosystems (Anhui) Corp. Ltd. for synthesis and seamless cloning into the applicant's deposited vector plasmid pssAAV-CMV-Fluc-hGH pA to replace Fluc therein, to obtain pssAAV-CMV-Aflibercept, pssAAV-CMV-Aflibercept opt2, and pssAAV-CMV-Aflibercept opt3.

The expression cassettes (i.e., the gene expression cassettes) carried in the above three plasmid vectors were CMV-Aflibercept-hGH pA (SEQ ID NO: 24), CMV-Aflibercept opt2-hGH pA (SEQ ID NO: 12), and CMV-Aflibercept opt3-hGH pA (SEQ ID NO: 56), respectively.
SEQ ID NO: 24 (the nucleotide sequence of CMV-Aflibercept-hGH pA) 
5’ACGCGTCTAGTTATTAATAGTAATCAATTACGGGGTCATTAGTTCATAGCCCAT
ATATGGAGTTCCGCGTTACATAACTTACGGTAAATGGCCCGCCTGGCTGACCGCCCA
ACGACCCCCGCCCATTGACGTCAATAATGACGTATGTTCCCATAGTAACGCCAATAG
GGACTTTCCATTGACGTCAATGGGTGGAGTATTTACGGTAAACTGCCCACTTGGCAG
TACATCAAGTGTATCATATGCCAAGTACGCCCCCTATTGACGTCAATGACGGTAAATG
GCCCGCCTGGCATTATGCCCAGTACATGACCTTATGGGACTTTCCTACTTGGCAGTA
CATCTACGTATTAGTCATCGCTATTACCATGGTGATGCGGTTTTGGCAGTACATCAAT
GGGCGTGGATAGCGGTTTGACTCACGGGGATTTCCAAGTCTCCACCCCATTGACGT
CAATGGGAGTTTGTTTTGGCACCAAAATCAACGGGACTTTCCAAAATGTCGTAACA
ACTCCGCCCCATTGACGCAAATGGGCGGTAGGCGTGTACGGTGGGAGGTCTATATAA
GCAGAGCTCGTTTAGTGAACCGTCAGATCGCCTGGAGACGCCATCCACGCTGTTTT
GACCTCCATAGAAGACACCGGGACCGATCCAGCCTCCGCGGATTCGAATCCCGGCC
GGGAACGGTGCATTGGAACGCGGATTCCCCGTGCCAAGAGTGACGTAAGTACCGCC
TATAGAGTCTATAGGCCCACAAAAAATGCTTTCTTCTTTTAATATACTTTTTTGTTTAT
CTTATTTCTAATACTTTCCCTAATCTCTTTCTTTCAGGGCAATAATGATACAATGTATC
ATGCCTCTTTGCACCATTCTAAAGAATAACAGTGATAATTTCTGGGTTAAGGCAATAG
CAATATTTCTGCATATAAATATTTCTGCATATAAATTGTAACTGATGTAAGAGGTTTCA
TATTGCTAATAGCAGCTACAATCCAGCTACCATTCTGCTTTTATTTTATGGTTGGGATA
AGGCTGGATTATTCTGAGTCCAAGCTAGGCCCTTTTGCTAATCATGTTCATACCTCTT
ATCTTCCTCCCACAGCTCCTGGGCAACGTGCTGGTCTGTGTGCTGGCCCATCACTTT
GGCAAAGAATTGGGATAATTACTAGATCGCCACCATGGTGTCATACTGGGATACTGG
AGTCTTGCTTTGTGCCCTGCTGTCCTGCCTCCTCCTGACTGGCTCCAGCTCGGGCTC
AGATACCGGTCGCCCCTTCGTGGAGATGTACTCCGAGATCCCGGAAATTATCCACAT
GACTGAGGGGCGCGAACTTGTGATCCCCTGCCGGGTCACCAGCCCGAACATTACTG
TGACTTTGAAGAAGTTCCCCCTGGACACCCTGATTCCGGATGGGAAGAGAATTATCT
 
GGGATTCACGGAAGGGATTCATCATCAGCAACGCGACCTACAAGGAAATTGGCCTC
CTCACTTGCGAAGCCACTGTGAACGGACACTTGTACAAGACCAACTACCTGACCCA
CCGCCAGACCAACACCATCATCGACGTCGTCCTGTCCCCTTCGCACGGGATCGAGC
TCTCGGTGGGAGAGAAGTTGGTGCTTAACTGCACCGCCCGGACGGAACTGAATGTG
GGAATCGACTTCAACTGGGAATACCCGTCCAGCAAGCATCAGCATAAGAAGCTGGT
GAACCGGGACCTCAAGACTCAGTCCGGCAGCGAAATGAAGAAGTTCCTGTCGACC
CTCACTATTGACGGAGTGACCAGATCCGACCAGGGCCTCTACACTTGCGCCGCTTC
CAGCGGACTCATGACCAAGAAGAACAGCACTTTCGTGAGGGTGCATGAGAAGGAC
AAGACCCACACGTGTCCGCCGTGCCCAGCCCCAGAGCTGCTGGGAGGCCCTTCCG
TGTTCCTGTTTCCGCCCAAGCCAAAGGATACCCTGATGATCTCAAGGACCCCTGAG
GTCACATGCGTCGTGGTGGATGTGTCGCACGAGGACCCTGAAGTCAAATTCAATTG
GTATGTGGACGGAGTGGAAGTCCACAACGCGAAAACCAAGCCGAGAGAAGAACA
GTACAATTCCACCTACCGGGTGGTGTCGGTGCTGACTGTGCTGCACCAGGACTGGC
TCAACGGAAAGGAGTACAAGTGCAAGGTGTCCAACAAGGCTCTGCCCGCACCTATT
GAAAAGACCATCTCCAAGGCCAAGGGTCAACCTCGCGAGCCTCAGGTGTACACTCT
GCCTCCAAGCCGGGACGAACTGACTAAGAACCAAGTCTCTCTGACCTGTTTGGTGA
AGGGCTTCTACCCGTCAGACATCGCAGTGGAGTGGGAGTCAAACGGTCAGCCGGA
GAACAACTACAAAACAACCCCCCCCGTGCTGGACTCCGACGGCTCCTTCTTCCTGT
ACTCCAAGCTTACCGTGGATAAGAGCCGCTGGCAACAGGGCAACGTGTTTTCCTGC
TCCGTCATGCACGAAGCCCTGCACAACCATTATACCCAGAAGTCCCTGTCGCTGTCC
CCCGGGAAATAGGCGGCCGCAAGCTTCGGGTGGCATCCCTGTGACCCCTCCCCAGT
GCCTCTCCTGGCCCTGGAAGTTGCCACTCCAGTGCCCACCAGCCTTGTCCTAATAAA
ATTAAGTTGCATCATTTTGTCTGACTAGGTGTCCTTCTATAATATTATGGGGTGGAGG
GGGGTGGTATGGAGCAAGGGGCAAGTTGGGAAGACAACCTGTAGGGCCTGCGGGG
TCTATTGGGAACCAAGCTGGAGTGCAGTGGCACAATCTTGGCTCACTGCAATCTCC
GCCTCCTGGGTTCAAGCGATTCTCCTGCCTCAGCCTCCCGAGTTGTTGGGATTCCAG
GCATGCATGACCAGGCTCAGCTAATTTTTGTTTTTTTGGTAGAGACGGGGTTTCACC
ATATTGGCCAGGCTGGTCTCCAACTCCTAATCTCAGGTGATCTACCCACCTTGGCCT
CCCAAATTGCTGGGATTACAGGCGTGAACCACTGCTCCCTTCCCTGTCCTTCGG3’
SEQ ID NO: 12 (the nucleotide sequence of CMV-Aflibercept opt2-hGH pA) 
5'ACGCGTCTAGTTATTAATAGTAATCAATTACGGGGTCATTAGTTCATAGCCCAT
ATATGGAGTTCCGCGTTACATAACTTACGGTAAATGGCCCGCCTGGCTGACCGCCCA
ACGACCCCCGCCCATTGACGTCAATAATGACGTATGTTCCCATAGTAACGCCAATAG
GGACTTTCCATTGACGTCAATGGGTGGAGTATTTACGGTAAACTGCCCACTTGGCAG
 
TACATCAAGTGTATCATATGCCAAGTACGCCCCCTATTGACGTCAATGACGGTAAATG
GCCCGCCTGGCATTATGCCCAGTACATGACCTTATGGGACTTTCCTACTTGGCAGTA
CATCTACGTATTAGTCATCGCTATTACCATGGTGATGCGGTTTTGGCAGTACATCAAT
GGGCGTGGATAGCGGTTTGACTCACGGGGATTTCCAAGTCTCCACCCCATTGACGT
CAATGGGAGTTTGTTTTGGCACCAAAATCAACGGGACTTTCCAAAATGTCGTAACA
ACTCCGCCCCATTGACGCAAATGGGCGGTAGGCGTGTACGGTGGGAGGTCTATATAA
GCAGAGCTCGTTTAGTGAACCGTCAGATCGCCTGGAGACGCCATCCACGCTGTTTT
GACCTCCATAGAAGACACCGGGACCGATCCAGCCTCCGCGGATTCGAATCCCGGCC
GGGAACGGTGCATTGGAACGCGGATTCCCCGTGCCAAGAGTGACGTAAGTACCGCC
TATAGAGTCTATAGGCCCACAAAAAATGCTTTCTTCTTTTAATATACTTTTTTGTTTAT
CTTATTTCTAATACTTTCCCTAATCTCTTTCTTTCAGGGCAATAATGATACAATGTATC
ATGCCTCTTTGCACCATTCTAAAGAATAACAGTGATAATTTCTGGGTTAAGGCAATAG
CAATATTTCTGCATATAAATATTTCTGCATATAAATTGTAACTGATGTAAGAGGTTTCA
TATTGCTAATAGCAGCTACAATCCAGCTACCATTCTGCTTTTATTTTATGGTTGGGATA
AGGCTGGATTATTCTGAGTCCAAGCTAGGCCCTTTTGCTAATCATGTTCATACCTCTT
ATCTTCCTCCCACAGCTCCTGGGCAACGTGCTGGTCTGTGTGCTGGCCCATCACTTT
GGCAAAGAATTGGGATAATTACTAGATCGCCACCATGGTGTCATACTGGGATACTGG
AGTCTTGCTTTGTGCCCTGCTGTCCTGCCTCCTCCTGACTGGCTCCAGCTCGGGCTC
TGATACAGGCAGACCCTTTGTGGAAATGTACTCTGAGATTCCTGAAATCATCCACAT
GACAGAGGGCAGAGAACTGGTGATCCCATGCAGAGTGACCAGCCCTAACATCACA
GTGACACTGAAGAAGTTCCCACTGGACACCCTGATCCCTGATGGAAAAAGAATCAT
TTGGGACAGCAGGAAGGGCTTTATCATCAGTAATGCCACCTACAAAGAGATAGGCC
TGCTCACATGTGAAGCTACAGTGAATGGCCACCTGTATAAAACAAACTACCTGACC
CATAGACAGACCAATACCATCATAGATGTGGTGCTGAGCCCCAGCCATGGAATAGAG
CTGTCTGTGGGGGAGAAGCTGGTACTGAACTGCACTGCCAGAACTGAGCTTAATGT
GGGCATAGACTTCAATTGGGAGTACCCCAGCTCCAAGCACCAACACAAGAAGCTG
GTCAACAGGGACCTGAAGACCCAGAGTGGCTCTGAAATGAAAAAGTTTCTGAGCA
CCCTGACAATAGATGGTGTTACAAGGAGTGATCAGGGCCTGTACACCTGTGCAGCT
TCTAGTGGCCTGATGACCAAAAAGAACAGCACCTTTGTGAGGGTGCATGAGAAGG
ACAAGACACATACCTGCCCTCCATGTCCTGCCCCTGAACTGCTGGGAGGACCTAGT
GTGTTTCTGTTCCCCCCCAAGCCTAAGGACACCCTGATGATCAGCAGGACCCCTGA
GGTGACATGTGTGGTGGTGGATGTGTCTCATGAGGACCCAGAGGTGAAGTTCAACT
GGTATGTGGATGGGGTGGAAGTGCACAATGCCAAAACCAAGCCCAGAGAGGAACA
GTACAACAGCACATACAGGGTGGTGTCTGTGCTGACAGTCCTGCACCAGGATTGGC
TGAATGGAAAGGAATACAAGTGCAAGGTGTCCAACAAGGCCCTGCCTGCTCCTATA
 
GAGAAGACCATCAGCAAGGCCAAGGGCCAGCCTAGAGAGCCTCAGGTGTACACCC
TGCCTCCCTCCAGAGATGAGCTGACAAAGAACCAGGTGTCCCTGACTTGCCTGGTC
AAGGGCTTCTACCCTAGTGACATAGCAGTGGAATGGGAAAGCAATGGCCAACCTGA
GAACAACTACAAGACAACCCCCCCAGTGTTGGATTCAGATGGCAGCTTCTTCCTGT
ACAGCAAGCTGACAGTGGACAAAAGCAGGTGGCAGCAGGGCAATGTGTTCAGCTG
CAGTGTGATGCATGAGGCCCTGCACAATCACTACACACAGAAAAGCCTGAGCCTGT
CTCCTGGCAAATGAGCGGCCGCAAGCTTCGGGTGGCATCCCTGTGACCCCTCCCCA
GTGCCTCTCCTGGCCCTGGAAGTTGCCACTCCAGTGCCCACCAGCCTTGTCCTAATA
AAATTAAGTTGCATCATTTTGTCTGACTAGGTGTCCTTCTATAATATTATGGGGTGGA
GGGGGGTGGTATGGAGCAAGGGGCAAGTTGGGAAGACAACCTGTAGGGCCTGCGG
GGTCTATTGGGAACCAAGCTGGAGTGCAGTGGCACAATCTTGGCTCACTGCAATCT
CCGCCTCCTGGGTTCAAGCGATTCTCCTGCCTCAGCCTCCCGAGTTGTTGGGATTCC
AGGCATGCATGACCAGGCTCAGCTAATTTTTGTTTTTTTGGTAGAGACGGGGTTTCA
CCATATTGGCCAGGCTGGTCTCCAACTCCTAATCTCAGGTGATCTACCCACCTTGGC
CTCCCAAATTGCTGGGATTACAGGCGTGAACCACTGCTCCCTTCCCTGTCCTTCGG3’
SEQ ID NO: 56 (the nucleotide sequence of CMV-Aflibercept opt3-hGH pA)

5'ACGCGTCTAGTTATTAATAGTAATCAATTACGGGGTCATTAGTTCATAGCCCAT
ATATGGAGTTCCGCGTTACATAACTTACGGTAAATGGCCCGCCTGGCTGACCGCCCA
ACGACCCCCGCCCATTGACGTCAATAATGACGTATGTTCCCATAGTAACGCCAATAG
GGACTTTCCATTGACGTCAATGGGTGGAGTATTTACGGTAAACTGCCCACTTGGCAG
TACATCAAGTGTATCATATGCCAAGTACGCCCCCTATTGACGTCAATGACGGTAAATG
GCCCGCCTGGCATTATGCCCAGTACATGACCTTATGGGACTTTCCTACTTGGCAGTA
CATCTACGTATTAGTCATCGCTATTACCATGGTGATGCGGTTTTGGCAGTACATCAAT
GGGCGTGGATAGCGGTTTGACTCACGGGGATTTCCAAGTCTCCACCCCATTGACGT
CAATGGGAGTTTGTTTTGGCACCAAAATCAACGGGACTTTCCAAAATGTCGTAACA
ACTCCGCCCCATTGACGCAAATGGGCGGTAGGCGTGTACGGTGGGAGGTCTATATAA
GCAGAGCTCGTTTAGTGAACCGTCAGATCGCCTGGAGACGCCATCCACGCTGTTTT
GACCTCCATAGAAGACACCGGGACCGATCCAGCCTCCGCGGATTCGAATCCCGGCC
GGGAACGGTGCATTGGAACGCGGATTCCCCGTGCCAAGAGTGACGTAAGTACCGCC
TATAGAGTCTATAGGCCCACAAAAAATGCTTTCTTCTTTTAATATACTTTTTTGTTTAT
CTTATTTCTAATACTTTCCCTAATCTCTTTCTTTCAGGGCAATAATGATACAATGTATC
ATGCCTCTTTGCACCATTCTAAAGAATAACAGTGATAATTTCTGGGTTAAGGCAATAG
CAATATTTCTGCATATAAATATTTCTGCATATAAATTGTAACTGATGTAAGAGGTTTCA
TATTGCTAATAGCAGCTACAATCCAGCTACCATTCTGCTTTTATTTTATGGTTGGGATA

AGGCTGGATTATTCTGAGTCCAAGCTAGGCCCTTTTGCTAATCATGTTCATACCTCTT
ATCTTCCTCCCACAGCTCCTGGGCAACGTGCTGGTCTGTGTGCTGGCCCATCACTTT
GGCAAAGAATTGGGATAATTACTAGATCGCCACCATGGTGTCATACTGGGATACTGG
AGTCTTGCTTTGTGCCCTGCTGTCCTGCCTCCTCCTGACTGGCTCCAGCTCGGGCTC
TGACACAGGCAGACCCTTTGTGGAAATGTACTCTGAGATCCCTGAGATCATCCACAT
GACAGAGGGCAGAGAGCTGGTCATCCCCTGCAGAGTGACAAGCCCCAACATCACA
GTGACCCTGAAGAAGTTCCCTCTGGACACACTGATCCCTGATGGCAAGAGAATCAT
CTGGGACAGCAGAAAGGGCTTCATCATCAGCAATGCCACCTACAAAGAGATTGGCC
TGCTGACCTGTGAAGCCACAGTGAATGGCCACCTGTACAAGACCAACTACCTGACA
CACAGACAGACCAACACCATCATTGATGTGGTGCTGAGCCCCAGCCATGGCATTGA
GCTGTCTGTGGGAGAGAAGCTGGTGCTGAACTGCACAGCCAGAACAGAGCTGAAT
GTGGGCATTGACTTCAACTGGGAGTACCCCAGCAGCAAGCACCAGCACAAGAAAC
TGGTCAACAGGGACCTGAAAACCCAGAGTGGCTCTGAGATGAAGAAATTCCTGAG
CACCCTGACCATTGATGGGGTCACCAGATCTGACCAGGGCCTGTACACATGTGCTG
CCAGCTCTGGACTGATGACCAAGAAAAACAGCACCTTTGTCAGAGTGCATGAGAA
GGACAAGACCCACACCTGTCCTCCATGTCCTGCACCTGAGCTGCTTGGAGGCCCCT
CTGTGTTCCTGTTTCCTCCAAAGCCTAAGGACACCCTGATGATCAGCAGAACACCT
GAAGTGACCTGTGTGGTGGTGGATGTGTCCCATGAGGACCCAGAAGTGAAGTTCAA
TTGGTATGTGGATGGGGTTGAAGTGCACAATGCCAAGACCAAGCCTAGAGAGGAAC
AGTACAACTCCACCTACAGAGTGGTGTCAGTGCTGACAGTGCTGCACCAGGACTGG
CTGAATGGCAAAGAGTACAAGTGCAAGGTGTCCAACAAGGCCCTGCCTGCTCCTAT
TGAGAAAACCATCTCCAAGGCCAAGGGCCAGCCTAGGGAACCCCAGGTTTACACA
CTGCCACCTAGCAGGGATGAGCTGACAAAGAACCAGGTGTCCCTGACCTGCCTGGT
CAAGGGCTTCTACCCCTCTGACATTGCTGTGGAATGGGAGAGCAATGGCCAGCCTG
AGAACAACTACAAGACAACCCCTCCTGTGCTGGACTCTGATGGCTCATTCTTCCTGT
ACAGCAAGCTGACTGTGGACAAGAGCAGATGGCAGCAGGGAAATGTGTTCAGCTG
CTCTGTGATGCATGAGGCCCTGCACAACCACTACACCCAGAAAAGCCTGAGCCTGT
CTCCTGGCAAGTGAGCGGCCGCAAGCTTCGGGTGGCATCCCTGTGACCCCTCCCCA
GTGCCTCTCCTGGCCCTGGAAGTTGCCACTCCAGTGCCCACCAGCCTTGTCCTAATA
AAATTAAGTTGCATCATTTTGTCTGACTAGGTGTCCTTCTATAATATTATGGGGTGGA
GGGGGGTGGTATGGAGCAAGGGGCAAGTTGGGAAGACAACCTGTAGGGCCTGCGG
GGTCTATTGGGAACCAAGCTGGAGTGCAGTGGCACAATCTTGGCTCACTGCAATCT
CCGCCTCCTGGGTTCAAGCGATTCTCCTGCCTCAGCCTCCCGAGTTGTTGGGATTCC
AGGCATGCATGACCAGGCTCAGCTAATTTTTGTTTTTTTGGTAGAGACGGGGTTTCA
CCATATTGGCCAGGCTGGTCTCCAACTCCTAATCTCAGGTGATCTACCCACCTTGGC

CTCCCAAATTGCTGGGATTACAGGCGTGAACCACTGCTCCCTTCCCTGTCCTTCGG3'

### Example 2: Comparison of Expression Levels of Two Codon-Optimized Aflibercept Sequences

To determine that the codon optimization could enhance the expression level of aflibercept, the pssAAV-CMV-Aflibercept, pssAAV-CMV-Aflibercept opt2, and pssAAV-CMV-Aflibercept opt3 constructed in Example 1 were separately transfected *in vitro* into HEK293 cells using lipo3000 (Thermo Fisher) in this example. Cell supernatants were collected 48 h after transfection, and aflibercept in the cell supernatants was assayed by the Elisa method. The kits were purchased from ImmunoGuide (Turkey), Cat. No.: IG-AA115. The experimental results showed that the Aflibercept opt3 sequence enabled the expression level of aflibercept to be enhanced (FIG. 2).

### Example 3: Construction of Aflibercept Plasmid Vectors with Modified Secretory Signal

The results in Example 2 demonstrated that Aflibercept opt3 showed the highest expression level. Therefore, Aflibercept opt3 was employed for further studies in all the subsequence examples.

To increase the secretion of aflibercept, its native secretory signal peptide was replaced. The native secretory signal peptide sequence of aflibercept in the above-mentioned plasmid was replaced respectively with artificially synthesized human albumin secretory signal peptide (hAlbuminSP or hAlbumin sp for short) sequence, human Opticin protein secretory signal peptide (hOpticinSP or hOpticin sp for short) sequence, human interleukin-2 secretory signal peptide (hIL-2SP or hIL2sp for short) sequence, and mouse receptor tyrosine kinase-like orphan receptor 1 secretory signal peptide (mouse ROR1 protein secretory signal peptide, mROR1SP or mROR1 sp for short) sequence, to obtain the following sequences: hAlbumin sp-Aflibercept opt3, hOpticin sp-Aflibercept opt3, hIL2sp-Aflibercept opt3, and mROR1 sp-Aflibercept opt3. Those sequences were respectively substituted for the Fluc sequence in the applicant's deposited pssAAV-CMV-Fluc-hGH pA (FIG. 1A), pssAAV-CBh-Fluc-hGH pA (FIG. 3A), and pssAAV-CB7-Fluc-hGH pA (FIG. 3B), to obtain the following plasmid vectors: pssAAV-CMV-hAlbumin sp-Aflibercept opt3 (FIG. 3C), pssAAV-CBh-hAlbumin sp-Aflibercept opt3 (FIG. 3D), and pssAAV-CB7-hAlbumin sp-Aflibercept opt3 (FIG. 3E); pssAAV-CMV-hOpticin sp-Aflibercept opt3 (FIG. 3F), pssAAV-CBh-hOpticin sp-Aflibercept opt3 (FIG. 3G), and pssAAV-CB7-hOpticin sp-Aflibercept opt3 (FIG. 3H); pssAAV-CMV-hIL2sp-Aflibercept opt3 (FIG. 31), pssAAV-CBh-hIL2sp-Aflibercept opt3 (FIG. 3J), and pssAAV-CB7-hIL2sp-Aflibercept opt3 (FIG. 3K); and pssAAV-CMV-mROR1 sp-Aflibercept opt3 (FIG. 3L), pssAAV-CBh-mROR1 sp-Aflibercept opt3 (FIG. 3M), and pssAAV-CB7-mROR1 sp-Aflibercept opt3 (FIG. 3N).

Finally, based on the patent CN114375195A published by Adverum Biotechnologies, Inc., the aflibercept expression cassette used by this corporation for construction of AAV vectors was obtained. This expression cassette was substituted for CMV-Fluc-hGH pA in pssAAV-CMV-Fluc-hGH pA to obtain pssAAV-ADVM-022 (FIG. 3O).

The specific procedures of construction were as follows.

### (1) Aflibercept sequence with altered secretory signal peptide

Aflibercept was a secretory protein, and the first 26 amino acids at the N-terminus constituted the secretory signal peptide of aflibercept. In this Example, a human albumin secretory signal peptide (hAlbuminSP) sequence (5' end-ATGAAGTGGGTGACCTTCATCAGCCTGCTGTTCCTGTTTTCTTCAGCCTACAGC-3' end (SEQ ID NO: 25)), a human Opticin protein secretory signal peptide (hOpticinSP) sequence (5' end-ATGAGACTGCTGGCCTTCCTGAGCCTGCTGGCCCTGGTGCTGCAGGAGACAGG CACC-3' end (SEQ ID NO: 26)), a human interleukin-2 secretory signal peptide (hIL-2SP) sequence (5' end-ATGTACAGAATGCAGCTGCTGAGCTGCATAGCCCTGTCTCTGGCCCTGGTGACC AACAGC-3' end (SEQ ID NO: 27)), and a mouse receptor tyrosine kinase-like orphan receptor 1 secretory signal peptide (mROR1SP) sequence (5' end-ATGCACAGACCCAGAAGGAGGGGCACCAGACCTCCACCTCTGGCTCTGCTGGCAG CCCTGCTGCTGGCAGCTAGAGGAGCAGATGCC-3' end SEQ ID NO: 28)) were respectively substituted for the gene sequence of the secretory signal peptide of Aflibercept opt3, to obtain four recombinant aflibercept sequences, i.e., hAlbumin sp-Aflibercept opt3 (SEQ ID NO: 29), encoding the amino acid sequence SEQ ID NO: 30; hOpticin sp-Aflibercept opt3 (SEQ ID NO: 31), encoding the amino acid sequence SEQ ID NO: 32; hIL2sp-Aflibercept opt3 (SEQ ID NO: 2), encoding the amino acid sequence SEQ ID NO: 1, and mROR1 sp-Aflibercept opt3 (SEQ ID NO: 33), encoding the amino acid sequence SEQ ID NO: 34.
SEQ ID NO: 29 (the nucleotide sequence of hAlbumin sp-Aflibercept opt3) 
5’ATGAAGTGGGTGACCTTCATCAGCCTGCTGTTCCTGTTTTCTTCAGCCTACAG
CTCTGACACAGGCAGACCCTTTGTGGAAATGTACTCTGAGATCCCTGAGATCATCCA
CATGACAGAGGGCAGAGAGCTGGTCATCCCCTGCAGAGTGACAAGCCCCAACATC
ACAGTGACCCTGAAGAAGTTCCCTCTGGACACACTGATCCCTGATGGCAAGAGAAT
CATCTGGGACAGCAGAAAGGGCTTCATCATCAGCAATGCCACCTACAAAGAGATTG
 
GCCTGCTGACCTGTGAAGCCACAGTGAATGGCCACCTGTACAAGACCAACTACCTG
ACACACAGACAGACCAACACCATCATTGATGTGGTGCTGAGCCCCAGCCATGGCAT
TGAGCTGTCTGTGGGAGAGAAGCTGGTGCTGAACTGCACAGCCAGAACAGAGCTG
AATGTGGGCATTGACTTCAACTGGGAGTACCCCAGCAGCAAGCACCAGCACAAGA
AACTGGTCAACAGGGACCTGAAAACCCAGAGTGGCTCTGAGATGAAGAAATTCCT
GAGCACCCTGACCATTGATGGGGTCACCAGATCTGACCAGGGCCTGTACACATGTG
CTGCCAGCTCTGGACTGATGACCAAGAAAAACAGCACCTTTGTCAGAGTGCATGAG
AAGGACAAGACCCACACCTGTCCTCCATGTCCTGCACCTGAGCTGCTTGGAGGCCC
CTCTGTGTTCCTGTTTCCTCCAAAGCCTAAGGACACCCTGATGATCAGCAGAACACC
TGAAGTGACCTGTGTGGTGGTGGATGTGTCCCATGAGGACCCAGAAGTGAAGTTCA
ATTGGTATGTGGATGGGGTTGAAGTGCACAATGCCAAGACCAAGCCTAGAGAGGAA
CAGTACAACTCCACCTACAGAGTGGTGTCAGTGCTGACAGTGCTGCACCAGGACTG
GCTGAATGGCAAAGAGTACAAGTGCAAGGTGTCCAACAAGGCCCTGCCTGCTCCTA
TTGAGAAAACCATCTCCAAGGCCAAGGGCCAGCCTAGGGAACCCCAGGTTTACAC
ACTGCCACCTAGCAGGGATGAGCTGACAAAGAACCAGGTGTCCCTGACCTGCCTG
GTCAAGGGCTTCTACCCCTCTGACATTGCTGTGGAATGGGAGAGCAATGGCCAGCC
TGAGAACAACTACAAGACAACCCCTCCTGTGCTGGACTCTGATGGCTCATTCTTCCT
GTACAGCAAGCTGACTGTGGACAAGAGCAGATGGCAGCAGGGAAATGTGTTCAGC
TGCTCTGTGATGCATGAGGCCCTGCACAACCACTACACCCAGAAAAGCCTGAGCCT
GTCTCCTGGCAAGTGA3’
SEQ ID NO: 30 (the amino acid sequence of hAlbumin sp-Aflibercept opt3) 
MKWVTFISLLFLFSSAYSSDTGRPFVEMYSEIPEIIHMTEGRELVIPCRVTSPNITVTL
KKFPLDTLIPDGKRIIWDSRKGFIISNATYKEIGLLTCEATVNGHLYKTNYLTHRQTNTII
DVVLSPSHGIELSVGEKLVLNCTARTELNVGIDFNWEYPSSKHQHKKLVNRDLKTQSG
SEMKKFLSTLTIDGVTRSDQGLYTCAASSGLMTKKNSTFVRVHEKDKTHTCPPCPAPEL
LGGPSVFLFPPKPKDTLMISRTPEVTCVVVDVSHEDPEVKFNWYVDGVEVHNAKTKP
REEQYNSTYRVVSVLTVLHQDWLNGKEYKCKVSNKALPAPIEKTISKAKGQPREPQVY
TLPPSRDELTKNQVSLTCLVKGFYPSDIAVEWESNGQPENNYKTTPPVLDSDGSFFLYSK
LTVDKSRWQQGNVFSCSVMHEALHNHYTQKSLSLSPGK*
SEQ ID NO: 31 (the nucleotide sequence of hOpticin sp-Aflibercept opt3) 
5’ATGAGACTGCTGGCCTTCCTGAGCCTGCTGGCCCTGGTGCTGCAGGAGACAG
GCACCTCTGACACAGGCAGACCCTTTGTGGAAATGTACTCTGAGATCCCTGAGATC
ATCCACATGACAGAGGGCAGAGAGCTGGTCATCCCCTGCAGAGTGACAAGCCCCA
ACATCACAGTGACCCTGAAGAAGTTCCCTCTGGACACACTGATCCCTGATGGCAAG
AGAATCATCTGGGACAGCAGAAAGGGCTTCATCATCAGCAATGCCACCTACAAAGA
 
GATTGGCCTGCTGACCTGTGAAGCCACAGTGAATGGCCACCTGTACAAGACCAACT
ACCTGACACACAGACAGACCAACACCATCATTGATGTGGTGCTGAGCCCCAGCCAT
GGCATTGAGCTGTCTGTGGGAGAGAAGCTGGTGCTGAACTGCACAGCCAGAACAG
AGCTGAATGTGGGCATTGACTTCAACTGGGAGTACCCCAGCAGCAAGCACCAGCAC
AAGAAACTGGTCAACAGGGACCTGAAAACCCAGAGTGGCTCTGAGATGAAGAAAT
TCCTGAGCACCCTGACCATTGATGGGGTCACCAGATCTGACCAGGGCCTGTACACA
TGTGCTGCCAGCTCTGGACTGATGACCAAGAAAAACAGCACCTTTGTCAGAGTGCA
TGAGAAGGACAAGACCCACACCTGTCCTCCATGTCCTGCACCTGAGCTGCTTGGAG
GCCCCTCTGTGTTCCTGTTTCCTCCAAAGCCTAAGGACACCCTGATGATCAGCAGAA
CACCTGAAGTGACCTGTGTGGTGGTGGATGTGTCCCATGAGGACCCAGAAGTGAA
GTTCAATTGGTATGTGGATGGGGTTGAAGTGCACAATGCCAAGACCAAGCCTAGAG
AGGAACAGTACAACTCCACCTACAGAGTGGTGTCAGTGCTGACAGTGCTGCACCA
GGACTGGCTGAATGGCAAAGAGTACAAGTGCAAGGTGTCCAACAAGGCCCTGCCT
GCTCCTATTGAGAAAACCATCTCCAAGGCCAAGGGCCAGCCTAGGGAACCCCAGGT
TTACACACTGCCACCTAGCAGGGATGAGCTGACAAAGAACCAGGTGTCCCTGACCT
GCCTGGTCAAGGGCTTCTACCCCTCTGACATTGCTGTGGAATGGGAGAGCAATGGC
CAGCCTGAGAACAACTACAAGACAACCCCTCCTGTGCTGGACTCTGATGGCTCATT
CTTCCTGTACAGCAAGCTGACTGTGGACAAGAGCAGATGGCAGCAGGGAAATGTG
TTCAGCTGCTCTGTGATGCATGAGGCCCTGCACAACCACTACACCCAGAAAAGCCT
GAGCCTGTCTCCTGGCAAGTGA3'
SEQ ID NO: 32 (the amino acid sequence of hOpticin sp-Aflibercept opt3) 
MRLLAFLSLLALVLQETGTSDTGRPFVEMYSEIPEIIHMTEGRELVIPCRVTSPNITV
TLKKFPLDTLIPDGKRIIWDSRKGFIISNATYKEIGLLTCEATVNGHLYKTNYLTHRQTNT
IIDVVLSPSHGIELSVGEKLVLNCTARTELNVGIDFNWEYPSSKHQHKKLVNRDLKTQS
GSEMKKFLSTLTIDGVTRSDQGLYTCAASSGLMTKKNSTFVRVHEKDKTHTCPPCPAPE
LLGGPSVFLFPPKPKDTLMISRTPEVTCVVVDVSHEDPEVKFNWYVDGVEVHNAKTKP
REEQYNSTYRVVSVLTVLHQDWLNGKEYKCKVSNKALPAPIEKTISKAKGQPREPQVY
TLPPSRDELTKNQVSLTCLVKGFYPSDIAVEWESNGQPENNYKTTPPVLDSDGSFFLYSK
LTVDKSRWQQGNVFSCSVMHEALHNHYTQKSLSLSPGK*
SEQ ID NO: 33 (the nucleotide sequence of mROR1 sp-Aflibercept opt3) 
5’ATGCACAGACCCAGAAGGAGGGGCACCAGACCTCCACCTCTGGCTCTGCTG
GCAGCCCTGCTGCTGGCAGCTAGAGGAGCAGATGCCTCTGACACAGGCAGACCCTT
TGTGGAAATGTACTCTGAGATCCCTGAGATCATCCACATGACAGAGGGCAGAGAGC
TGGTCATCCCCTGCAGAGTGACAAGCCCCAACATCACAGTGACCCTGAAGAAGTTC
CCTCTGGACACACTGATCCCTGATGGCAAGAGAATCATCTGGGACAGCAGAAAGGG
 
CTTCATCATCAGCAATGCCACCTACAAAGAGATTGGCCTGCTGACCTGTGAAGCCA
CAGTGAATGGCCACCTGTACAAGACCAACTACCTGACACACAGACAGACCAACAC
CATCATTGATGTGGTGCTGAGCCCCAGCCATGGCATTGAGCTGTCTGTGGGAGAGA
AGCTGGTGCTGAACTGCACAGCCAGAACAGAGCTGAATGTGGGCATTGACTTCAA
CTGGGAGTACCCCAGCAGCAAGCACCAGCACAAGAAACTGGTCAACAGGGACCTG
AAAACCCAGAGTGGCTCTGAGATGAAGAAATTCCTGAGCACCCTGACCATTGATGG
GGTCACCAGATCTGACCAGGGCCTGTACACATGTGCTGCCAGCTCTGGACTGATGA
CCAAGAAAAACAGCACCTTTGTCAGAGTGCATGAGAAGGACAAGACCCACACCTG
TCCTCCATGTCCTGCACCTGAGCTGCTTGGAGGCCCCTCTGTGTTCCTGTTTCCTCC
AAAGCCTAAGGACACCCTGATGATCAGCAGAACACCTGAAGTGACCTGTGTGGTG
GTGGATGTGTCCCATGAGGACCCAGAAGTGAAGTTCAATTGGTATGTGGATGGGGT
TGAAGTGCACAATGCCAAGACCAAGCCTAGAGAGGAACAGTACAACTCCACCTAC
AGAGTGGTGTCAGTGCTGACAGTGCTGCACCAGGACTGGCTGAATGGCAAAGAGT
ACAAGTGCAAGGTGTCCAACAAGGCCCTGCCTGCTCCTATTGAGAAAACCATCTCC
AAGGCCAAGGGCCAGCCTAGGGAACCCCAGGTTTACACACTGCCACCTAGCAGGG
ATGAGCTGACAAAGAACCAGGTGTCCCTGACCTGCCTGGTCAAGGGCTTCTACCCC
TCTGACATTGCTGTGGAATGGGAGAGCAATGGCCAGCCTGAGAACAACTACAAGAC
AACCCCTCCTGTGCTGGACTCTGATGGCTCATTCTTCCTGTACAGCAAGCTGACTGT
GGACAAGAGCAGATGGCAGCAGGGAAATGTGTTCAGCTGCTCTGTGATGCATGAG
GCCCTGCACAACCACTACACCCAGAAAAGCCTGAGCCTGTCTCCTGGCAAGTGA3’
SEQ ID NO: 34 (the amino acid sequence of mROR1 sp-Aflibercept opt3)

MHRPRRRGTRPPPLALLAALLLAARGADASDTGRPFVEMYSEIPEIIHMTEGRELV
IPCRVTSPNITVTLKKFPLDTLIPDGKRIIWDSRKGFIISNATYKEIGLLTCEATVNGHLYK
TNYLTHRQTNTIIDVVLSPSHGIELSVGEKLVLNCTARTELNVGIDFNWEYPSSKHQHK
KLVNRDLKTQSGSEMKKFLSTLTIDGVTRSDQGLYTCAASSGLMTKKNSTFVRVHEKD
KTHTCPPCPAPELLGGPSVFLFPPKPKDTLMISRTPEVTCVVVDVSHEDPEVKFNWYVD
GVEVHNAKTKPREEQYNSTYRVVSVLTVLHQDWLNGKEYKCKVSNKALPAPIEKTIS
KAKGQPREPQVYTLPPSRDELTKNQVSLTCLVKGFYPSDIAVEWESNGQPENNYKTTPP
VLDSDGSFFLYSKLTVDKSRWQQGNVFSCSVMHEALHNHYTQKSLSLSPGK*

### (2) Construction of AAV vectors carrying hAlbumin sp-Aflibercept opt3, hOpticin sp-Aflibercept opt3, hIL2sp-Aflibercept opt3, or mROR1 sp-Aflibercept opt3 expression cassette

The "5'-GCCACC-3'" sequence was added to the 5' end of hAlbumin sp-Aflibercept opt3 (SEQ ID NO: 29), hOpticin sp-Aflibercept opt3 (SEQ ID NO: 31), hIL2sp-Aflibercept opt3 (SEQ ID NO: 2), and mROR1 sp-Aflibercept opt3 (SEQ ID NO: 33), respectively. Subsequently, these sequences were provided to General Biosystems (Anhui) Corp. Ltd. for synthesis and seamless cloning into the applicant's deposited three plasmid vectors pssAAV-CMV-Fluc-hGH pA, pssAAV-CBh-Fluc-hGH pA, and pssAAV-CB7-Fluc-hGH pA to replace Fluc therein, to obtain pssAAV-CMV-hAlbumin sp-Aflibercept opt3, pssAAV-CBh-hAlbumin sp-Aflibercept opt3, and pssAAV-CB7-hAlbumin sp-Aflibercept opt3; and pssAAV-CMV-hOpticin sp-Aflibercept opt3, pssAAV-CBh-hOpticin sp-Aflibercept opt3, and pssAAV-CB7-hOpticin sp-Aflibercept opt3; and pssAAV-CMV-hIL2sp-Aflibercept opt3, pssAAV-CBh-hIL2sp-Aflibercept opt3, and pssAAV-CB7-hIL2sp-Aflibercept opt3; and pssAAV-CMV-mROR1 sp-Aflibercept opt3, pssAAV-CBh-mROR1 sp-Aflibercept opt3, and pssAAV-CB7-mROR1 sp-Aflibercept opt3.

The expression cassettes (i.e., the gene expression cassettes) carried in the above 12 plasmid vectors were CMV-hAlbumin sp-Aflibercept opt3-hGH pA (SEQ ID NO: 35), CBh-hAlbumin sp-Aflibercept opt3-hGH pA (SEQ ID NO: 36), and CB7-hAlbumin sp-Aflibercept opt3-hGH pA (SEQ ID NO: 37); and CMV-hOpticin sp-Aflibercept opt3-hGH pA (SEQ ID NO: 38), CBh-hOpticin sp-Aflibercept opt3-hGH pA (SEQ ID NO: 39), and CB7-hOpticin sp-Aflibercept opt3-hGH pA (SEQ ID NO: 40); and CMV-hIL2sp-Aflibercept opt3-hGH pA (SEQ ID NO: 8), CBh-hIL2sp-Aflibercept opt3-hGH pA (SEQ ID NO: 9), and CB7-hIL2sp-Aflibercept opt3-hGH pA (SEQ ID NO: 10); and CMV-mROR1 sp-Aflibercept opt3-hGH pA (SEQ ID NO: 41), CBh-mROR1 sp-Aflibercept opt3-hGH pA (SEQ ID NO: 42), and CB7-mROR1 sp-Aflibercept opt3-hGH pA (SEQ ID NO: 43), respectively.

### (3) Construction of AAV vectors carrying the aflibercept expression cassette for comparative studies

To package AAVs used for comparative studies in subsequent examples, the aflibercept expression cassette (SEQ ID NO: 52), designated as ADVM-022 here, used for constructing the AAV vectors was obtained in this example based on the patent CN114375195A published by Adverum Biotechnologies, Inc. The sequence was provided to General Biosystems (Anhui) Corp. Ltd. for synthesis and seamless cloning into the applicant's deposited plasmid pssAAV-CMV-Fluc-hGH pA. This expression cassette was substituted for CMV-Flue-hGH pA in pssAAV-CMV-Flue-hGH pA to obtain pssAAV-ADVM-022 (FIG. 3O).
SEQ ID NO: 52 (the nucleotide sequence of ADVM-022 expression cassette)

5’ACTTACGGTAAATGGCCCGCCTGGCTGACCGCCCAACGACCCCCGCCCATTG
ACGTCAATAATGACGTATGTTCCCATAGTAACGCCAATAGGGACTTTCCATTGACGTC
AATGGGTGGAGTATTTACGGTAAACTGCCCACTTGGCAGTACATCAAGTGTATCATA
TGCCAAGTCCGCCCCCTATTGACGTCAATGACGGTAAATGGCCCGCCTGGCATTATG
CCCAGTACATGACCTTACGGGACTTTCCTACTTGGCAGTACATCTACGTATTAGTCAT

CGCTATTACCATGCTGATGCGGTTTTGGCAGTACACCAATGGGCGTGGATAGCGGTT
TGACTCACGGGGATTTCCAAGTCTCCACCCCATTGACGTCAATGGGAGTTTGTTTTG
GCACCAAAATCAACGGGACTTTCCAAAATGTCGTAATAACCCCGCCCCGTTGACGC
AAATGGGCGGTAGGCGTGTACGGTGGGAGGTCTATATAAGCAGAGCTCGTTTAGTG
AACCGTCAGATCGCCTGGAGAGGCCATCCACGCTGTTTTGACCTCCATAGTGGACA
CCGGGACCGATCCAGCCTCCGCGTCTCAGGGGAGATCTCGTTTAGTGAACCGTCAG
ATCCTCACTCTCTTCCGCATCGCTGTCTGCGAGGGCCAGCTGTTGGGCTCGCGGTTG
AGGACAAACTCTTCGCGGTCTTTCCAGTACTCTTGGATCGGAAACCCGTCGGCCTC
CGAACGGTACTCCGCCACCGAGGGACCTGAGCGAGTCCGCATCGACCGGATCGGA
AAACCTCTCGAGAAAGGCGTCTAACCAGTCACAGTCGCAAGGTAGGCTGAGCACC
GTGGCGGGCGGCAGCGGGTGGCGGTCGGGGTTGTTTCTGGCGGAGGTGCTGCTGA
TGATGTAATTAAAGTAGGCGGTCTTGAGACGGCGGATGGTCGAGGTGAGGTGTGGC
AGGCTTGAGATCCAGCTGTTGGGGTGAGTACTCCCTCTCAAAAGCGGGCATTACTT
CTGCGCTAAGATTGTCAGTTTCCAAAAACGAGGAGGATTTGATATTCACCTGGCCCG
ATCTGGCCATACACTTGAGTGACAATGACATCCACTTTGCCTTTCTCTCCACAGGTG
TCCACTCCCAGGTCCAAGTTTAAACGCCGCCACCATGGTGTCATACTGGGATACTGG
AGTCTTGCTTTGTGCCCTGCTGTCCTGCCTCCTCCTGACTGGCTCCAGCTCGGGCTC
AGATACCGGTCGCCCCTTCGTGGAGATGTACTCCGAGATCCCGGAAATTATCCACAT
GACTGAGGGGCGCGAACTTGTGATCCCCTGCCGGGTCACCAGCCCGAACATTACTG
TGACTTTGAAGAAGTTCCCCCTGGACACCCTGATTCCGGATGGGAAGAGAATTATCT
GGGATTCACGGAAGGGATTCATCATCAGCAACGCGACCTACAAGGAAATTGGCCTC
CTCACTTGCGAAGCCACTGTGAACGGACACTTGTACAAGACCAACTACCTGACCCA
CCGCCAGACCAACACCATCATCGACGTCGTCCTGTCCCCTTCGCACGGGATCGAGC
TCTCGGTGGGAGAGAAGTTGGTGCTTAACTGCACCGCCCGGACGGAACTGAATGTG
GGAATCGACTTCAACTGGGAATACCCGTCCAGCAAGCATCAGCATAAGAAGCTGGT
GAACCGGGACCTCAAGACTCAGTCCGGCAGCGAAATGAAGAAGTTCCTGTCGACC
CTCACTATTGACGGAGTGACCAGATCCGACCAGGGCCTCTACACTTGCGCCGCTTC
CAGCGGACTCATGACCAAGAAGAACAGCACTTTCGTGAGGGTGCATGAGAAGGAC
AAGACCCACACGTGTCCGCCGTGCCCAGCCCCAGAGCTGCTGGGAGGCCCTTCCG
TGTTCCTGTTTCCGCCCAAGCCAAAGGATACCCTGATGATCTCAAGGACCCCTGAG
GTCACATGCGTCGTGGTGGATGTGTCGCACGAGGACCCTGAAGTCAAATTCAATTG
GTATGTGGACGGAGTGGAAGTCCACAACGCGAAAACCAAGCCGAGAGAAGAACA
GTACAATTCCACCTACCGGGTGGTGTCGGTGCTGACTGTGCTGCACCAGGACTGGC
TCAACGGAAAGGAGTACAAGTGCAAGGTGTCCAACAAGGCTCTGCCCGCACCTATT
GAAAAGACCATCTCCAAGGCCAAGGGTCAACCTCGCGAGCCTCAGGTGTACACTCT

GCCTCCAAGCCGGGACGAACTGACTAAGAACCAAGTCTCTCTGACCTGTTTGGTGA
AGGGCTTCTACCCGTCAGACATCGCAGTGGAGTGGGAGTCAAACGGTCAGCCGGA
GAACAACTACAAAACAACCCCCCCCGTGCTGGACTCCGACGGCTCCTTCTTCCTGT
ACTCCAAGCTTACCGTGGATAAGAGCCGCTGGCAACAGGGCAACGTGTTTTCCTGC
TCCGTCATGCACGAAGCCCTGCACAACCATTATACCCAGAAGTCCCTGTCGCTGTCC
CCCGGGAAATAGTGACTGTTCTCATCACATCATATCAAGGTTATATACCATCAATATT
GCCACAGATGTTACTTAGCCTTTTAATATTTCTCTAATTTAGTGTATATGCAATGATAG
TTCTCTGATTTCTGAGATTGAGTTTCTCATGTGTAATGATTATTTAGAGTTTCTCTTTC
ATCTGTTCAAATTTTTGTCTAGTTTTATTTTTTACTGATTTGTAAGACTTCTTTTTATAA
TCTGCATATTACAATTCTCTTTACTGGGGTGTTGCAAATATTTTCTGTCATTCTATGGC
CTGACTTTTCTTAATGGTTTTTTAATTTTAAAAATAAGTCTTAATATTCATGCAATCTA
ATTAACAATCTTTTCTTTGTGGTTAGGACTTTGAGTCATAAGAAATTTTTCTCTACAC
TGAAGTCATGATGGCATGCTTCTATATTATTTTCTAAAAGATTTAAAGTTTTGCCTTCT
CCATTTAGACTTATAATTCACTGGAATTTTTTTGTGTGTATGGTATGACATATGGGTTC
CCTTTTATTTTTTACATATAAATATATTTCCCTGTTTTTCTAAAAAAGAAAAAGATCAT
CATTTTCCCATTGTAAAATGCCATATTTTTTTCATAGGTCACTTACATATATCAATGGG
TCTGTTTCTGAGCTCTACTCTATTTTATCAGCCTCACTGTCTATCCCCACACATCTCAT
GCTTTGCTCTAAATCTTGATATTTAGTGGAACATTCTTTCCCATTTTGTTCTACAAGAA
TATTTTTGTTATTGTCTTTGGGCTTTCTATATACATTTTGAAATGAGGTTGACAAGTTA
CCTAGGAAAACTGTCTTCCTGCCCGGGTGGCATCCCTGTGACCCCTCCCCAGTGCCT
CTCCTGGCCCTGGAAGTTGCCACTCCAGTGCCCACCAGCCTTGTCCTAATAAAATTA
AGTTGCATCATTTTGTCTGACTAGGTGTCCTTCTATAATATTATGGGGTGGAGGGGGG
TGGTATGGAGCAAGGGGCCCAAGTTGGGAAGAAACCTGTAGGGCCTGCG3’

### Example 4: Comparison of Four Secretory Signal Peptides

To determine that altering the secretory signal peptide could enhance the secretion level of the recombinant aflibercept, pssAAV-CMV-Aflibercept opt3, pssAAV-CMV-hAlbumin sp-Aflibercept opt3, pssAAV-CMV-hOpticin sp-Aflibercept opt3, pssAAV-CMV-hIL2sp-Aflibercept opt3, and pssAAV-CMV-mROR1 sp-Aflibercept opt3 constructed in Example 1 or Example 3 were respectively transfected *in vitro* into HEK293 cells using lipo3000 (ThermoFisher) in this example. Cell supernatants (diluted 5-fold with sterile PBS) and cells were harvested 48 h after transfection. After the cells were lysed using Ripa lysate (Beyotime, Beijing), the total protein was extracted. After the protein concentration was determined, the total protein concentrations in the cells were normalized. Aflibercept in both the cell supernatant and the total cell protein was assayed by Western blot. Because aflibercept had an Fc structure of an antibody, WB was conducted only with an HRP-tagged anti-Fc antibody (Jackson ImmunoResearch, USA), without adding a secondary antibody. The experimental results showed that all the four modified secretory signals could significantly improve the secretion of aflibercept; and the human interleukin-2 and mouse ROR1 protein secretory signal peptides were more effective (FIG. 4).

### Example 5: Design and Construction of AAV Capsid Protein Expression Plasmid pAAV-RC2_IVT

### 1. Construction of RC2_IVB-NotI plasmid as intermediate plasmid

In this example, a reverse P5 promoter sequence was added upstream of the *Rep* sequence in pAAV-RC2 (purchased from CellBiolabs, Cat. No.: VPK-410-SER2), and a NotI restriction endonuclease site was inserted at 1752 bp of the *Cap2* sequence. General Biosystems (Anhui) Corp. Ltd. was commissioned to construct this intermediate plasmid RC2_IVB-NotI. The specific sequence was as shown below:
RC2_IVB-NotI plasmid vector sequence (SEQ ID NO: 13)

GCGCGCCGATATCTTCAAACCTCCCGCTTCAAAATGGAGACCCTGCGTGCTCAC
TCGGGCCCCCCCCCCCAGCGTGACCACATGGTGTCGCAAAATGTCGCAAAACACTC
ACGTGACCTCTAATACAGGACCTCCCTAACCCTATGACGTAATTCACGTCACGACTC
CACCCCTCCGTTAACGCCCCGCGCCGGCCGCTCTAGAACTAGTGGATCCCCCGGAA
GATCAGAAGTTCCTATTCCGAAGTTCCTATTCTCTAGAAAGTATAGGAACTTCTGATC
TGCGCAGCCGCCATGCCGGGGTTTTACGAGATTGTGATTAAGGTCCCCAGCGACCTT
GACGAGCATCTGCCCGGCATTTCTGACAGCTTTGTGAACTGGGTGGCCGAGAAGGA
ATGGGAGTTGCCGCCAGATTCTGACATGGATCTGAATCTGATTGAGCAGGCACCCCT
GACCGTGGCCGAGAAGCTGCAGCGCGACTTTCTGACGGAATGGCGCCGTGTGAGT
AAGGCCCCGGAGGCCCTTTTCTTTGTGCAATTTGAGAAGGGAGAGAGCTACTTCCA
CATGCACGTGCTCGTGGAAACCACCGGGGTGAAATCCATGGTTTTGGGACGTTTCC
TGAGTCAGATTCGCGAAAAACTGATTCAGAGAATTTACCGCGGGATCGAGCCGACT
TTGCCAAACTGGTTCGCGGTCACAAAGACCAGAAATGGCGCCGGAGGCGGGAACA
AGGTGGTGGATGAGTGCTACATCCCCAATTACTTGCTCCCCAAAACCCAGCCTGAG
CTCCAGTGGGCGTGGACTAATATGGAACAGTATTTAAGCGCCTGTTTGAATCTCACG
GAGCGTAAACGGTTGGTGGCGCAGCATCTGACGCACGTGTCGCAGACGCAGGAGC
AGAACAAAGAGAATCAGAATCCCAATTCTGATGCGCCGGTGATCAGATCAAAAACT
TCAGCCAGGTACATGGAGCTGGTCGGGTGGCTCGTGGACAAGGGGATTACCTCGGA
GAAGCAGTGGATCCAGGAGGACCAGGCCTCATACATCTCCTTCAATGCGGCCTCCA
ACTCGCGGTCCCAAATCAAGGCTGCCTTGGACAATGCGGGAAAGATTATGAGCCTG

ACTAAAACCGCCCCCGACTACCTGGTGGGCCAGCAGCCCGTGGAGGACATTTCCAG
CAATCGGATTTATAAAATTTTGGAACTAAACGGGTACGATCCCCAATATGCGGCTTCC
GTCTTTCTGGGATGGGCCACGAAAAAGTTCGGCAAGAGGAACACCATCTGGCTGTT
TGGGCCTGCAACTACCGGGAAGACCAACATCGCGGAGGCCATAGCCCACACTGTGC
CCTTCTACGGGTGCGTAAACTGGACCAATGAGAACTTTCCCTTCAACGACTGTGTC
GACAAGATGGTGATCTGGTGGGAGGAGGGGAAGATGACCGCCAAGGTCGTGGAGT
CGGCCAAAGCCATTCTCGGAGGAAGCAAGGTGCGCGTGGACCAGAAATGCAAGTC
CTCGGCCCAGATAGACCCGACTCCCGTGATCGTCACCTCCAACACCAACATGTGCG
CCGTGATTGACGGGAACTCAACGACCTTCGAACACCAGCAGCCGTTGCAAGACCG
GATGTTCAAATTTGAACTCACCCGCCGTCTGGATCATGACTTTGGGAAGGTCACCA
AGCAGGAAGTCAAAGACTTTTTCCGGTGGGCAAAGGATCACGTGGTTGAGGTGGA
GCATGAATTCTACGTCAAAAAGGGTGGAGCCAAGAAAAGACCCGCCCCCAGTGAC
GCAGATATAAGTGAGCCCAAACGGGTGCGCGAGTCAGTTGCGCAGCCATCGACGTC
AGACGCGGAAGCTTCGATCAACTACGCAGACAGGTACCAAAACAAATGTTCTCGTC
ACGTGGGCATGAATCTGATGCTGTTTCCCTGCAGACAATGCGAGAGAATGAATCAG
AATTCAAATATCTGCTTCACTCACGGACAGAAAGACTGTTTAGAGTGCTTTCCCGTG
TCAGAATCTCAACCCGTTTCTGTCGTCAAAAAGGCGTATCAGAAACTGTGCTACATT
CATCATATCATGGGAAAGGTGCCAGACGCTTGCACTGCCTGCGATCTGGTCAATGTG
GATTTGGATGACTGCATCTTTGAACAATAAATGATTTAAATCAGGTATGGCTGCCGAT
GGTTATCTTCCAGATTGGCTCGAGGACACTCTCTCTGAAGGAATAAGACAGTGGTG
GAAGCTCAAACCTGGCCCACCACCACCAAAGCCCGCAGAGCGGCATAAGGACGAC
AGCAGGGGTCTTGTGCTTCCTGGGTACAAGTACCTCGGACCCTTCAACGGACTCGA
CAAGGGAGAGCCGGTCAACGAGGCAGACGCCGCGGCCCTCGAGCACGACAAAGC
CTACGACCGGCAGCTCGACAGCGGAGACAACCCGTACCTCAAGTACAACCACGCC
GACGCGGAGTTTCAGGAGCGCCTTAAAGAAGATACGTCTTTTGGGGGCAACCTCGG
ACGAGCAGTCTTCCAGGCGAAAAAGAGGGTTCTTGAACCTCTGGGCCTGGTTGAG
GAACCTGTTAAGACGGCTCCGGGAAAAAAGAGGCCGGTAGAGCACTCTCCTGTGG
AGCCAGACTCCTCCTCGGGAACCGGAAAGGCGGGCCAGCAGCCTGCAAGAAAAA
GATTGAATTTTGGTCAGACTGGAGACGCAGACTCAGTACCTGACCCCCAGCCTCTC
GGACAGCCACCAGCAGCCCCCTCTGGTCTGGGAACTAATACGATGGCTACAGGCAG
TGGCGCACCAATGGCAGACAATAACGAGGGCGCCGACGGAGTGGGTAATTCCTCG
GGAAATTGGCATTGCGATTCCACATGGATGGGCGACAGAGTCATCACCACCAGCAC
CCGAACCTGGGCCCTGCCCACCTACAACAACCACCTCTACAAACAAATTTCCAGCC
AATCAGGAGCCTCGAACGACAATCACTACTTTGGCTACAGCACCCCTTGGGGGTATT
TTGACTTCAACAGATTCCACTGCCACTTTTCACCACGTGACTGGCAAAGACTCATC

AACAACAACTGGGGATTCCGACCCAAGAGACTCAACTTCAAGCTCTTTAACATTCA
AGTCAAAGAGGTCACGCAGAATGACGGTACGACGATTGCCAATAACCTTACCA
GCACGGTTCAGGTGTTTACTGACTCGGAGTACCAGCTCCCGTACGTCCTCGGCTCG
GCGCATCAAGGATGCCTCCCGCCGTTCCCAGCAGACGTCTTCATGGTGCCACAGTAT
GGATACCTCACCCTGAACAACGGGAGTCAGGCAGTAGGACGCTCTTCATTTTACTG
CCTGGAGTACTTTCCTTCTCAGATGCTGCGTACCGGAAACAACTTTACCTTCAGCTA
CACTTTTGAGGACGTTCCTTTCCACAGCAGCTACGCTCACAGCCAGAGTCTGGACC
GTCTCATGAATCCTCTCATCGACCAGTACCTGTATTACTTGAGCAGAACAAACACTC
CAAGTGGAACCACCACGCAGTCAAGGCTTCAGTTTTCTCAGGCCGGAGCGAGTGA
CATTCGGGACCAGTCTAGGAACTGGCTTCCTGGACCCTGTTACCGCCAGCAGCGAG
TATCAAAGACATCTGCGGATAACAACAACAGTGAATACTCGTGGACTGGAGCTACC
AAGTACCACCTCAATGGCAGAGACTCTCTGGTGAATCCGGGCCCGGCCATGGCAAG
CCACAAGGACGATGAAGAAAAGTTTTTTCCTCAGAGCGGGGTTCTCATCTTTGGGA
AGCAAGGCTCAGAGAAAACAAATGTGGACATTGAAAAGGTCATGATTACAGACGA
AGAGGAAATCAGGACAACCAATCCCGTGGCTACGGAGCAGTATGGTTCTGTATCTA
CCAACCTCCAGCGCGGCCGCAACAGACAAGCAGCTACCGCAGATGTCAACACACA
AGGCGTTCTTCCAGGCATGGTCTGGCAGGACAGAGATGTGTACCTTCAGGGGCCCA
TCTGGGCAAAGATTCCACACACGGACGGACATTTTCACCCCTCTCCCCTCATGGGT
GGATTCGGACTTAAACACCCTCCTCCACAGATTCTCATCAAGAACACCCCGGTACCT
GCGAATCCTTCGACCACCTTCAGTGCGGCAAAGTTTGCTTCCTTCATCACACAGTAC
TCCACGGGACAGGTCAGCGTGGAGATCGAGTGGGAGCTGCAGAAGGAAAACAGC
AAACGCTGGAATCCCGAAATTCAGTACACTTCCAACTACAACAAGTCTGTTAATGTG
GACTTTACTGTGGACACTAATGGCGTGTATTCAGAGCCTCGCCCCATTGGCACCAGA
TACCTGACTCGTAATCTGTAATTGCTTGTTAATCAATAAACCGTTTAATTCGTTTCAG
TTGAACTTTGGTCTCTGCGTATTTCTTTCTTATCTAGTTTCCATGGCTACGTAGATAAG
TAGCATGGCGGGTTAATCATTAACTACAGCCCGGGCGTTTAAACAGCGGGCGGAGG
GGTGGAGTCGTGACGTGAATTACGTCATAGGGTTAGGGAGGTCCTGTATTAGAGGTC
ACGTGAGTGTTTTGCGACATTTTGCGACACCATGTGGTCTCGCTGGGGGGGGGGGC
CCGAGTGAGCACGCAGGGTCTCCATTTTGAAGCGGGAGGTTTGAACGAGCGCTGG
CGCGCTCACTGGCCGTCGTTTTACAACGTCGTGACTGGGAAAACCCTGGCGTTACC
CAACTTAATCGCCTTGCAGCACATCCCCCTTTCGCCAGCTGGCGTAATAGCGAAGAG
GCCCGCACCGATCGCCCTTCCCAACAGTTGCGCAGCCTGAATGGCGAATGGAAATT
GTAAGCGTTAATATTTTGTTAAAATTCGCGTTAAATTTTTGTTAAATCAGCTCATTTTT
TTAACCAATAGGCCGAAATCGGCAAAATCCCTTATAAATCAAAAGAATAGACCGAGA
TAGGGTTGAGTGTTGTTCCAGTTTGGAACAAGAGTCCACTATTAAGAACGTGGACT

CCAACGTCAAAGGGCGAAAAACCGTCTATCAGGGCGATGGCCCACTACGTGAACCA
TCACCCTAATCAAGTTTTTTGGGGTCGAGGTGCCGTAAAGCACTAAATCGGAACCCT
AAAGGGAGCCCCCGATTTAGAGCTTGACGGGGAAAGCCGGCGAACGTGGCGAGAA
AGGAAGGGAAGAAAGCGAAAGGAGCGGGCGCTAGGGCGCTGGCAAGTGTAGCGG
TCACGCTGCGCGTAACCACCACACCCGCCGCGCTTAATGCGCCGCTACAGGGCGCG
TCAGGTGGCACTTTTCGGGGAAATGTGCGCGGAACCCCTATTTGTTTATTTTTCTAAA
TACATTCAAATATGTATCCGCTCATGAGACAATAACCCTGATAAATGCTTCAATAATAT
TGAAAAAGGAAGAGTATGAGTATTCAACATTTCCGTGTCGCCCTTATTCCCTTTTTTG
CGGCATTTTGCCTTCCTGTTTTTGCTCACCCAGAAACGCTGGTGAAAGTAAAAGATG
CTGAAGATCAGTTGGGTGCACGAGTGGGTTACATCGAACTGGATCTCAACAGCGGT
AAGATCCTTGAGAGTTTTCGCCCCGAAGAACGTTTTCCAATGATGAGCACTTTTAAA
GTTCTGCTATGTGGCGCGGTATTATCCCGTATTGACGCCGGGCAAGAGCAACTCGGT
CGCCGCATACACTATTCTCAGAATGACTTGGTTGAGTACTCACCAGTCACAGAAAA
GCATCTTACGGATGGCATGACAGTAAGAGAATTATGCAGTGCTGCCATAACCATGAG
TGATAACACTGCGGCCAACTTACTTCTGACAACGATCGGAGGACCGAAGGAGCTAA
CCGCTTTTTTGCACAACATGGGGGATCATGTAACTCGCCTTGATCGTTGGGAACCGG
AGCTGAATGAAGCCATACCAAACGACGAGCGTGACACCACGATGCCTGTAGCAATG
GCAACAACGTTGCGCAAACTATTAACTGGCGAACTACTTACTCTAGCTTCCCGGCA
ACAATTAATAGACTGGATGGAGGCGGATAAAGTTGCAGGACCACTTCTGCGCTCGG
CCCTTCCGGCTGGCTGGTTTATTGCTGATAAATCTGGAGCCGGTGAGCGTGGGTCTC
GCGGTATCATTGCAGCACTGGGGCCAGATGGTAAGCCCTCCCGTATCGTAGTTATCT
ACACGACGGGGAGTCAGGCAACTATGGATGAACGAAATAGACAGATCGCTGAGATA
GGTGCCTCACTGATTAAGCATTGGTAACTGTCAGACCAAGTTTACTCATATATACTTT
AGATTGATTTAAAACTTCATTTTTAATTTAAAAGGATCTAGGTGAAGATCCTTTTTGA
TAATCTCATGACCAAAATCCCTTAACGTGAGTTTTCGTTCCACTGAGCGTCAGACCC
CGTAGAAAAGATCAAAGGATCTTCTTGAGATCCTTTTTTTCTGCGCGTAATCTGCTG
CTTGCAAACAAAAAAACCACCGCTACCAGCGGTGGTTTGTTTGCCGGATCAAGAGC
TACCAACTCTTTTTCCGAAGGTAACTGGCTTCAGCAGAGCGCAGATACCAAATACTG
TTCTTCTAGTGTAGCCGTAGTTAGGCCACCACTTCAAGAACTCTGTAGCACCGCCTA
CATACCTCGCTCTGCTAATCCTGTTACCAGTGGCTGCTGCCAGTGGCGATAAGTCGT
GTCTTACCGGGTTGGACTCAAGACGATAGTTACCGGATAAGGCGCAGCGGTCGGGC
TGAACGGGGGGTTCGTGCACACAGCCCAGCTTGGAGCGAACGACCTACACCGAAC
TGAGATACCTACAGCGTGAGCTATGAGAAAGCGCCACGCTTCCCGAAGGGAGAAA
GGCGGACAGGTATCCGGTAAGCGGCAGGGTCGGAACAGGAGAGCGCACGAGGGA
GCTTCCAGGGGGAAACGCCTGGTATCTTTATAGTCCTGTCGGGTTTCGCCACCTCTG

ACTTGAGCGTCGATTTTTGTGATGCTCGTCAGGGGGGCGGAGCCTATGGAAAAACG
CCAGCAACGCGGCCTTTTTACGGTTCCTGGCCTTTTGCTGGCCTTTTGCTCACATGT
TCTTTCCTGCGTTATCCCCTGATTCTGTGGATAACCGTATTACCGCCTTTGAGTGAGC
TGATACCGCTCGCCGCAGCCGAACGACCGAGCGCAGCGAGTCAGTGAGCGAGGAA
GCGGAAGAGCGCCCAATACGCAAACCGCCTCTCCCCGCGCGTTGGCCGATTCATTA
ATGCAGCTGGCACGACAGGTTTCCCGACTGGAAAGCGGGCAGTGAGCGCAACGCA
ATTAATGTGAGTTAGCTCACTCATTAGGCACCCCAGGCTTTACACTTTATGCTTCCGG
CTCGTATGTTGTGTGGAATTGTGAGCGGATAACAATTTCACACAGGAAACAGCTATG
ACCATGATTACGCCAA

### 2. Construction of AAV capsid protein expression plasmid encoding capsid protein of interest

The AAV capsid protein expression plasmid encoding the capsid protein of interest was constructed by the Gibson assembly method (the specific steps could be found in Gibson Assembly^{®} Chemical Transformation Protocol (E2611)). The PCR fragment was connected by Gibson assembly to the fragment of the intermediate plasmid RC2_IVB-NotI constructed in step 1 by linearization with the NotI enzyme to obtain different AAV capsid plasmids encoding the capsid protein of interest. The PCR fragments were obtained by template-free PCR, and the primers were paired themselves to perform PCR amplification. The primer sequences were shown in Table 2 below.

**Table 2:**

| Vector No. | PCR Primer |
|---|---|
| RC2_IVT1 | Forward primer (SEQ ID NO: 84): |
| | AGCGGCTATTGTAGCCAGATCACCTCTGGCAGCAGCGTTGCCTCTCTGGAGGTTG GTAGA |
| | Reverse primer (SEQ ID NO: 85): |
| | GCTGCTGCCAGAGGTGATCTGGCTACAATAGCCGCTAGACAAGCAGCTACCGCA GATGTC |
| RC2_IVT2 | Forward primer (SEQ ID NO: 86): |
| | TGCAGCGAGGCGTCCCAAATCTCCTCTGGCTGCAGCGTTGCCTCTCTGGAGGTTG GTAGATAC |
| | Reverse primer (SEQ ID NO: 87): |
| | GCTGCAGCCAGAGGAGATTTGGGACGCCTCGCTGCAAGACAAGCAGCTACCGC AGATGTCAAC |
| RC2_IVT3 | Forward primer (SEQ ID NO: 88): |
| | TGCTGCAATATTCTGCAAATCACCTCGGGCAGCGGCGTTGCCTCTCTGGAGGTTG GTAGATACAG |
| | Reverse primer (SEQ ID NO: 89): |
| | GCCGCTGCCCGAGGTGATTTGCAGAATATTGCAGCAAGACAAGCAGCTACCGCA |
| | GATGTCAACAC |
| RC2_IVT4 | Forward primer (SEQ ID NO: 90): |
| | AGCTGCCAAATTCTGGAGGTCACCTCTTGCCGCAGCGTTGCCTCTCTGGAGGTTG GTAGATACAG |
| | Reverse primer (SEQ ID NO: 91): |
| | GCTGCGGCAAGAGGTGACCTCCAGAATTTGGCAGCTAGACAAGCAGCTACCGCA GATGTCAACAC |
| RC2_IVT5 | Forward primer (SEQ ID NO: 92): |
| | GGCGGCAATTGCTGCCAGATCACCTCTGGCTGCAGCGTTGCCTCTCTGGAGGTTG GTAGATACAG |
| | Reverse primer (SEQ ID NO: 93): |
| | GCTGCAGCCAGAGGTGATCTGGCAGCAATTGCCGCCAGACAAGCAGCTACCGCA GATGTCAACAC |
| RC2_IVT6 | Forward primer (SEQ ID NO: 94): |
| | CGCTGCCAGGGTTGCCAAGTCGCCTCTTGCAGCTGCGTTGCCTCTCTGGAGGTTG GTAGATACAG |
| | Reverse primer (SEQ ID NO: 95): |
| | GCAGCTGCAAGAGGCGACTTGGCAACCCTGGCAGCGAGACAAGCAGCTACCGC AGATGTCAACACAC |
| RC2_IVT7 | Forward primer (SEQ ID NO: 96): |
| | GGCGGCTGGCGTTGCCAAATCACCCCTAGCAGCCGCGTTGCCTCTCTGGAGGTT GGTAGATACAG |
| | Reverse primer (SEQ ID NO: 97): |
| | GCGGCTGCTAGGGGTGATTTGGCAACGCCAGCCGCCAGACAAGCAGCTACCGCA GATGTCAACAC |
| RC2_IVT8 | Forward primer (SEQ ID NO: 98): |
| | AGCTGCGGGTCTACCCAAATCACCGCGGGCTGCTGCGTTGCCTCTCTGGAGGTT GGTAGATACAG |
| | Reverse primer (SEQ ID NO: 99): |
| | ...ence (...) ... GCAGCAGCCCGCGGTGATTTGGGTAGACCCGCAGCTAGACAAGCAGCTACCGCA GATGTCAACAC |
| RC2_IVT9 | Forward primer (SEQ ID NO: 100): |
| | CGCAGCAAGTGTACCAAGGTCTCCTCTGGCTGCTGCGTTGCCTCTCTGGAGGTTG GTAGATACAG |
| | Reverse primer (SEQ ID NO: 101): |
| | GCAGCAGCCAGAGGAGACCTTGGTACACTTGCTGCGAGACAAGCAGCTACCGC AGATGTCAACACAC |
| RC2_IVT10 | Forward primer (SEQ ID NO: 102): |
| | TGCTGCAATCCTTGCGAGGTCACCTCTTGCAGCCGCGTTGCCTCTCTGGAGGTTG GTAGATACAG |
| | Reverse primer (SEQ ID NO: 103): |
| | GCGGCTGCAAGAGGTGACCTCGCAAGGATTGCAGCAAGACAAGCAGCTACCGC AGATGTCAACAC |
| RC2_IVT11 | Forward primer (SEQ ID NO: 104): |
| | AGCGGCTATATTTTGCAGGCTGCCTCTCGCAGCAGCGTTGCCTCTCTGGAGGTTG GTAGATACAG |
| | Reverse primer (SEQ ID NO: 105): |
| | GCTGCTGCGAGAGGCAGCCTGCAAAATATAGCCGCTAGACAAGCAGCTACCGCA GATGTCAACAC |
| RC2_IVT12 | Forward primer (SEQ ID NO: 106): |
| | GGCAGCCAGATTTTGGAGAGATCCTCGTGCTGCTGCGTTGCCTCTCTGGAGGTTG GTAGATACAG |
| | Reverse primer (SEQ ID NO: 107): |
| | GCAGCAGCACGAGGATCTCTCCAAAATCTGGCTGCCAGACAAGCAGCTACCGCA GATGTCAACAC |
| RC2_IVT13 | Forward primer (SEQ ID NO: 108): |
| | TGCCGCAAGACTGCCCCTAGCAGCGGCGTTGCCTCTCTGGAGGTTGGTAGATAC AGAACCATACTG |
| | Reverse primer (SEQ ID NO: 109): |
| | GCCGCTGCTAGGGGCAGTCTTGCGGCAAGACAAGCAGCTACCGCAGATGTCAAC ACACAAGGCG |
| RC2_IVT14 | Forward primer (SEQ ID NO: 110): |
| | CGGTAGCTGCTTGTCTGCCGTTTCTCTGGAGGTTGGTAGATACAG |
| | Reverse primer (SEQ ID NO: 111): |
| | AACCTCCAGAGAAACGGCAGACAAGCAGCTACCGCAGATGTC |
| RC2_IVT15 | Forward primer (SEQ ID NO: 14): |
| | GGCTGCTGCATGGGCCCGTCCGTTTCCTGCTGCTGCGTTGCCTCTCTGGAGGTTG GTAGATACAG |
| | Reverse primer (SEQ ID NO: 15): |
| | GCAGCAGCAGGAAACGGACGGGCCCATGCAGCAGCCAGACAAGCAGCTACCGC AGATGTCAACAC |

Experimental results: In this example, the plasmid DNA to be constructed was identified by enzyme digestion and sanger sequencing, which confirmed successful construction of the expression plasmids of the corresponding capsid proteins. The amino acid sequences of the AAV capsid proteins expressed by the AAV capsid protein expression plasmids were shown in Table 3 below.

**Table 3:**

| **Name and Amino Acid Sequence of Cap/AAV Capsid Protein** | **Sequence of Inserted or Substituted Targeting Peptide** | **Amino Acid Sequence of AAV Capsid Protein (Inserted or substituted targeting peptides shown in bold and underlined)** |
|---|---|---|
| IVT1 | AAARGDLAT | MAADGYLPDWLEDTLSEGIRQWWKLKPGPPPPKPAER |
| (SEQ ID NO: 70) | IAA (SEQ ID NO: 57) | HKDDSRGLVLPGYKYLGPFNGLDKGEPVNEADAAALE HDKAYDRQLDSGDNPYLKYNHADAEFQERLKEDTSFG GNLGRAVFQAKKRVLEPLGLVEEPVKTAPGKKRPVEHS PVEPDSSSGTGKAGQQPARKRLNFGQTGDADSVPDPQP LGQPPAAPSGLGTNTMATGSGAPMADNNEGADGVGN SSGNWHCDSTWMGDRVITTSTRTWALPTYNNHLYKQI SSQSGASNDNHYFGYSTPWGYFDFNRFHCHFSPRDWQ RLINNNWGFRPKRLNFKLFNIQVKEVTQNDGTTTIANN LTSTVQVFTDSEYQLPYVLGSAHQGCLPPFPADVFMVP QYGYLTLNNGSQAVGRSSFYCLEYFPSQMLRTGNNFTF SYTFEDVPFHSSYAHSQSLDRLMNPLIDQYLYYLSRTNT PSGTTTQSRLQFSQAGASDIRDQSRNWLPGPCYRQQRV SKTSADNNNSEYSWTGATKYHLNGRDSLVNPGPAMAS HKDDEEKFFPQSGVLIFGKQGSEKTNVDIEKVMITDEE EIRTTNPVATEQYGSVSTNLQRGNAAARGDLATIAARQ AATADVNTQGVLPGMVWQDRDVYLQGPIWAKIPHTD GHFHPSPLMGGFGLKHPPPQILIKNTPVPANPSTTFSAA KFASFITQYSTGQVSVEIEWELQKENSKRWNPEIQYTSN YNKSVNVDFTVDTNGVYSEPRPIGTRYLTRNL* |
| IVT2 (SEQ ID NO: 71) | AAARGDLGR LAA (SEQ ID NO: 58) | MAADGYLPDWLEDTLSEGIRQWWKLKPGPPPPKPAER HKDDSRGLVLPGYKYLGPFNGLDKGEPVNEADAAALE HDKAYDRQLDSGDNPYLKYNHADAEFQERLKEDTSFG GNLGRAVFQAKKRVLEPLGLVEEPVKTAPGKKRPVEHS PVEPDSSSGTGKAGQQPARKRLNFGQTGDADSVPDPQP LGQPPAAPSGLGTNTMATGSGAPMADNNEGADGVGN SSGNWHCDSTWMGDRVITTSTRTWALPTYNNHLYKQI SSQSGASNDNHYFGYSTPWGYFDFNRFHCHFSPRDWQ RLINNNWGFRPKRLNFKLFNIQVKEVTQNDGTTTIANN LTSTVQVFTDSEYQLPYVLGSAHQGCLPPFPADVFMVP QYGYLTLNNGSQAVGRSSFYCLEYFPSQMLRTGNNFTF SYTFEDVPFHSSYAHSQSLDRLMNPLIDQYLYYLSRTNT PSGTTTQSRLQFSQAGASDIRDQSRNWLPGPCYRQQRV SKTSADNNNSEYSWTGATKYHLNGRDSLVNPGPAMAS HKDDEEKFFPQSGVLIFGKQGSEKTNVDIEKVMITDEE EIRTTNPVATEQYGSVSTNLQRGNAAARGDLGRLAAR QAATADVNTQGVLPGMVWQDRDVYLQGPIWAKIPHT DGHFHPSPLMGGFGLKHPPPQILIKNTPVPANPSTTFSA AKFASFITQYSTGQVSVEIEWELQKENSKRWNPEIQYTS NYNKSVNVDFTVDTNGVYSEPRPIGTRYLTRNL* |
| IVT3 (SEQ ID NO: 72) | AAARGDLQN IAA (SEQ ID NO: 59) | MAADGYLPDWLEDTLSEGIRQWWKLKPGPPPPKPAER HKDDSRGLVLPGYKYLGPFNGLDKGEPVNEADAAALE HDKAYDRQLDSGDNPYLKYNHADAEFQERLKEDTSFG GNLGRAVFQAKKRVLEPLGLVEEPVKTAPGKKRPVEHS PVEPDSSSGTGKAGQQPARKRLNFGQTGDADSVPDPQP LGQPPAAPSGLGTNTMATGSGAPMADNNEGADGVGN |
| | | SSGNWHCDSTWMGDRVITTSTRTWALPTYNNHLYKQI SSQSGASNDNHYFGYSTPWGYFDFNRFHCHFSPRDWQ RLINNNWGFRPKRLNFKLFNIQVKEVTQNDGTTTIANN LTSTVQVFTDSEYQLPYVLGSAHQGCLPPFPADVFMVP QYGYLTLNNGSQAVGRSSFYCLEYFPSQMLRTGNNFTF SYTFEDVPFHSSYAHSQSLDRLMNPLIDQYLYYLSRTNT PSGTTTQSRLQFSQAGASDIRDQSRNWLPGPCYRQQRV SKTSADNNNSEYSWTGATKYHLNGRDSLVNPGPAMAS HKDDEEKFFPQSGVLIFGKQGSEKTNVDIEKVMITDEE EIRTTNPVATEQYGSVSTNLQRGNAAARGDLQNIAAR QAATADVNTQGVLPGMVWQDRDVYLQGPIWAKIPHT DGHFHPSPLMGGFGLKHPPPQILIKNTPVPANPSTTFSA AKFASFITQYSTGQVSVEIEWELQKENSKRWNPEIQYTS NYNKSVNVDFTVDTNGVYSEPRPIGTRYLTRNL* |
| IVT4 (SEQ ID NO: 73) | AAARGDLQN LAA (SEQ ID NO: 60) | MAADGYLPDWLEDTLSEGIRQWWKLKPGPPPPKPAER HKDDSRGLVLPGYKYLGPFNGLDKGEPVNEADAAALE HDKAYDRQLDSGDNPYLKYNHADAEFQERLKEDTSFG GNLGRAVFQAKKRVLEPLGLVEEPVKTAPGKKRPVEHS PVEPDSSSGTGKAGQQPARKRLNFGQTGDADSVPDPQP LGQPPAAPSGLGTNTMATGSGAPMADNNEGADGVGN SSGNWHCDSTWMGDRVITTSTRTWALPTYNNHLYKQI SSQSGASNDNHYFGYSTPWGYFDFNRFHCHFSPRDWQ RLINNNWGFRPKRLNFKLFNIQVKEVTQNDGTTTIANN LTSTVQVFTDSEYQLPYVLGSAHQGCLPPFPADVFMVP QYGYLTLNNGSQAVGRSSFYCLEYFPSQMLRTGNNFTF SYTFEDVPFHSSYAHSQSLDRLMNPLIDQYLYYLSRTNT PSGTTTQSRLQFSQAGASDIRDQSRNWLPGPCYRQQRV SKTSADNNNSEYSWTGATKYHLNGRDSLVNPGPAMAS HKDDEEKFFPQSGVLIFGKQGSEKTNVDIEKVMITDEE EIRTTNPVATEQYGSVSTNLQRGNAAARGDLQNLAAR QAATADVNTQGVLPGMVWQDRDVYLQGPIWAKIPHT DGHFHPSPLMGGFGLKHPPPQILIKNTPVPANPSTTFSA AKFASFITQYSTGQVSVEIEWELQKENSKRWNPEIQYTS NYNKSVNVDFTVDTNGVYSEPRPIGTRYLTRNL* |
| IVT5 (SEQ ID NO: 74) | AAARGDLAA IAA (SEQ ID NO: 61) | MAADGYLPDWLEDTLSEGIRQWWKLKPGPPPPKPAER HKDDSRGLVLPGYKYLGPFNGLDKGEPVNEADAAALE HDKAYDRQLDSGDNPYLKYNHADAEFQERLKEDTSFG GNLGRAVFQAKKRVLEPLGLVEEPVKTAPGKKRPVEHS PVEPDSSSGTGKAGQQPARKRLNFGQTGDADSVPDPQP LGQPPAAPSGLGTNTMATGSGAPMADNNEGADGVGN SSGNWHCDSTWMGDRVITTSTRTWALPTYNNHLYKQI SSQSGASNDNHYFGYSTPWGYFDFNRFHCHFSPRDWQ RLINNNWGFRPKRLNFKLFNIQVKEVTQNDGTTTIANN LTSTVQVFTDSEYQLPYVLGSAHQGCLPPFPADVFMVP QYGYLTLNNGSQAVGRSSFYCLEYFPSQMLRTGNNFTF |
| | | SYTFEDVPFHSSYAHSQSLDRLMNPLIDQYLYYLSRTNT PSGTTTQSRLQFSQAGASDIRDQSRNWLPGPCYRQQRV SKTSADNNNSEYSWTGATKYHLNGRDSLVNPGPAMAS HKDDEEKFFPQSGVLIFGKQGSEKTNVDIEKVMITDEE EIRTTNPVATEQYGSVSTNLQRGNAAARGDLAAIAARQ AATADVNTQGVLPGMVWQDRDVYLQGPIWAKIPHTD GHFHPSPLMGGFGLKHPPPQILIKNTPVPANPSTTFSAA KFASFITQYSTGQVSVEIEWELQKENSKRWNPEIQYTSN YNKSVNVDFTVDTNGVYSEPRPIGTRYLTRNL* |
| IVT6 (SEQ ID NO: 75) | AAARGDLAT LAA (SEQ ID NO: 62) | MAADGYLPDWLEDTLSEGIRQWWKLKPGPPPPKPAER HKDDSRGLVLPGYKYLGPFNGLDKGEPVNEADAAALE HDKAYDRQLDSGDNPYLKYNHADAEFQERLKEDTSFG GNLGRAVFQAKKRVLEPLGLVEEPVKTAPGKKRPVEHS PVEPDSSSGTGKAGQQPARKRLNFGQTGDADSVPDPQP LGQPPAAPSGLGTNTMATGSGAPMADNNEGADGVGN SSGNWHCDSTWMGDRVITTSTRTWALPTYNNHLYKQI SSQSGASNDNHYFGYSTPWGYFDFNRFHCHFSPRDWQ RLINNNWGFRPKRLNFKLFNIQVKEVTQNDGTTTIANN LTSTVQVFTDSEYQLPYVLGSAHQGCLPPFPADVFMVP QYGYLTLNNGSQAVGRSSFYCLEYFPSQMLRTGNNFTF SYTFEDVPFHSSYAHSQSLDRLMNPLIDQYLYYLSRTNT PSGTTTQSRLQFSQAGASDIRDQSRNWLPGPCYRQQRV SKTSADNNNSEYSWTGATKYHLNGRDSLVNPGPAMAS HKDDEEKFFPQSGVLIFGKQGSEKTNVDIEKVMITDEE EIRTTNPVATEQYGSVSTNLQRGNAAARGDLATLAAR QAATADVNTQGVLPGMVWQDRDVYLQGPIWAKIPHT DGHFHPSPLMGGFGLKHPPPQILIKNTPVPANPSTTFSA AKFASFITQYSTGQVSVEIEWELQKENSKRWNPEIQYTS NYNKSVNVDFTVDTNGVYSEPRPIGTRYLTRNL* |
| IVT7 (SEQ ID NO: 76) | AAARGDLAT PAA (SEQ ID NO: 63) | MAADGYLPDWLEDTLSEGIRQWWKLKPGPPPPKPAER HKDDSRGLVLPGYKYLGPFNGLDKGEPVNEADAAALE HDKAYDRQLDSGDNPYLKYNHADAEFQERLKEDTSFG GNLGRAVFQAKKRVLEPLGLVEEPVKTAPGKKRPVEHS PVEPDSSSGTGKAGQQPARKRLNFGQTGDADSVPDPQP LGQPPAAPSGLGTNTMATGSGAPMADNNEGADGVGN SSGNWHCDSTWMGDRVITTSTRTWALPTYNNHLYKQI SSQSGASNDNHYFGYSTPWGYFDFNRFHCHFSPRDWQ RLINNNWGFRPKRLNFKLFNIQVKEVTQNDGTTTIANN LTSTVQVFTDSEYQLPYVLGSAHQGCLPPFPADVFMVP QYGYLTLNNGSQAVGRSSFYCLEYFPSQMLRTGNNFTF SYTFEDVPFHSSYAHSQSLDRLMNPLIDQYLYYLSRTNT PSGTTTQSRLQFSQAGASDIRDQSRNWLPGPCYRQQRV SKTSADNNNSEYSWTGATKYHLNGRDSLVNPGPAMAS HKDDEEKFFPQSGVLIFGKQGSEKTNVDIEKVMITDEE EIRTTNPVATEQYGSVSTNLQRGNAAARGDLATPAARQ |
| | | AATADVNTQGVLPGMVWQDRDVYLQGPIWAKIPHTD GHFHPSPLMGGFGLKHPPPQILIKNTPVPANPSTTFSAA KFASFITQYSTGQVSVEIEWELQKENSKRWNPEIQYTSN YNKSVNVDFTVDTNGVYSEPRPIGTRYLTRNL* |
| IVT8 (SEQ ID NO: 77) | AAARGDLGR PAA (SEQ ID NO: 64) | MAADGYLPDWLEDTLSEGIRQWWKLKPGPPPPKPAER HKDDSRGLVLPGYKYLGPFNGLDKGEPVNEADAAALE HDKAYDRQLDSGDNPYLKYNHADAEFQERLKEDTSFG GNLGRAVFQAKKRVLEPLGLVEEPVKTAPGKKRPVEHS PVEPDSSSGTGKAGQQPARKRLNFGQTGDADSVPDPQP LGQPPAAPSGLGTNTMATGSGAPMADNNEGADGVGN SSGNWHCDSTWMGDRVITTSTRTWALPTYNNHLYKQI SSQSGASNDNHYFGYSTPWGYFDFNRFHCHFSPRDWQ RLINNNWGFRPKRLNFKLFNIQVKEVTQNDGTTTIANN LTSTVQVFTDSEYQLPYVLGSAHQGCLPPFPADVFMVP QYGYLTLNNGSQAVGRSSFYCLEYFPSQMLRTGNNFTF SYTFEDVPFHSSYAHSQSLDRLMNPLIDQYLYYLSRTNT PSGTTTQSRLQFSQAGASDIRDQSRNWLPGPCYRQQRV SKTSADNNNSEYSWTGATKYHLNGRDSLVNPGPAMAS HKDDEEKFFPQSGVLIFGKQGSEKTNVDIEKVMITDEE EIRTTNPVATEQYGSVSTNLQRGNAAARGDLGRPAAR QAATADVNTQGVLPGMVWQDRDVYLQGPIWAKIPHT DGHFHPSPLMGGFGLKHPPPQILIKNTPVPANPSTTFSA AKFASFITQYSTGQVSVEIEWELQKENSKRWNPEIQYTS NYNKSVNVDFTVDTNGVYSEPRPIGTRYLTRNL* |
| IVT9 (SEQ ID NO: 78) | AAARGDLGT LAA (SEQ ID NO: 65) | MAADGYLPDWLEDTLSEGIRQWWKLKPGPPPPKPAER HKDDSRGLVLPGYKYLGPFNGLDKGEPVNEADAAALE HDKAYDRQLDSGDNPYLKYNHADAEFQERLKEDTSFG GNLGRAVFQAKKRVLEPLGLVEEPVKTAPGKKRPVEHS PVEPDSSSGTGKAGQQPARKRLNFGQTGDADSVPDPQP LGQPPAAPSGLGTNTMATGSGAPMADNNEGADGVGN SSGNWHCDSTWMGDRVITTSTRTWALPTYNNHLYKQI SSQSGASNDNHYFGYSTPWGYFDFNRFHCHFSPRDWQ RLINNNWGFRPKRLNFKLFNIQVKEVTQNDGTTTIANN LTSTVQVFTDSEYQLPYVLGSAHQGCLPPFPADVFMVP QYGYLTLNNGSQAVGRSSFYCLEYFPSQMLRTGNNFTF SYTFEDVPFHSSYAHSQSLDRLMNPLIDQYLYYLSRTNT PSGTTTQSRLQFSQAGASDIRDQSRNWLPGPCYRQQRV SKTSADNNNSEYSWTGATKYHLNGRDSLVNPGPAMAS HKDDEEKFFPQSGVLIFGKQGSEKTNVDIEKVMITDEE EIRTTNPVATEQYGSVSTNLQRGNAAARGDLGTLAAR QAATADVNTQGVLPGMVWQDRDVYLQGPIWAKIPHT DGHFHPSPLMGGFGLKHPPPQILIKNTPVPANPSTTFSA AKFASFITQYSTGQVSVEIEWELQKENSKRWNPEIQYTS NYNKSVNVDFTVDTNGVYSEPRPIGTRYLTRNL* |
| IVT10 (SEQ ID NO: 79) | AAARGDLAR IAA (SEQ ID NO: 66) | MAADGYLPDWLEDTLSEGIRQWWKLKPGPPPPKPAER HKDDSRGLVLPGYKYLGPFNGLDKGEPVNEADAAALE HDKAYDRQLDSGDNPYLKYNHADAEFQERLKEDTSFG GNLGRAVFQAKKRVLEPLGLVEEPVKTAPGKKRPVEHS PVEPDSSSGTGKAGQQPARKRLNFGQTGDADSVPDPQP LGQPPAAPSGLGTNTMATGSGAPMADNNEGADGVGN SSGNWHCDSTWMGDRVITTSTRTWALPTYNNHLYKQI SSQSGASNDNHYFGYSTPWGYFDFNRFHCHFSPRDWQ RLINNNWGFRPKRLNFKLFNIQVKEVTQNDGTTTIANN LTSTVQVFTDSEYQLPYVLGSAHQGCLPPFPADVFMVP QYGYLTLNNGSQAVGRSSFYCLEYFPSQMLRTGNNFTF SYTFEDVPFHSSYAHSQSLDRLMNPLIDQYLYYLSRTNT PSGTTTQSRLQFSQAGASDIRDQSRNWLPGPCYRQQRV SKTSADNNNSEYSWTGATKYHLNGRDSLVNPGPAMAS HKDDEEKFFPQSGVLIFGKQGSEKTNVDIEKVMITDEE EIRTTNPVATEQYGSVSTNLQRGNAAARGDLARIAARQ AATADVNTQGVLPGMVWQDRDVYLQGPIWAKIPHTD GHFHPSPLMGGFGLKHPPPQILIKNTPVPANPSTTFSAA KFASFITQYSTGQVSVEIEWELQKENSKRWNPEIQYTSN YNKSVNVDFTVDTNGVYSEPRPIGTRYLTRNL* |
| IVT11 (SEQ ID NO: 80) | AAARGSLQN IAA (SEQ ID NO: 67) | MAADGYLPDWLEDTLSEGIRQWWKLKPGPPPPKPAER HKDDSRGLVLPGYKYLGPFNGLDKGEPVNEADAAALE HDKAYDRQLDSGDNPYLKYNHADAEFQERLKEDTSFG GNLGRAVFQAKKRVLEPLGLVEEPVKTAPGKKRPVEHS PVEPDSSSGTGKAGQQPARKRLNFGQTGDADSVPDPQP LGQPPAAPSGLGTNTMATGSGAPMADNNEGADGVGN SSGNWHCDSTWMGDRVITTSTRTWALPTYNNHLYKQI SSQSGASNDNHYFGYSTPWGYFDFNRFHCHFSPRDWQ RLINNNWGFRPKRLNFKLFNIQVKEVTQNDGTTTIANN LTSTVQVFTDSEYQLPYVLGSAHQGCLPPFPADVFMVP QYGYLTLNNGSQAVGRSSFYCLEYFPSQMLRTGNNFTF SYTFEDVPFHSSYAHSQSLDRLMNPLIDQYLYYLSRTNT PSGTTTQSRLQFSQAGASDIRDQSRNWLPGPCYRQQRV SKTSADNNNSEYSWTGATKYHLNGRDSLVNPGPAMAS HKDDEEKFFPQSGVLIFGKQGSEKTNVDIEKVMITDEE EIRTTNPVATEQYGSVSTNLQRGNAAARGSLQNIAARQ AATADVNTQGVLPGMVWQDRDVYLQGPIWAKIPHTD GHFHPSPLMGGFGLKHPPPQILIKNTPVPANPSTTFSAA KFASFITQYSTGQVSVEIEWELQKENSKRWNPEIQYTSN YNKSVNVDFTVDTNGVYSEPRPIGTRYLTRNL* |
| IVT12 (SEQ ID NO:81) | AAARGSLQN LAA (SEQ ID NO: 68) | MAADGYLPDWLEDTLSEGIRQWWKLKPGPPPPKPAER HKDDSRGLVLPGYKYLGPFNGLDKGEPVNEADAAALE HDKAYDRQLDSGDNPYLKYNHADAEFQERLKEDTSFG GNLGRAVFQAKKRVLEPLGLVEEPVKTAPGKKRPVEHS PVEPDSSSGTGKAGQQPARKRLNFGQTGDADSVPDPQP |
| | | LGQPPAAPSGLGTNTMATGSGAPMADNNEGADGVGN SSGNWHCDSTWMGDRVITTSTRTWALPTYNNHLYKQI SSQSGASNDNHYFGYSTPWGYFDFNRFHCHFSPRDWQ RLINNNWGFRPKRLNFKLFNIQVKEVTQNDGTTTIANN LTSTVQVFTDSEYQLPYVLGSAHQGCLPPFPADVFMVP QYGYLTLNNGSQAVGRSSFYCLEYFPSQMLRTGNNFTF SYTFEDVPFHSSYAHSQSLDRLMNPLIDQYLYYLSRTNT PSGTTTQSRLQFSQAGASDIRDQSRNWLPGPCYRQQRV SKTSADNNNSEYSWTGATKYHLNGRDSLVNPGPAMAS HKDDEEKFFPQSGVLIFGKQGSEKTNVDIEKVMITDEE EIRTTNPVATEQYGSVSTNLQRGNAAARGSLQNLAAR QAATADVNTQGVLPGMVWQDRDVYLQGPIWAKIPHT DGHFHPSPLMGGFGLKHPPPQILIKNTPVPANPSTTFSA AKFASFITQYSTGQVSVEIEWELQKENSKRWNPEIQYTS NYNKSVNVDFTVDTNGVYSEPRPIGTRYLTRNL* |
| IVT13 (SEQ ID NO: 82) | AAARGSLAA (SEQ ID NO: 69) | MAADGYLPDWLEDTLSEGIRQWWKLKPGPPPPKPAER HKDDSRGLVLPGYKYLGPFNGLDKGEPVNEADAAALE HDKAYDRQLDSGDNPYLKYNHADAEFQERLKEDTSFG GNLGRAVFQAKKRVLEPLGLVEEPVKTAPGKKRPVEHS PVEPDSSSGTGKAGQQPARKRLNFGQTGDADSVPDPQP LGQPPAAPSGLGTNTMATGSGAPMADNNEGADGVGN SSGNWHCDSTWMGDRVITTSTRTWALPTYNNHLYKQI SSQSGASNDNHYFGYSTPWGYFDFNRFHCHFSPRDWQ RLINNNWGFRPKRLNFKLFNIQVKEVTQNDGTTTIANN LTSTVQVFTDSEYQLPYVLGSAHQGCLPPFPADVFMVP QYGYLTLNNGSQAVGRSSFYCLEYFPSQMLRTGNNFTF SYTFEDVPFHSSYAHSQSLDRLMNPLIDQYLYYLSRTNT PSGTTTQSRLQFSQAGASDIRDQSRNWLPGPCYRQQRV SKTSADNNNSEYSWTGATKYHLNGRDSLVNPGPAMAS HKDDEEKFFPQSGVLIFGKQGSEKTNVDIEKVMITDEE EIRTTNPVATEQYGSVSTNLQRGNAAARGSLAARQAAT ADVNTQGVLPGMVWQDRDVYLQGPIWAKIPHTDGHF HPSPLMGGFGLKHPPPQILIKNTPVPANPSTTFSAAKFAS FITQYSTGQVSVEIEWELQKENSKRWNPEIQYTSNYNK SVNVDFTVDTNGVYSEPRPIGTRYLTRNL* |
| IVT14 (SEQ ID NO: 83) | NG | MAADGYLPDWLEDTLSEGIRQWWKLKPGPPPPKPAER HKDDSRGLVLPGYKYLGPFNGLDKGEPVNEADAAALE HDKAYDRQLDSGDNPYLKYNHADAEFQERLKEDTSFG GNLGRAVFQAKKRVLEPLGLVEEPVKTAPGKKRPVEHS PVEPDSSSGTGKAGQQPARKRLNFGQTGDADSVPDPQP LGQPPAAPSGLGTNTMATGSGAPMADNNEGADGVGN SSGNWHCDSTWMGDRVITTSTRTWALPTYNNHLYKQI SSQSGASNDNHYFGYSTPWGYFDFNRFHCHFSPRDWQ RLINNNWGFRPKRLNFKLFNIQVKEVTQNDGTTTIANN LTSTVQVFTDSEYQLPYVLGSAHQGCLPPFPADVFMVP |
| | | QYGYLTLNNGSQAVGRSSFYCLEYFPSQMLRTGNNFTF SYTFEDVPFHSSYAHSQSLDRLMNPLIDQYLYYLSRTNT PSGTTTQSRLQFSQAGASDIRDQSRNWLPGPCYRQQRV SKTSADNNNSEYSWTGATKYHLNGRDSLVNPGPAMAS HKDDEEKFFPQSGVLIFGKQGSEKTNVDIEKVMITDEE EIRTTNPVATEQYGSVSTNLQRNGRQAATADVNTQGVL PGMVWQDRDVYLQGPIWAKIPHTDGHFHPSPLMGGFG LKHPPPQILIKNTPVPANPSTTFSAAKFASFITQYSTGQV SVEIEWELQKENSKRWNPEIQYTSNYNKSVNVDFTVDT NGVYSEPRPIGTRYLTRNL* |
| IVT15 (SEQ ID NO: 16) | AAAGNGRA HAAA (SEQ ID NO: 11) | MAADGYLPDWLEDTLSEGIRQWWKLKPGPPPPKPAER HKDDSRGLVLPGYKYLGPFNGLDKGEPVNEADAAALE HDKAYDRQLDSGDNPYLKYNHADAEFQERLKEDTSFG GNLGRAVFQAKKRVLEPLGLVEEPVKTAPGKKRPVEHS PVEPDSSSGTGKAGQQPARKRLNFGQTGDADSVPDPQP LGQPPAAPSGLGTNTMATGSGAPMADNNEGADGVGN SSGNWHCDSTWMGDRVITTSTRTWALPTYNNHLYKQI SSQSGASNDNHYFGYSTPWGYFDFNRFHCHFSPRDWQ RLINNNWGFRPKRLNFKLFNIQVKEVTQNDGTTTIANN LTSTVQVFTDSEYQLPYVLGSAHQGCLPPFPADVFMVP QYGYLTLNNGSQAVGRSSFYCLEYFPSQMLRTGNNFTF SYTFEDVPFHSSYAHSQSLDRLMNPLIDQYLYYLSRTNT PSGTTTQSRLQFSQAGASDIRDQSRNWLPGPCYRQQRV SKTSADNNNSEYSWTGATKYHLNGRDSLVNPGPAMAS HKDDEEKFFPQSGVLIFGKQGSEKTNVDIEKVMITDEE EIRTTNPVATEQYGSVSTNLQRGNAAAGNGRAHAAAR QAATADVNTQGVLPGMVWQDRDVYLQGPIWAKIPHT DGHFHPSPLMGGFGLKHPPPQILIKNTPVPANPSTTFSA AKFASFITQYSTGQVSVEIEWELQKENSKRWNPEIQYTS NYNKSVNVDFTVDTNGVYSEPRPIGTRYLTRNL* |

### Example 6: Packaging and Identification of Viruses

In this example, the hIL2sp-Aflibercept opt3 sequence, which was proved to have the best secretory effect in Example 4, was selected for viral packaging and further studies.

With reference to the literature (Xiao X, et al. J Virol. 1998; 72(3):2224-2232.) along with some adjustments, the recombinant AAVs were packaged using a three-plasmid packaging system. All the AAVs used IVT15 constructed in Example 5 as a capsid. The HEK 293T cells were seeded in a 100 mm culture dish and grown to 70% to 80% confluency for plasmid transfection. 5 µg of the pssAAV-CMV-hIL2sp-Aflibercept opt3 plasmid (or pssAAV-CBh-hIL2sp-Aflibercept opt3 or pssAAV-CB7-hIL2sp-Aflibercept opt3 plasmid) constructed in Example 3, 5 µg of the RepCap plasmid pAAV-RC2_IVT15 with serotype IVT15 constructed in Example 5, and 10 µg of phepler plasmid (purchased and stored by the applicant from Cell Biolabs Inc.; this plasmid containing adenovirus-derived helper function genes E2A, E4, VA RNA and the like required for preparation of recombinant AAV viruses via three-plasmid co-transfection into HEK293 (or HEK293T) cells) were co-transfected into the HEK293T cells using poly ethylenimine linear (PEI). Cells and supernatants were harvested at 72 h after transfection. The cells were pelleted by centrifugation at a low speed and lysed by adding 0.5% sodium deoxycholate (w/v). 50 U/mL Benzonase and 2 mM MgCl₂ were added, and incubation was carried out at 37°C for 2 hours to digest free DNA molecules, while pelleting the supernatant on ice with a one-fifth volume of a solution of 40% PEG8000 in 2.5 M NaCl solution. The cell lysate and the supernatant were mixed and then centrifuged. The supernatant was measured and purified by the iodixanol ultracentrifugation purification method to finally obtain rAAV IVT15-CMV-hIL2sp-Aflibercept opt3, rAAV IVT15-CBh-hIL2sp-Aflibercept opt3, and rAAV IVT15-CB7-hIL2sp-Aflibercept opt3. Subsequently, the titers of the purified AAVs were determined by qPCR and the purified AAVs were stored in a refrigerator at -80°C.

The specific determination method was as follows:
The physical titer of the prepared AAV viral genome was determined by the qPCR method. The specific procedures were as follows:
Primers and a probe for qPCR detection were designed for the identical sequence hIL2sp-Aflibercept opt3:
Aflibercept opt3-Q-F: 5'- GCTTCTACCCCTCTGACATTGC -3' (SEQ ID NO: 17)
Aflibercept opt3-Q-R: 5'- CAGGAAGAATGAGCCATCAGAGT -3' (SEQ ID NO: 18)
Aflibercept opt3-Q-P: 5'- CCAGCCTGAGAACAACTACAAGACAACCCC -3' (SEQ ID NO: 19)
Aflibercept opt3-Q-F and Aflibercept opt3-Q-R were primers, and Aflibercept opt3-Q-P was a probe. The probe was labeled with an FAM fluorescent protein at the 5' end and conjugated to a BlackBerry quencher at the 3' end. The primers and probe were synthesized by ThermoFisher Scientific. A 107-bp fragment in the hIL2sp-Aflibercept opt3 sequence was specifically amplified using Aflibercept opt3-F and Aflibercept opt3-R as primers. Based on the TaqMan probe binding assay, the physical titer of the viral genome was determined by a fluorescent quantitative PCR instrument (type: Q5, ThermoFisher) using the Premix Ex Taq (Probe qPCR) reagent (TaKaRa, Beijing, China), where the pssAAV-CB7-hIL2sp-Aflibercept opt3 plasmid at a concentration of 1.0 × 10⁸ copies/mL and its 10-fold gradient dilution samples were used as standard samples. The operational procedures were carried out in accordance with the instructions of the reagent used. The viruses were processed by the method described in the literature (Aurnhammer C, et al. Hum Gene Ther Methods. 2012; 23(1): 18-28.).

The same method described in this example was used to package the virus rAAV2 7m8 ADVM-022 used in other examples for comparative studies.

5 µg of the pssAAV-ADVM-022 plasmid, 5 µg of the applicant's deposited RepCap plasmid pAAV-2 7m8 with the serotype AAV2 7m8, and 10 µg of phepler plasmid were co-transfected into the HEK293T cells to package rAAV2 7m8 ADVM-022. Subsequently, the titers of the purified AAVs were determined by qPCR and the purified AAVs were stored in a refrigerator at -80°C.

The specific determination method was as follows:
The physical titer of the prepared AAV viral genome was determined by the qPCR method. The specific procedures were as follows:
Primers and a probe for qPCR detection were designed for the identical sequence ADVM-022:
ADVM-022-Q-F: 5'-ACCAAGCCGAGAGAAGAACA-3' (SEQ ID NO: 53)
ADVM-022-Q-R: 5'-TGTTGGACACCTTGCACTTG-3' (SEQ ID NO: 54)
ADVM-022-Q-P: 5'-CAGCACCGACACCACCCGGT-3' (SEQ ID NO: 55)
ADVM-022-Q-F and ADVM-022-Q-R were primers and ADVM-022-Q-P was a probe. The probe was labeled with an FAM fluorescent protein at the 5' end and conjugated to a BlackBerry quencher at the 3' end. The primers and probe were synthesized by ThermoFisher Scientific. A 112-bp fragment in the ADVM-022 sequence was specifically amplified using ADVM-022-F and ADVM-022-R as primers. Based on the TaqMan probe binding assay, the physical titer of the viral genome was determined by a fluorescent quantitative PCR instrument (type: Q5, ThermoFisher) using the Premix Ex Taq (Probe qPCR) reagent (TaKaRa, Beijing, China), where the pssAAV-ADVM-022 plasmid at a concentration of 1.0 × 10⁸ copies/mL and its 10-fold gradient dilution samples were used as standard samples.

### Example 7: Expression of Virus in Mice

To evaluate the expression of several viruses carrying the aflibercept expression cassette, 45 wild-type c57 mice aged 6-8 weeks were randomly assigned to five groups, with nine mice in each group. Each group received intravitreal injections, at a dose of 6.0 × 10⁹ vg, of rAAV IVT15-CMV-hIL2sp-Aflibercept opt3, rAAV IVT15-CBh-hIL2sp-Aflibercept opt3, rAAV IVT15-CB7-hIL2sp-Aflibercept opt3, or rAAV2 7m8-ADVM-022 (as a control virus) or 1 µL of PBS (as a negative control) into both eyes, which were designated as Group A1, Group A2, Group A3, Group A4, and Group A5. At the three time points -2 weeks, week 4 and 6 weeks after injection, the aqueous humor was collected from both eyes of three mice (a total of six eyes) each time and used for determining the concentration (i.e., the expression level) of secretory aflibercept by Elisa. The kits for the aflibercept assay were purchased from ImmunoGuide (Turkey), Cat. No.: IG-AA115. The results showed that aflibercept could be constantly expressed from Week 2 to Week 6 in all the mice injected with any of the three viruses carrying the hIL2sp-Aflibercept opt3 expression cassette (FIG. 5). Among them, Group A3 (i.e., the mice injected with rAAV IVT15-CB7-hIL2sp-Aflibercept opt3) exhibited the highest expression levels in all the three time points. In addition, the control virus rAAV2 7m8-ADVM-022 (group A4) could also mediate sustained expression of aflibercept from Week 2 to Week 6, but its mean expression level was lower than those in Groups A1 to A3.

### Example 8: Construction of Choroidal Neovascularization (CNV) Models in Mice Injected with Virus

The rAAV IVT15-CMV-hIL2sp-Aflibercept opt3 and rAAV IVT15-CB7-hIL2sp-Aflibercept opt3 were selected for further functional studies. 40 wild-type c57 mice aged 6-8 weeks were randomly assigned to four groups, with 10 mice in each group. Each group received an intravitreal injection, at a dose of 6.0 ×10⁹ vg, of rAAV IVT15-CMV-hIL2sp-Aflibercept opt3 or rAAV IVT15-CB7-hIL2sp-Aflibercept opt3, or rAAV2 7m8-ADVM-022 (as a control virus) or 1 µL of PBS (as a negative control) into the right eye (single eye), which were designated as Group G1, Group G2, Group G3 and Group G4, respectively. The above mice were subjected to laser-induced modeling at Day 28 after injection to construct Choroidal Neovascularization (CNV) mouse models. The construction procedures were as follows: A mouse was placed at an appropriate position under a slit lamp, and the eyeball surface was perpendicular to the beam of the slit lamp. A handheld coverslip was used as a contact lens and the optic disc was moved to the center of the field of view, so that the red blood vessels were clearly visible. The bilateral Bruch membrane of the mouse was disrupted by photocoagulation induced by Ar laser at a wavelength of 532 nm. Four laser photocoagulation spots were formed at the posterior pole of the retina, with a power of 100 mW, duration of 100 ms, and a spot size of 50 µm. For all the mice, modeling was performed only on the dosed eye (i.e., the right eye). Another ten wild-type c57 mice of the same age (10-12 weeks old) were subjected to CNV modeling in the right eye. The mice received an intravitreal injection of 2.5 µg Eylea (aflibercept, purchased from Bayer (China) Limited, Beijing, China) in the right eye three days after modeling, which was designated as Group G5. (Since the vitreal half-life of the aflibercept protein was only 4.7 days, the conventional practice in CNV mouse models is to perform modeling 2-3 days after administration in order to exert the optimal anti-angiogenic effect. Accordingly, to achieve the best therapeutic effect in Group G5, the mice in Group G5 were administered three days after modeling.) After modeling, all the mice were subjected to the OCT detection to detect each burn site and determine whether the modeling was successful. The results showed that models were successfully constructed in all the mice of Groups G1 to G5 (FIG. 6).

### Example 9: Reduction of Neovascular Leakage by Virus Carrying Aflibercept Expression Cassette in CNV Mouse Model

The mice in Groups G1 to G5 described in Example 8 were subjected to the Fluorescein Fundus Angiography (FFA) examination at Day 7 and Day 14 after CNV modeling. The specific procedures were as follows: After anesthesia, the mice were transferred to an observation stage. An ophthalmoscope was adjusted to observe the visual field of the fundus. sodium fluorescein was injected into the tail vein. Fundus imaging commenced immediately after the injection of sodium fluorescein and lasted for 0 to 5 min. FA images at specific time points were captured for fluorescence leakage scoring (scales I to IV), i.e., evaluation on the severity of neovascular leakage. The captured images were processed using the Image-Pro Plus 6.0 software to calculate the fluorescent spot area, i.e., the neovascular leakage area.

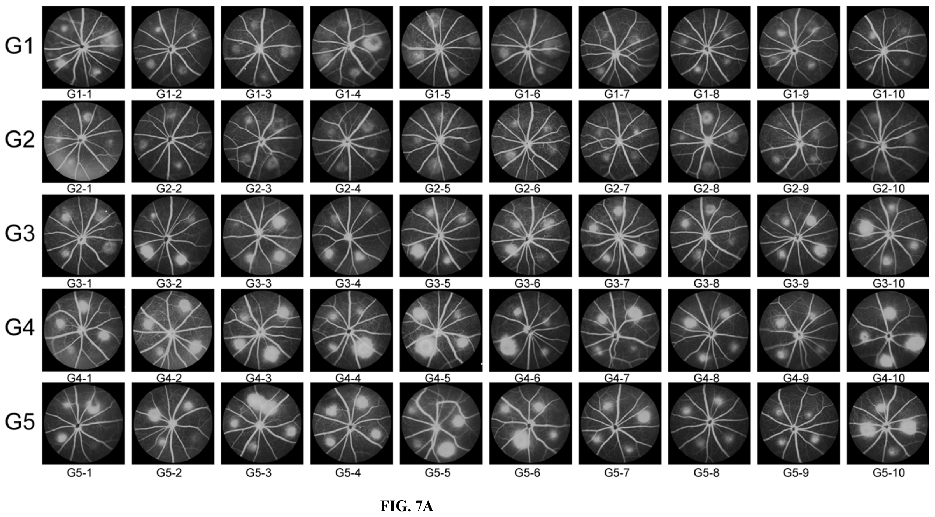

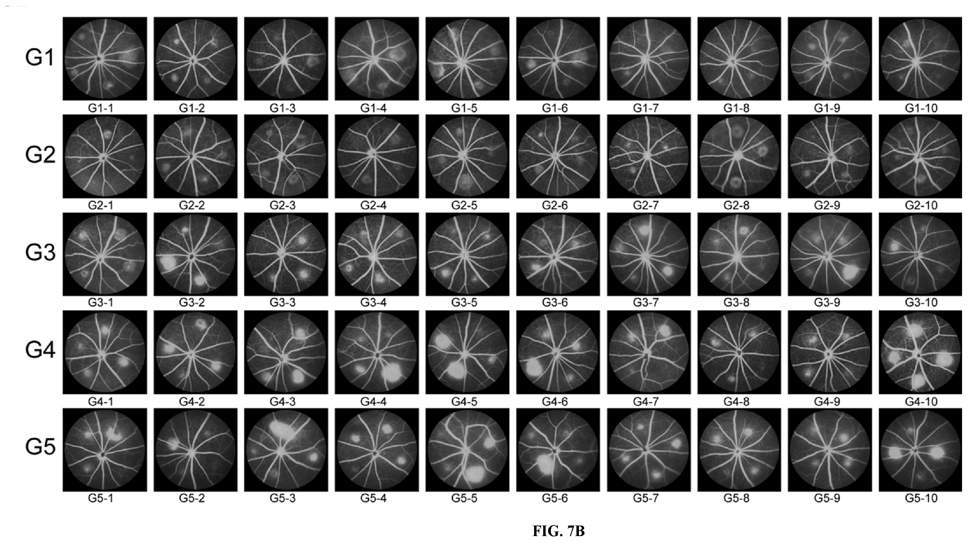

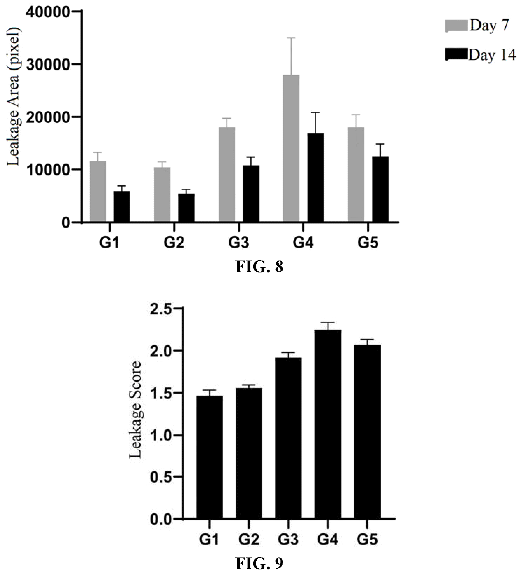

The FFA examination results showed that at Day 7 and Day 14 after modeling, the fluorescent spot areas and intensities (i.e., the severity of neovascular leakage) in the mice of Groups G1 and G2 were significantly less than those in the mice of Groups G3 to G5 (FIGS. 7A and 7B). The results of fluorescence area calculated using Image-Pro Plus 6.0 showed that the fluorescent spot areas at Day 7 to Day 14 after modeling in the mice of Groups G1 and G2 were considerably decreased, of which the decrease in Group G2 was more remarkable (FIG. 8), suggesting a better improvement in the neovascular leakage.

In this example, technicians with expertise in ophthalmology were invited to score the neovascular leakage (fluorescent spot area and intensity) in the images captured during FFA examination in a randomized, double-blind manner. The scoring was performed according to the standard described in the literature (Song, J.H, et al. J. Mol. Sci. 2020; 21(8), 2842.). Three technicians were invited to score all the fluorescent spots separately and the average score for each fluorescent spot was calculated. The results showed that the mice of Groups G1 and G2 exhibited a lower score of neovascular leakage at Day 14 after modeling, suggesting less leakage (FIG. 9).

### Example 10: Reduction of Neovascular Leakage by Virus Carrying Aflibercept Expression Cassette in Cynomolgus Monkey CNV Model

The rAAV IVT15-CB7-hIL2sp-Aflibercept opt3 was selected for further functional studies. Four male cynomolgus monkeys of 3 to 5 years old were randomly assigned to two groups, with two cynomolgus monkeys in each group. Either of the groups received an intravitreal injection, at a dose of 2.5 E +11 (i.e., 2.5×10¹¹) vg, of rAAV IVT15-CB7-hIL2sp-Aflibercept opt3 into both eyes in a volume of 50 µL (N1); and the other group received an intravitreal injection of 50 µL of PBS into both eyes (N2). The two groups of animals were designated as Group N1 and Group N2, respectively. The above animals were subjected to laser-induced modeling at Day 28 after injection to construct choroidal neovascularization (CNV) models in cynomolgus monkeys. The specific procedures were as follows: 1) Pupil dilation: compound tropicamide eye drops were instilled into both eyes of the animals for pupil dilation. 2) Anesthesia: intramuscular anesthesia was performed first (Zoletil 50, 5 mg/kg, concentration: 50 mg/mL; combined with xylazine hydrochloride injection, 0.4 mg/kg, concentration: 20 mg/mL). Anesthetic had been supplemented depending on the anesthetic state of the animals. Benzoxazole hydrochloride injection was injected at the same volume as that of the xylazine hydrochloride injection to help the animals recover quickly. 3) Laser photocoagulation: a corneal contact lens was coated with carbomer gel and placed in front of the cornea of the animal. After the fundus was clearly observed, photocoagulation was performed at approximately 1.5 to 2 PD from the fovea centralis, avoiding major vessels. The laser parameters were as follows: wavelength: 532 nm, power: 400 to 600 mW, spot diameter: 50 µm, and exposure time: 100 ms. 4) OCT and FP examinations were performed immediately after modeling to confirm that the Bruch's membrane was punctured without severe hemorrhage, indicating successful modeling. The animals were subjected to the Fluorescein Fundus Angiography (FFA) examination at Day 14 and Day 28 after CNV modeling. The specific procedures were as follows: After anesthesia, the monkeys were transferred to an observation stage. An ophthalmoscope was adjusted to observe the visual field of the fundus. Sodium fluorescein was injected into the tail vein. Fundus imaging commenced immediately after the injection of sodium fluorescein and lasted for 0 to 5 min. FA images at specific time points were captured for fluorescence leakage grading (scales I to IV), i.e., evaluation on the severity of neovascular leakage. The FFA examination results showed that at Day 14 and Day 28 after modeling, the fluorescent spot areas and intensities (i.e., the severity of neovascular leakage) in the animals of Group N1 were significantly less than those in the animals of Group N2 (FIG. 10), indicating that rAAV IVT15-CB7-hIL2sp-Aflibercept opt3 suppressed CNV formation.

In this example, technicians with expertise in ophthalmology were invited to score the neovascular leakage (based on the fluorescent spot area and intensity) in the images captured during FFA examination in a randomized, double-blind manner. The scoring was performed according to the standard described in the literature (Song, J.H, et al. J. Mol. Sci. 2020; 21(8), 2842.). Three technicians were invited to score all the fluorescent spots separately. The results showed that the animals in Group N1 exhibited lower proportions of grade 4 fluorescent spots at Day 14 and Day 28 after modeling (FIG. 11).

### Nucleotide sequences of some gene expression cassettes and vectors in Example 1 and Example 3

SEQ ID NO: 35 (CMV-hAlbumin sp-Aflibercept opt3-hGH pA) 
5’ACGCGTCTAGTTATTAATAGTAATCAATTACGGGGTCATTAGTTCATAGCCCAT
ATATGGAGTTCCGCGTTACATAACTTACGGTAAATGGCCCGCCTGGCTGACCGCCCA
ACGACCCCCGCCCATTGACGTCAATAATGACGTATGTTCCCATAGTAACGCCAATAG
GGACTTTCCATTGACGTCAATGGGTGGAGTATTTACGGTAAACTGCCCACTTGGCAG
TACATCAAGTGTATCATATGCCAAGTACGCCCCCTATTGACGTCAATGACGGTAAATG
GCCCGCCTGGCATTATGCCCAGTACATGACCTTATGGGACTTTCCTACTTGGCAGTA
CATCTACGTATTAGTCATCGCTATTACCATGGTGATGCGGTTTTGGCAGTACATCAAT
GGGCGTGGATAGCGGTTTGACTCACGGGGATTTCCAAGTCTCCACCCCATTGACGT
CAATGGGAGTTTGTTTTGGCACCAAAATCAACGGGACTTTCCAAAATGTCGTAACA
ACTCCGCCCCATTGACGCAAATGGGCGGTAGGCGTGTACGGTGGGAGGTCTATATAA
GCAGAGCTCGTTTAGTGAACCGTCAGATCGCCTGGAGACGCCATCCACGCTGTTTT
GACCTCCATAGAAGACACCGGGACCGATCCAGCCTCCGCGGATTCGAATCCCGGCC
GGGAACGGTGCATTGGAACGCGGATTCCCCGTGCCAAGAGTGACGTAAGTACCGCC
TATAGAGTCTATAGGCCCACAAAAAATGCTTTCTTCTTTTAATATACTTTTTTGTTTAT
CTTATTTCTAATACTTTCCCTAATCTCTTTCTTTCAGGGCAATAATGATACAATGTATC
ATGCCTCTTTGCACCATTCTAAAGAATAACAGTGATAATTTCTGGGTTAAGGCAATAG
CAATATTTCTGCATATAAATATTTCTGCATATAAATTGTAACTGATGTAAGAGGTTTCA
TATTGCTAATAGCAGCTACAATCCAGCTACCATTCTGCTTTTATTTTATGGTTGGGATA
AGGCTGGATTATTCTGAGTCCAAGCTAGGCCCTTTTGCTAATCATGTTCATACCTCTT
ATCTTCCTCCCACAGCTCCTGGGCAACGTGCTGGTCTGTGTGCTGGCCCATCACTTT
GGCAAAGAATTGGGATAATTACTAGATCGCCACCATGAAGTGGGTGACCTTCATCAG
CCTGCTGTTCCTGTTTTCTTCAGCCTACAGCTCTGACACAGGCAGACCCTTTGTGGA
AATGTACTCTGAGATCCCTGAGATCATCCACATGACAGAGGGCAGAGAGCTGGTCA
TCCCCTGCAGAGTGACAAGCCCCAACATCACAGTGACCCTGAAGAAGTTCCCTCTG
GACACACTGATCCCTGATGGCAAGAGAATCATCTGGGACAGCAGAAAGGGCTTCAT
 
CATCAGCAATGCCACCTACAAAGAGATTGGCCTGCTGACCTGTGAAGCCACAGTGA
ATGGCCACCTGTACAAGACCAACTACCTGACACACAGACAGACCAACACCATCATT
GATGTGGTGCTGAGCCCCAGCCATGGCATTGAGCTGTCTGTGGGAGAGAAGCTGGT
GCTGAACTGCACAGCCAGAACAGAGCTGAATGTGGGCATTGACTTCAACTGGGAGT
ACCCCAGCAGCAAGCACCAGCACAAGAAACTGGTCAACAGGGACCTGAAAACCC
AGAGTGGCTCTGAGATGAAGAAATTCCTGAGCACCCTGACCATTGATGGGGTCACC
AGATCTGACCAGGGCCTGTACACATGTGCTGCCAGCTCTGGACTGATGACCAAGAA
AAACAGCACCTTTGTCAGAGTGCATGAGAAGGACAAGACCCACACCTGTCCTCCAT
GTCCTGCACCTGAGCTGCTTGGAGGCCCCTCTGTGTTCCTGTTTCCTCCAAAGCCTA
AGGACACCCTGATGATCAGCAGAACACCTGAAGTGACCTGTGTGGTGGTGGATGTG
TCCCATGAGGACCCAGAAGTGAAGTTCAATTGGTATGTGGATGGGGTTGAAGTGCA
CAATGCCAAGACCAAGCCTAGAGAGGAACAGTACAACTCCACCTACAGAGTGGTG
TCAGTGCTGACAGTGCTGCACCAGGACTGGCTGAATGGCAAAGAGTACAAGTGCA
AGGTGTCCAACAAGGCCCTGCCTGCTCCTATTGAGAAAACCATCTCCAAGGCCAAG
GGCCAGCCTAGGGAACCCCAGGTTTACACACTGCCACCTAGCAGGGATGAGCTGAC
AAAGAACCAGGTGTCCCTGACCTGCCTGGTCAAGGGCTTCTACCCCTCTGACATTG
CTGTGGAATGGGAGAGCAATGGCCAGCCTGAGAACAACTACAAGACAACCCCTCC
TGTGCTGGACTCTGATGGCTCATTCTTCCTGTACAGCAAGCTGACTGTGGACAAGA
GCAGATGGCAGCAGGGAAATGTGTTCAGCTGCTCTGTGATGCATGAGGCCCTGCAC
AACCACTACACCCAGAAAAGCCTGAGCCTGTCTCCTGGCAAGTGAGCGGCCGCAA
GCTTCGGGTGGCATCCCTGTGACCCCTCCCCAGTGCCTCTCCTGGCCCTGGAAGTT
GCCACTCCAGTGCCCACCAGCCTTGTCCTAATAAAATTAAGTTGCATCATTTTGTCTG
ACTAGGTGTCCTTCTATAATATTATGGGGTGGAGGGGGGTGGTATGGAGCAAGGGGC
AAGTTGGGAAGACAACCTGTAGGGCCTGCGGGGTCTATTGGGAACCAAGCTGGAG
TGCAGTGGCACAATCTTGGCTCACTGCAATCTCCGCCTCCTGGGTTCAAGCGATTCT
CCTGCCTCAGCCTCCCGAGTTGTTGGGATTCCAGGCATGCATGACCAGGCTCAGCTA
ATTTTTGTTTTTTTGGTAGAGACGGGGTTTCACCATATTGGCCAGGCTGGTCTCCAA
CTCCTAATCTCAGGTGATCTACCCACCTTGGCCTCCCAAATTGCTGGGATTACAGGC
GTGAACCACTGCTCCCTTCCCTGTCCTTCGG3’
SEQ ID NO: 36 (CBh-hAlbumin sp-Aflibercept opt3-hGH pA) 
5'ACGCGTCTAGTTATTAATAGTAATCAATTACGGGGTCATTAGTTCATAGCCCAT
ATATGGAGTTCCGCGTTACATAACTTACGGTAAATGGCCCGCCTGGCTGACCGCCCA
ACGACCCCCGCCCATTGACGTCAATAATGACGTATGTTCCCATAGTAACGCCAATAG
GGACTTTCCATTGACGTCAATGGGTGGAGTATTTACGGTAAACTGCCCACTTGGCAG
 
TACATCAAGTGTATCATATGCCAAGTACGCCCCCTATTGACGTCAATGACGGTAAATG
GCCCGCCTGGCATTATGCCCAGTACATGACCTTACGGGACTTTCCTACTTGGCAGTA
CATCTACGTATTAGTCATCGCTATTACCATGGTCGAGGTGAGCCCCACGTTCTGCTTC
ACTCTCCCCATCTCCCCCCCCTCCCCACCCCCAATTTTGTATTTATTTATTTTTTAATTA
TTTTGTGCAGCGATGGGGGCGGGGGGGGGGGGGGCGCGCGCCAGGCGGGGCGGG
GCGGGGCGAGGGGCGGGGCGGGGCGAGGCGGAGAGGTGCGGCGGCAGCCAATCA
GAGCGGCGCGCTCCGAAAGTTTCCTTTTATGGCGAGGCGGCGGCGGCGGCGGCCCT
ATAAAAAGCGAAGCGCGCGGCGGGCGGGGAGTCGCTGCGTTGCCTTCGCCCCGTG
CCCCGCTCCGCGCCGCCTCGCGCCGCCCGCCCCGGCTCTGACTGACCGCGTTACTC
CCACAGGTGAGCGGGCGGGACGGCCCTTCTCCTCCGGGCTGTAATTAGCAAGAGGT
AAGGGTTTAAGGGATGGTTGGTTGGTGGGGTATTAATGTTTAATTACCTGTTTTACAG
GCCTGAAATCACTTGGTTTTAGGTTGGAATTACTAGATCGCCACCATGAAGTGGGTG
ACCTTCATCAGCCTGCTGTTCCTGTTTTCTTCAGCCTACAGCTCTGACACAGGCAGA
CCCTTTGTGGAAATGTACTCTGAGATCCCTGAGATCATCCACATGACAGAGGGCAG
AGAGCTGGTCATCCCCTGCAGAGTGACAAGCCCCAACATCACAGTGACCCTGAAG
AAGTTCCCTCTGGACACACTGATCCCTGATGGCAAGAGAATCATCTGGGACAGCAG
AAAGGGCTTCATCATCAGCAATGCCACCTACAAAGAGATTGGCCTGCTGACCTGTG
AAGCCACAGTGAATGGCCACCTGTACAAGACCAACTACCTGACACACAGACAGAC
CAACACCATCATTGATGTGGTGCTGAGCCCCAGCCATGGCATTGAGCTGTCTGTGGG
AGAGAAGCTGGTGCTGAACTGCACAGCCAGAACAGAGCTGAATGTGGGCATTGAC
TTCAACTGGGAGTACCCCAGCAGCAAGCACCAGCACAAGAAACTGGTCAACAGGG
ACCTGAAAACCCAGAGTGGCTCTGAGATGAAGAAATTCCTGAGCACCCTGACCATT
GATGGGGTCACCAGATCTGACCAGGGCCTGTACACATGTGCTGCCAGCTCTGGACT
GATGACCAAGAAAAACAGCACCTTTGTCAGAGTGCATGAGAAGGACAAGACCCAC
ACCTGTCCTCCATGTCCTGCACCTGAGCTGCTTGGAGGCCCCTCTGTGTTCCTGTTT
CCTCCAAAGCCTAAGGACACCCTGATGATCAGCAGAACACCTGAAGTGACCTGTGT
GGTGGTGGATGTGTCCCATGAGGACCCAGAAGTGAAGTTCAATTGGTATGTGGATG
GGGTTGAAGTGCACAATGCCAAGACCAAGCCTAGAGAGGAACAGTACAACTCCAC
CTACAGAGTGGTGTCAGTGCTGACAGTGCTGCACCAGGACTGGCTGAATGGCAAA
GAGTACAAGTGCAAGGTGTCCAACAAGGCCCTGCCTGCTCCTATTGAGAAAACCAT
CTCCAAGGCCAAGGGCCAGCCTAGGGAACCCCAGGTTTACACACTGCCACCTAGCA
GGGATGAGCTGACAAAGAACCAGGTGTCCCTGACCTGCCTGGTCAAGGGCTTCTAC
CCCTCTGACATTGCTGTGGAATGGGAGAGCAATGGCCAGCCTGAGAACAACTACAA
GACAACCCCTCCTGTGCTGGACTCTGATGGCTCATTCTTCCTGTACAGCAAGCTGAC
TGTGGACAAGAGCAGATGGCAGCAGGGAAATGTGTTCAGCTGCTCTGTGATGCATG
 
AGGCCCTGCACAACCACTACACCCAGAAAAGCCTGAGCCTGTCTCCTGGCAAGTG
AGCGGCCGCAAGCTTCGGGTGGCATCCCTGTGACCCCTCCCCAGTGCCTCTCCTGG
CCCTGGAAGTTGCCACTCCAGTGCCCACCAGCCTTGTCCTAATAAAATTAAGTTGCA
TCATTTTGTCTGACTAGGTGTCCTTCTATAATATTATGGGGTGGAGGGGGGTGGTATG
GAGCAAGGGGCAAGTTGGGAAGACAACCTGTAGGGCCTGCGGGGTCTATTGGGAA
CCAAGCTGGAGTGCAGTGGCACAATCTTGGCTCACTGCAATCTCCGCCTCCTGGGT
TCAAGCGATTCTCCTGCCTCAGCCTCCCGAGTTGTTGGGATTCCAGGCATGCATGAC
CAGGCTCAGCTAATTTTTGTTTTTTTGGTAGAGACGGGGTTTCACCATATTGGCCAG
GCTGGTCTCCAACTCCTAATCTCAGGTGATCTACCCACCTTGGCCTCCCAAATTGCT
GGGATTACAGGCGTGAACCACTGCTCCCTTCCCTGTCCTTCGG3'
SEQ ID NO: 37 (CB7-hAlbumin sp-Aflibercept opt3-hGH pA) 
5'ACGCGTCTAGTTATTAATAGTAATCAATTACGGGGTCATTAGTTCATAGCCCAT
ATATGGAGTTCCGCGTTACATAACTTACGGTAAATGGCCCGCCTGGCTGACCGCCCA
ACGACCCCCGCCCATTGACGTCAATAATGACGTATGTTCCCATAGTAACGCCAATAG
GGACTTTCCATTGACGTCAATGGGTGGAGTATTTACGGTAAACTGCCCACTTGGCAG
TACATCAAGTGTATCATATGCCAAGTACGCCCCCTATTGACGTCAATGACGGTAAATG
GCCCGCCTGGCATTATGCCCAGTACATGACCTTACGGGACTTTCCTACTTGGCAGTA
CATCTACGTATTAGTCATCGCTATTACCATGGTCGAGGTGAGCCCCACGTTCTGCTTC
ACTCTCCCCATCTCCCCCCCCTCCCCACCCCCAATTTTGTATTTATTTATTTTTTAATTA
TTTTGTGCAGCGATGGGGGCGGGGGGGGGGGGGGCGCGCGCCAGGCGGGGCGGG
GCGGGGCGAGGGGCGGGGCGGGGCGAGGCGGAGAGGTGCGGCGGCAGCCAATCA
GAGCGGCGCGCTCCGAAAGTTTCCTTTTATGGCGAGGCGGCGGCGGCGGCGGCCCT
ATAAAAAGCGAAGCGCGCGGCGGGCGGGAGTCGCTGCGCGCTGCCTTCGCCCCGT
GCCCCGCTCCGCCGCCGCCTCGCGCCGCCCGCCCCGGCTCTGACTGACCGCGTTAC
TCCCACAGGTGAGCGGGCGGGACGGCCCTTCTCCTCCGGGCTGTAATTAGCGCTTG
GTTTAATGACGGCTTGTTTCTTTTCTGTGGCTGCGTGAAAGCCTTGAGGGGCTCCGG
GAGGGCCCTTTGTGCGGGGGGAGCGGCTCGGGGGGTGCGTGCGTGTGTGTGTGCG
TGGGGAGCGCCGCGTGCGGCTCCGCGCTGCCCGGCGGCTGTGAGCGCTGCGGGCG
CGGCGCGGGGCTTTGTGCGCTCCGCAGTGTGCGCGAGGGGAGCGCGGCCGGGGGC
GGTGCCCCGCGGTGCGGGGGGGGCTGCGAGGGGAACAAAGGCTGCGTGCGGGGT
GTGTGCGTGGGGGGGTGAGCAGGGGGTGTGGGCGCGTCGGTCGGGCTGCAACCCC
CCCTGCACCCCCCTCCCCGAGTTGCTGAGCACGGCCCGGCTTCGGGTGCGGGGCTC
CGTACGGGGCGTGGCGCGGGGCTCGCCGTGCCGGGCGGGGGGTGGCGGCAGGTGG
GGGTGCCGGGCGGGGCGGGGCCGCCTCGGGCCGGGGAGGGCTCGGGGGAGGGGC
 
GCGGCGGCCCCCGGAGCGCCGGCGGCTGTCGAGGCGCGGCGAGCCGCAGCCATTG
CCTTTTATGGTAATCGTGCGAGAGGGCGCAGGGACTTCCTTTGTCCCAAATCTGTGC
GGAGCCGAAATCTGGGAGGCGCCGCCGCACCCCCTCTAGCGGGCGCGGGGCGAAG
CGGTGCGGCGCCGGCAGGAAGGAAATGGGCGGGGAGGGCCTTCGTGCGTCGCCGC
GCCGCCGTCCCCTTCTCCCTCTCCAGCCTCGGGGCTGTCCGCGGGGGGACGGCTGC
CTTCGGGGGGGACGGGGCAGGGCGGGGTTCGGCTTCTGGCGTGTGACCGGCGGCT
CTAGAGCCTCTGCTAACCATGTTCATGCCTTCTTCTTTTTCCTACAGCTCCTGGGCAA
CGTGCTGGTTATTGTGCTGTCTCATCATTTTGGCAAAGAATTACTAGATCGCCACCAT
GAAGTGGGTGACCTTCATCAGCCTGCTGTTCCTGTTTTCTTCAGCCTACAGCTCTGA
CACAGGCAGACCCTTTGTGGAAATGTACTCTGAGATCCCTGAGATCATCCACATGAC
AGAGGGCAGAGAGCTGGTCATCCCCTGCAGAGTGACAAGCCCCAACATCACAGTG
ACCCTGAAGAAGTTCCCTCTGGACACACTGATCCCTGATGGCAAGAGAATCATCTG
GGACAGCAGAAAGGGCTTCATCATCAGCAATGCCACCTACAAAGAGATTGGCCTGC
TGACCTGTGAAGCCACAGTGAATGGCCACCTGTACAAGACCAACTACCTGACACAC
AGACAGACCAACACCATCATTGATGTGGTGCTGAGCCCCAGCCATGGCATTGAGCT
GTCTGTGGGAGAGAAGCTGGTGCTGAACTGCACAGCCAGAACAGAGCTGAATGTG
GGCATTGACTTCAACTGGGAGTACCCCAGCAGCAAGCACCAGCACAAGAAACTGG
TCAACAGGGACCTGAAAACCCAGAGTGGCTCTGAGATGAAGAAATTCCTGAGCAC
CCTGACCATTGATGGGGTCACCAGATCTGACCAGGGCCTGTACACATGTGCTGCCA
GCTCTGGACTGATGACCAAGAAAAACAGCACCTTTGTCAGAGTGCATGAGAAGGA
CAAGACCCACACCTGTCCTCCATGTCCTGCACCTGAGCTGCTTGGAGGCCCCTCTG
TGTTCCTGTTTCCTCCAAAGCCTAAGGACACCCTGATGATCAGCAGAACACCTGAA
GTGACCTGTGTGGTGGTGGATGTGTCCCATGAGGACCCAGAAGTGAAGTTCAATTG
GTATGTGGATGGGGTTGAAGTGCACAATGCCAAGACCAAGCCTAGAGAGGAACAGT
ACAACTCCACCTACAGAGTGGTGTCAGTGCTGACAGTGCTGCACCAGGACTGGCTG
AATGGCAAAGAGTACAAGTGCAAGGTGTCCAACAAGGCCCTGCCTGCTCCTATTGA
GAAAACCATCTCCAAGGCCAAGGGCCAGCCTAGGGAACCCCAGGTTTACACACTG
CCACCTAGCAGGGATGAGCTGACAAAGAACCAGGTGTCCCTGACCTGCCTGGTCA
AGGGCTTCTACCCCTCTGACATTGCTGTGGAATGGGAGAGCAATGGCCAGCCTGAG
AACAACTACAAGACAACCCCTCCTGTGCTGGACTCTGATGGCTCATTCTTCCTGTAC
AGCAAGCTGACTGTGGACAAGAGCAGATGGCAGCAGGGAAATGTGTTCAGCTGCT
CTGTGATGCATGAGGCCCTGCACAACCACTACACCCAGAAAAGCCTGAGCCTGTCT
CCTGGCAAGTGAGCGGCCGCAAGCTTCGGGTGGCATCCCTGTGACCCCTCCCCAGT
GCCTCTCCTGGCCCTGGAAGTTGCCACTCCAGTGCCCACCAGCCTTGTCCTAATAAA
ATTAAGTTGCATCATTTTGTCTGACTAGGTGTCCTTCTATAATATTATGGGGTGGAGG
 
GGGGTGGTATGGAGCAAGGGGCAAGTTGGGAAGACAACCTGTAGGGCCTGCGGGG
TCTATTGGGAACCAAGCTGGAGTGCAGTGGCACAATCTTGGCTCACTGCAATCTCC
GCCTCCTGGGTTCAAGCGATTCTCCTGCCTCAGCCTCCCGAGTTGTTGGGATTCCAG
GCATGCATGACCAGGCTCAGCTAATTTTTGTTTTTTTGGTAGAGACGGGGTTTCACC
ATATTGGCCAGGCTGGTCTCCAACTCCTAATCTCAGGTGATCTACCCACCTTGGCCT
CCCAAATTGCTGGGATTACAGGCGTGAACCACTGCTCCCTTCCCTGTCCTTCGG3’
SEQ ID NO: 38 (CMV-hOpticin sp-Aflibercept opt3-hGH pA) 
5’ACGCGTCTAGTTATTAATAGTAATCAATTACGGGGTCATTAGTTCATAGCCCAT
ATATGGAGTTCCGCGTTACATAACTTACGGTAAATGGCCCGCCTGGCTGACCGCCCA
ACGACCCCCGCCCATTGACGTCAATAATGACGTATGTTCCCATAGTAACGCCAATAG
GGACTTTCCATTGACGTCAATGGGTGGAGTATTTACGGTAAACTGCCCACTTGGCAG
TACATCAAGTGTATCATATGCCAAGTACGCCCCCTATTGACGTCAATGACGGTAAATG
GCCCGCCTGGCATTATGCCCAGTACATGACCTTATGGGACTTTCCTACTTGGCAGTA
CATCTACGTATTAGTCATCGCTATTACCATGGTGATGCGGTTTTGGCAGTACATCAAT
GGGCGTGGATAGCGGTTTGACTCACGGGGATTTCCAAGTCTCCACCCCATTGACGT
CAATGGGAGTTTGTTTTGGCACCAAAATCAACGGGACTTTCCAAAATGTCGTAACA
ACTCCGCCCCATTGACGCAAATGGGCGGTAGGCGTGTACGGTGGGAGGTCTATATAA
GCAGAGCTCGTTTAGTGAACCGTCAGATCGCCTGGAGACGCCATCCACGCTGTTTT
GACCTCCATAGAAGACACCGGGACCGATCCAGCCTCCGCGGATTCGAATCCCGGCC
GGGAACGGTGCATTGGAACGCGGATTCCCCGTGCCAAGAGTGACGTAAGTACCGCC
TATAGAGTCTATAGGCCCACAAAAAATGCTTTCTTCTTTTAATATACTTTTTTGTTTAT
CTTATTTCTAATACTTTCCCTAATCTCTTTCTTTCAGGGCAATAATGATACAATGTATC
ATGCCTCTTTGCACCATTCTAAAGAATAACAGTGATAATTTCTGGGTTAAGGCAATAG
CAATATTTCTGCATATAAATATTTCTGCATATAAATTGTAACTGATGTAAGAGGTTTCA
TATTGCTAATAGCAGCTACAATCCAGCTACCATTCTGCTTTTATTTTATGGTTGGGATA
AGGCTGGATTATTCTGAGTCCAAGCTAGGCCCTTTTGCTAATCATGTTCATACCTCTT
ATCTTCCTCCCACAGCTCCTGGGCAACGTGCTGGTCTGTGTGCTGGCCCATCACTTT
GGCAAAGAATTGGGATAATTACTAGATCGCCACCATGAGACTGCTGGCCTTCCTGAG
CCTGCTGGCCCTGGTGCTGCAGGAGACAGGCACCTCTGACACAGGCAGACCCTTT
GTGGAAATGTACTCTGAGATCCCTGAGATCATCCACATGACAGAGGGCAGAGAGCT
GGTCATCCCCTGCAGAGTGACAAGCCCCAACATCACAGTGACCCTGAAGAAGTTCC
CTCTGGACACACTGATCCCTGATGGCAAGAGAATCATCTGGGACAGCAGAAAGGGC
TTCATCATCAGCAATGCCACCTACAAAGAGATTGGCCTGCTGACCTGTGAAGCCAC
AGTGAATGGCCACCTGTACAAGACCAACTACCTGACACACAGACAGACCAACACC
 
ATCATTGATGTGGTGCTGAGCCCCAGCCATGGCATTGAGCTGTCTGTGGGAGAGAA
GCTGGTGCTGAACTGCACAGCCAGAACAGAGCTGAATGTGGGCATTGACTTCAACT
GGGAGTACCCCAGCAGCAAGCACCAGCACAAGAAACTGGTCAACAGGGACCTGA
AAACCCAGAGTGGCTCTGAGATGAAGAAATTCCTGAGCACCCTGACCATTGATGGG
GTCACCAGATCTGACCAGGGCCTGTACACATGTGCTGCCAGCTCTGGACTGATGAC
CAAGAAAAACAGCACCTTTGTCAGAGTGCATGAGAAGGACAAGACCCACACCTGT
CCTCCATGTCCTGCACCTGAGCTGCTTGGAGGCCCCTCTGTGTTCCTGTTTCCTCCA
AAGCCTAAGGACACCCTGATGATCAGCAGAACACCTGAAGTGACCTGTGTGGTGGT
GGATGTGTCCCATGAGGACCCAGAAGTGAAGTTCAATTGGTATGTGGATGGGGTTG
AAGTGCACAATGCCAAGACCAAGCCTAGAGAGGAACAGTACAACTCCACCTACAG
AGTGGTGTCAGTGCTGACAGTGCTGCACCAGGACTGGCTGAATGGCAAAGAGTAC
AAGTGCAAGGTGTCCAACAAGGCCCTGCCTGCTCCTATTGAGAAAACCATCTCCAA
GGCCAAGGGCCAGCCTAGGGAACCCCAGGTTTACACACTGCCACCTAGCAGGGAT
GAGCTGACAAAGAACCAGGTGTCCCTGACCTGCCTGGTCAAGGGCTTCTACCCCTC
TGACATTGCTGTGGAATGGGAGAGCAATGGCCAGCCTGAGAACAACTACAAGACA
ACCCCTCCTGTGCTGGACTCTGATGGCTCATTCTTCCTGTACAGCAAGCTGACTGTG
GACAAGAGCAGATGGCAGCAGGGAAATGTGTTCAGCTGCTCTGTGATGCATGAGGC
CCTGCACAACCACTACACCCAGAAAAGCCTGAGCCTGTCTCCTGGCAAGTGAGCG
GCCGCAAGCTTCGGGTGGCATCCCTGTGACCCCTCCCCAGTGCCTCTCCTGGCCCT
GGAAGTTGCCACTCCAGTGCCCACCAGCCTTGTCCTAATAAAATTAAGTTGCATCAT
TTTGTCTGACTAGGTGTCCTTCTATAATATTATGGGGTGGAGGGGGGTGGTATGGAG
CAAGGGGCAAGTTGGGAAGACAACCTGTAGGGCCTGCGGGGTCTATTGGGAACCA
AGCTGGAGTGCAGTGGCACAATCTTGGCTCACTGCAATCTCCGCCTCCTGGGTTCA
AGCGATTCTCCTGCCTCAGCCTCCCGAGTTGTTGGGATTCCAGGCATGCATGACCAG
GCTCAGCTAATTTTTGTTTTTTTGGTAGAGACGGGGTTTCACCATATTGGCCAGGCT
GGTCTCCAACTCCTAATCTCAGGTGATCTACCCACCTTGGCCTCCCAAATTGCTGGG
ATTACAGGCGTGAACCACTGCTCCCTTCCCTGTCCTTCGG3'
SEQ ID NO: 39 (CBh-hOpticin sp-Aflibercept opt3-hGH pA) 
5'ACGCGTCTAGTTATTAATAGTAATCAATTACGGGGTCATTAGTTCATAGCCCAT
ATATGGAGTTCCGCGTTACATAACTTACGGTAAATGGCCCGCCTGGCTGACCGCCCA
ACGACCCCCGCCCATTGACGTCAATAATGACGTATGTTCCCATAGTAACGCCAATAG
GGACTTTCCATTGACGTCAATGGGTGGAGTATTTACGGTAAACTGCCCACTTGGCAG
TACATCAAGTGTATCATATGCCAAGTACGCCCCCTATTGACGTCAATGACGGTAAATG
GCCCGCCTGGCATTATGCCCAGTACATGACCTTACGGGACTTTCCTACTTGGCAGTA
 
CATCTACGTATTAGTCATCGCTATTACCATGGTCGAGGTGAGCCCCACGTTCTGCTTC
ACTCTCCCCATCTCCCCCCCCTCCCCACCCCCAATTTTGTATTTATTTATTTTTTAATTA
TTTTGTGCAGCGATGGGGGCGGGGGGGGGGGGGGCGCGCGCCAGGCGGGGCGGG
GCGGGGCGAGGGGCGGGGCGGGGCGAGGCGGAGAGGTGCGGCGGCAGCCAATCA
GAGCGGCGCGCTCCGAAAGTTTCCTTTTATGGCGAGGCGGCGGCGGCGGCGGCCCT
ATAAAAAGCGAAGCGCGCGGCGGGCGGGGAGTCGCTGCGTTGCCTTCGCCCCGTG
CCCCGCTCCGCGCCGCCTCGCGCCGCCCGCCCCGGCTCTGACTGACCGCGTTACTC
CCACAGGTGAGCGGGCGGGACGGCCCTTCTCCTCCGGGCTGTAATTAGCAAGAGGT
AAGGGTTTAAGGGATGGTTGGTTGGTGGGGTATTAATGTTTAATTACCTGTTTTACAG
GCCTGAAATCACTTGGTTTTAGGTTGGAATTACTAGATCGCCACCATGAGACTGCTG
GCCTTCCTGAGCCTGCTGGCCCTGGTGCTGCAGGAGACAGGCACCTCTGACACAG
GCAGACCCTTTGTGGAAATGTACTCTGAGATCCCTGAGATCATCCACATGACAGAG
GGCAGAGAGCTGGTCATCCCCTGCAGAGTGACAAGCCCCAACATCACAGTGACCC
TGAAGAAGTTCCCTCTGGACACACTGATCCCTGATGGCAAGAGAATCATCTGGGAC
AGCAGAAAGGGCTTCATCATCAGCAATGCCACCTACAAAGAGATTGGCCTGCTGAC
CTGTGAAGCCACAGTGAATGGCCACCTGTACAAGACCAACTACCTGACACACAGA
CAGACCAACACCATCATTGATGTGGTGCTGAGCCCCAGCCATGGCATTGAGCTGTCT
GTGGGAGAGAAGCTGGTGCTGAACTGCACAGCCAGAACAGAGCTGAATGTGGGCA
TTGACTTCAACTGGGAGTACCCCAGCAGCAAGCACCAGCACAAGAAACTGGTCAA
CAGGGACCTGAAAACCCAGAGTGGCTCTGAGATGAAGAAATTCCTGAGCACCCTG
ACCATTGATGGGGTCACCAGATCTGACCAGGGCCTGTACACATGTGCTGCCAGCTCT
GGACTGATGACCAAGAAAAACAGCACCTTTGTCAGAGTGCATGAGAAGGACAAGA
CCCACACCTGTCCTCCATGTCCTGCACCTGAGCTGCTTGGAGGCCCCTCTGTGTTCC
TGTTTCCTCCAAAGCCTAAGGACACCCTGATGATCAGCAGAACACCTGAAGTGACC
TGTGTGGTGGTGGATGTGTCCCATGAGGACCCAGAAGTGAAGTTCAATTGGTATGT
GGATGGGGTTGAAGTGCACAATGCCAAGACCAAGCCTAGAGAGGAACAGTACAAC
TCCACCTACAGAGTGGTGTCAGTGCTGACAGTGCTGCACCAGGACTGGCTGAATGG
CAAAGAGTACAAGTGCAAGGTGTCCAACAAGGCCCTGCCTGCTCCTATTGAGAAA
ACCATCTCCAAGGCCAAGGGCCAGCCTAGGGAACCCCAGGTTTACACACTGCCACC
TAGCAGGGATGAGCTGACAAAGAACCAGGTGTCCCTGACCTGCCTGGTCAAGGGC
TTCTACCCCTCTGACATTGCTGTGGAATGGGAGAGCAATGGCCAGCCTGAGAACAA
CTACAAGACAACCCCTCCTGTGCTGGACTCTGATGGCTCATTCTTCCTGTACAGCAA
GCTGACTGTGGACAAGAGCAGATGGCAGCAGGGAAATGTGTTCAGCTGCTCTGTGA
TGCATGAGGCCCTGCACAACCACTACACCCAGAAAAGCCTGAGCCTGTCTCCTGGC
AAGTGAGCGGCCGCAAGCTTCGGGTGGCATCCCTGTGACCCCTCCCCAGTGCCTCT
 
CCTGGCCCTGGAAGTTGCCACTCCAGTGCCCACCAGCCTTGTCCTAATAAAATTAAG
TTGCATCATTTTGTCTGACTAGGTGTCCTTCTATAATATTATGGGGTGGAGGGGGGTG
GTATGGAGCAAGGGGCAAGTTGGGAAGACAACCTGTAGGGCCTGCGGGGTCTATTG
GGAACCAAGCTGGAGTGCAGTGGCACAATCTTGGCTCACTGCAATCTCCGCCTCCT
GGGTTCAAGCGATTCTCCTGCCTCAGCCTCCCGAGTTGTTGGGATTCCAGGCATGCA
TGACCAGGCTCAGCTAATTTTTGTTTTTTTGGTAGAGACGGGGTTTCACCATATTGG
CCAGGCTGGTCTCCAACTCCTAATCTCAGGTGATCTACCCACCTTGGCCTCCCAAAT
TGCTGGGATTACAGGCGTGAACCACTGCTCCCTTCCCTGTCCTTCGG3'
SEQ ID NO: 40 (CB7-hOpticin sp-Aflibercept opt3-hGH pA) 
5'ACGCGTCTAGTTATTAATAGTAATCAATTACGGGGTCATTAGTTCATAGCCCAT
ATATGGAGTTCCGCGTTACATAACTTACGGTAAATGGCCCGCCTGGCTGACCGCCCA
ACGACCCCCGCCCATTGACGTCAATAATGACGTATGTTCCCATAGTAACGCCAATAG
GGACTTTCCATTGACGTCAATGGGTGGAGTATTTACGGTAAACTGCCCACTTGGCAG
TACATCAAGTGTATCATATGCCAAGTACGCCCCCTATTGACGTCAATGACGGTAAATG
GCCCGCCTGGCATTATGCCCAGTACATGACCTTACGGGACTTTCCTACTTGGCAGTA
CATCTACGTATTAGTCATCGCTATTACCATGGTCGAGGTGAGCCCCACGTTCTGCTTC
ACTCTCCCCATCTCCCCCCCCTCCCCACCCCCAATTTTGTATTTATTTATTTTTTAATTA
TTTTGTGCAGCGATGGGGGCGGGGGGGGGGGGGGCGCGCGCCAGGCGGGGCGGG
GCGGGGCGAGGGGCGGGGCGGGGCGAGGCGGAGAGGTGCGGCGGCAGCCAATCA
GAGCGGCGCGCTCCGAAAGTTTCCTTTTATGGCGAGGCGGCGGCGGCGGCGGCCCT
ATAAAAAGCGAAGCGCGCGGCGGGCGGGAGTCGCTGCGCGCTGCCTTCGCCCCGT
GCCCCGCTCCGCCGCCGCCTCGCGCCGCCCGCCCCGGCTCTGACTGACCGCGTTAC
TCCCACAGGTGAGCGGGCGGGACGGCCCTTCTCCTCCGGGCTGTAATTAGCGCTTG
GTTTAATGACGGCTTGTTTCTTTTCTGTGGCTGCGTGAAAGCCTTGAGGGGCTCCGG
GAGGGCCCTTTGTGCGGGGGGAGCGGCTCGGGGGGTGCGTGCGTGTGTGTGTGCG
TGGGGAGCGCCGCGTGCGGCTCCGCGCTGCCCGGCGGCTGTGAGCGCTGCGGGCG
CGGCGCGGGGCTTTGTGCGCTCCGCAGTGTGCGCGAGGGGAGCGCGGCCGGGGGC
GGTGCCCCGCGGTGCGGGGGGGGCTGCGAGGGGAACAAAGGCTGCGTGCGGGGT
GTGTGCGTGGGGGGGTGAGCAGGGGGTGTGGGCGCGTCGGTCGGGCTGCAACCCC
CCCTGCACCCCCCTCCCCGAGTTGCTGAGCACGGCCCGGCTTCGGGTGCGGGGCTC
CGTACGGGGCGTGGCGCGGGGCTCGCCGTGCCGGGCGGGGGGTGGCGGCAGGTGG
GGGTGCCGGGCGGGGCGGGGCCGCCTCGGGCCGGGGAGGGCTCGGGGGAGGGGC
GCGGCGGCCCCCGGAGCGCCGGCGGCTGTCGAGGCGCGGCGAGCCGCAGCCATTG
CCTTTTATGGTAATCGTGCGAGAGGGCGCAGGGACTTCCTTTGTCCCAAATCTGTGC
 
GGAGCCGAAATCTGGGAGGCGCCGCCGCACCCCCTCTAGCGGGCGCGGGGCGAAG
CGGTGCGGCGCCGGCAGGAAGGAAATGGGCGGGGAGGGCCTTCGTGCGTCGCCGC
GCCGCCGTCCCCTTCTCCCTCTCCAGCCTCGGGGCTGTCCGCGGGGGGACGGCTGC
CTTCGGGGGGGACGGGGCAGGGCGGGGTTCGGCTTCTGGCGTGTGACCGGCGGCT
CTAGAGCCTCTGCTAACCATGTTCATGCCTTCTTCTTTTTCCTACAGCTCCTGGGCAA
CGTGCTGGTTATTGTGCTGTCTCATCATTTTGGCAAAGAATTACTAGATCGCCACCAT
GAGACTGCTGGCCTTCCTGAGCCTGCTGGCCCTGGTGCTGCAGGAGACAGGCACCT
CTGACACAGGCAGACCCTTTGTGGAAATGTACTCTGAGATCCCTGAGATCATCCACA
TGACAGAGGGCAGAGAGCTGGTCATCCCCTGCAGAGTGACAAGCCCCAACATCAC
AGTGACCCTGAAGAAGTTCCCTCTGGACACACTGATCCCTGATGGCAAGAGAATCA
TCTGGGACAGCAGAAAGGGCTTCATCATCAGCAATGCCACCTACAAAGAGATTGGC
CTGCTGACCTGTGAAGCCACAGTGAATGGCCACCTGTACAAGACCAACTACCTGAC
ACACAGACAGACCAACACCATCATTGATGTGGTGCTGAGCCCCAGCCATGGCATTG
AGCTGTCTGTGGGAGAGAAGCTGGTGCTGAACTGCACAGCCAGAACAGAGCTGAA
TGTGGGCATTGACTTCAACTGGGAGTACCCCAGCAGCAAGCACCAGCACAAGAAA
CTGGTCAACAGGGACCTGAAAACCCAGAGTGGCTCTGAGATGAAGAAATTCCTGA
GCACCCTGACCATTGATGGGGTCACCAGATCTGACCAGGGCCTGTACACATGTGCT
GCCAGCTCTGGACTGATGACCAAGAAAAACAGCACCTTTGTCAGAGTGCATGAGA
AGGACAAGACCCACACCTGTCCTCCATGTCCTGCACCTGAGCTGCTTGGAGGCCCC
TCTGTGTTCCTGTTTCCTCCAAAGCCTAAGGACACCCTGATGATCAGCAGAACACCT
GAAGTGACCTGTGTGGTGGTGGATGTGTCCCATGAGGACCCAGAAGTGAAGTTCAA
TTGGTATGTGGATGGGGTTGAAGTGCACAATGCCAAGACCAAGCCTAGAGAGGAAC
AGTACAACTCCACCTACAGAGTGGTGTCAGTGCTGACAGTGCTGCACCAGGACTGG
CTGAATGGCAAAGAGTACAAGTGCAAGGTGTCCAACAAGGCCCTGCCTGCTCCTAT
TGAGAAAACCATCTCCAAGGCCAAGGGCCAGCCTAGGGAACCCCAGGTTTACACA
CTGCCACCTAGCAGGGATGAGCTGACAAAGAACCAGGTGTCCCTGACCTGCCTGGT
CAAGGGCTTCTACCCCTCTGACATTGCTGTGGAATGGGAGAGCAATGGCCAGCCTG
AGAACAACTACAAGACAACCCCTCCTGTGCTGGACTCTGATGGCTCATTCTTCCTGT
ACAGCAAGCTGACTGTGGACAAGAGCAGATGGCAGCAGGGAAATGTGTTCAGCTG
CTCTGTGATGCATGAGGCCCTGCACAACCACTACACCCAGAAAAGCCTGAGCCTGT
CTCCTGGCAAGTGAGCGGCCGCAAGCTTCGGGTGGCATCCCTGTGACCCCTCCCCA
GTGCCTCTCCTGGCCCTGGAAGTTGCCACTCCAGTGCCCACCAGCCTTGTCCTAATA
AAATTAAGTTGCATCATTTTGTCTGACTAGGTGTCCTTCTATAATATTATGGGGTGGA
GGGGGGTGGTATGGAGCAAGGGGCAAGTTGGGAAGACAACCTGTAGGGCCTGCGG
GGTCTATTGGGAACCAAGCTGGAGTGCAGTGGCACAATCTTGGCTCACTGCAATCT
 
CCGCCTCCTGGGTTCAAGCGATTCTCCTGCCTCAGCCTCCCGAGTTGTTGGGATTCC
AGGCATGCATGACCAGGCTCAGCTAATTTTTGTTTTTTTGGTAGAGACGGGGTTTCA
CCATATTGGCCAGGCTGGTCTCCAACTCCTAATCTCAGGTGATCTACCCACCTTGGC
CTCCCAAATTGCTGGGATTACAGGCGTGAACCACTGCTCCCTTCCCTGTCCTTCGG3'
SEQ ID NO: 41 (CMV-mROR1 sp-Aflibercept opt3-hGH pA) 
5'ACGCGTCTAGTTATTAATAGTAATCAATTACGGGGTCATTAGTTCATAGCCCAT
ATATGGAGTTCCGCGTTACATAACTTACGGTAAATGGCCCGCCTGGCTGACCGCCCA
ACGACCCCCGCCCATTGACGTCAATAATGACGTATGTTCCCATAGTAACGCCAATAG
GGACTTTCCATTGACGTCAATGGGTGGAGTATTTACGGTAAACTGCCCACTTGGCAG
TACATCAAGTGTATCATATGCCAAGTACGCCCCCTATTGACGTCAATGACGGTAAATG
GCCCGCCTGGCATTATGCCCAGTACATGACCTTATGGGACTTTCCTACTTGGCAGTA
CATCTACGTATTAGTCATCGCTATTACCATGGTGATGCGGTTTTGGCAGTACATCAAT
GGGCGTGGATAGCGGTTTGACTCACGGGGATTTCCAAGTCTCCACCCCATTGACGT
CAATGGGAGTTTGTTTTGGCACCAAAATCAACGGGACTTTCCAAAATGTCGTAACA
ACTCCGCCCCATTGACGCAAATGGGCGGTAGGCGTGTACGGTGGGAGGTCTATATAA
GCAGAGCTCGTTTAGTGAACCGTCAGATCGCCTGGAGACGCCATCCACGCTGTTTT
GACCTCCATAGAAGACACCGGGACCGATCCAGCCTCCGCGGATTCGAATCCCGGCC
GGGAACGGTGCATTGGAACGCGGATTCCCCGTGCCAAGAGTGACGTAAGTACCGCC
TATAGAGTCTATAGGCCCACAAAAAATGCTTTCTTCTTTTAATATACTTTTTTGTTTAT
CTTATTTCTAATACTTTCCCTAATCTCTTTCTTTCAGGGCAATAATGATACAATGTATC
ATGCCTCTTTGCACCATTCTAAAGAATAACAGTGATAATTTCTGGGTTAAGGCAATAG
CAATATTTCTGCATATAAATATTTCTGCATATAAATTGTAACTGATGTAAGAGGTTTCA
TATTGCTAATAGCAGCTACAATCCAGCTACCATTCTGCTTTTATTTTATGGTTGGGATA
AGGCTGGATTATTCTGAGTCCAAGCTAGGCCCTTTTGCTAATCATGTTCATACCTCTT
ATCTTCCTCCCACAGCTCCTGGGCAACGTGCTGGTCTGTGTGCTGGCCCATCACTTT
GGCAAAGAATTGGGATAATTACTAGATCGCCACCATGCACAGACCCAGAAGGAGGG
GCACCAGACCTCCACCTCTGGCTCTGCTGGCAGCCCTGCTGCTGGCAGCTAGAGGA
GCAGATGCCTCTGACACAGGCAGACCCTTTGTGGAAATGTACTCTGAGATCCCTGA
GATCATCCACATGACAGAGGGCAGAGAGCTGGTCATCCCCTGCAGAGTGACAAGCC
CCAACATCACAGTGACCCTGAAGAAGTTCCCTCTGGACACACTGATCCCTGATGGC
AAGAGAATCATCTGGGACAGCAGAAAGGGCTTCATCATCAGCAATGCCACCTACAA
AGAGATTGGCCTGCTGACCTGTGAAGCCACAGTGAATGGCCACCTGTACAAGACCA
ACTACCTGACACACAGACAGACCAACACCATCATTGATGTGGTGCTGAGCCCCAGC
CATGGCATTGAGCTGTCTGTGGGAGAGAAGCTGGTGCTGAACTGCACAGCCAGAA
 
CAGAGCTGAATGTGGGCATTGACTTCAACTGGGAGTACCCCAGCAGCAAGCACCA
GCACAAGAAACTGGTCAACAGGGACCTGAAAACCCAGAGTGGCTCTGAGATGAAG
AAATTCCTGAGCACCCTGACCATTGATGGGGTCACCAGATCTGACCAGGGCCTGTA
CACATGTGCTGCCAGCTCTGGACTGATGACCAAGAAAAACAGCACCTTTGTCAGAG
TGCATGAGAAGGACAAGACCCACACCTGTCCTCCATGTCCTGCACCTGAGCTGCTT
GGAGGCCCCTCTGTGTTCCTGTTTCCTCCAAAGCCTAAGGACACCCTGATGATCAGC
AGAACACCTGAAGTGACCTGTGTGGTGGTGGATGTGTCCCATGAGGACCCAGAAG
TGAAGTTCAATTGGTATGTGGATGGGGTTGAAGTGCACAATGCCAAGACCAAGCCT
AGAGAGGAACAGTACAACTCCACCTACAGAGTGGTGTCAGTGCTGACAGTGCTGC
ACCAGGACTGGCTGAATGGCAAAGAGTACAAGTGCAAGGTGTCCAACAAGGCCCT
GCCTGCTCCTATTGAGAAAACCATCTCCAAGGCCAAGGGCCAGCCTAGGGAACCCC
AGGTTTACACACTGCCACCTAGCAGGGATGAGCTGACAAAGAACCAGGTGTCCCTG
ACCTGCCTGGTCAAGGGCTTCTACCCCTCTGACATTGCTGTGGAATGGGAGAGCAA
TGGCCAGCCTGAGAACAACTACAAGACAACCCCTCCTGTGCTGGACTCTGATGGCT
CATTCTTCCTGTACAGCAAGCTGACTGTGGACAAGAGCAGATGGCAGCAGGGAAAT
GTGTTCAGCTGCTCTGTGATGCATGAGGCCCTGCACAACCACTACACCCAGAAAAG
CCTGAGCCTGTCTCCTGGCAAGTGAGCGGCCGCAAGCTTCGGGTGGCATCCCTGTG
ACCCCTCCCCAGTGCCTCTCCTGGCCCTGGAAGTTGCCACTCCAGTGCCCACCAGC
CTTGTCCTAATAAAATTAAGTTGCATCATTTTGTCTGACTAGGTGTCCTTCTATAATAT
TATGGGGTGGAGGGGGGTGGTATGGAGCAAGGGGCAAGTTGGGAAGACAACCTGT
AGGGCCTGCGGGGTCTATTGGGAACCAAGCTGGAGTGCAGTGGCACAATCTTGGCT
CACTGCAATCTCCGCCTCCTGGGTTCAAGCGATTCTCCTGCCTCAGCCTCCCGAGTT
GTTGGGATTCCAGGCATGCATGACCAGGCTCAGCTAATTTTTGTTTTTTTGGTAGAG
ACGGGGTTTCACCATATTGGCCAGGCTGGTCTCCAACTCCTAATCTCAGGTGATCTA
CCCACCTTGGCCTCCCAAATTGCTGGGATTACAGGCGTGAACCACTGCTCCCTTCCC
TGTCCTTCGG3’
SEQ ID NO: 42 (CBh-mROR1 sp-Aflibercept opt3-hGH pA) 
5’ACGCGTCTAGTTATTAATAGTAATCAATTACGGGGTCATTAGTTCATAGCCCAT
ATATGGAGTTCCGCGTTACATAACTTACGGTAAATGGCCCGCCTGGCTGACCGCCCA
ACGACCCCCGCCCATTGACGTCAATAATGACGTATGTTCCCATAGTAACGCCAATAG
GGACTTTCCATTGACGTCAATGGGTGGAGTATTTACGGTAAACTGCCCACTTGGCAG
TACATCAAGTGTATCATATGCCAAGTACGCCCCCTATTGACGTCAATGACGGTAAATG
GCCCGCCTGGCATTATGCCCAGTACATGACCTTACGGGACTTTCCTACTTGGCAGTA
CATCTACGTATTAGTCATCGCTATTACCATGGTCGAGGTGAGCCCCACGTTCTGCTTC
 
ACTCTCCCCATCTCCCCCCCCTCCCCACCCCCAATTTTGTATTTATTTATTTTTTAATTA
TTTTGTGCAGCGATGGGGGCGGGGGGGGGGGGGGCGCGCGCCAGGCGGGGCGGG
GCGGGGCGAGGGGCGGGGCGGGGCGAGGCGGAGAGGTGCGGCGGCAGCCAATCA
GAGCGGCGCGCTCCGAAAGTTTCCTTTTATGGCGAGGCGGCGGCGGCGGCCCT
ATAAAAAGCGAAGCGCGCGGCGGGCGGGGAGTCGCTGCGTTGCCTTCGCCCCGTG
CCCCGCTCCGCGCCGCCTCGCGCCGCCCGCCCCGGCTCTGACTGACCGCGTTACTC
CCACAGGTGAGCGGGCGGGACGGCCCTTCTCCTCCGGGCTGTAATTAGCAAGAGGT
AAGGGTTTAAGGGATGGTTGGTTGGTGGGGTATTAATGTTTAATTACCTGTTTTACAG
GCCTGAAATCACTTGGTTTTAGGTTGGAATTACTAGATCGCCACCATGCACAGACCC
AGAAGGAGGGGCACCAGACCTCCACCTCTGGCTCTGCTGGCAGCCCTGCTGCTGG
CAGCTAGAGGAGCAGATGCCTCTGACACAGGCAGACCCTTTGTGGAAATGTACTCT
GAGATCCCTGAGATCATCCACATGACAGAGGGCAGAGAGCTGGTCATCCCCTGCAG
AGTGACAAGCCCCAACATCACAGTGACCCTGAAGAAGTTCCCTCTGGACACACTGA
TCCCTGATGGCAAGAGAATCATCTGGGACAGCAGAAAGGGCTTCATCATCAGCAAT
GCCACCTACAAAGAGATTGGCCTGCTGACCTGTGAAGCCACAGTGAATGGCCACCT
GTACAAGACCAACTACCTGACACACAGACAGACCAACACCATCATTGATGTGGTGC
TGAGCCCCAGCCATGGCATTGAGCTGTCTGTGGGAGAGAAGCTGGTGCTGAACTGC
ACAGCCAGAACAGAGCTGAATGTGGGCATTGACTTCAACTGGGAGTACCCCAGCA
GCAAGCACCAGCACAAGAAACTGGTCAACAGGGACCTGAAAACCCAGAGTGGCT
CTGAGATGAAGAAATTCCTGAGCACCCTGACCATTGATGGGGTCACCAGATCTGAC
CAGGGCCTGTACACATGTGCTGCCAGCTCTGGACTGATGACCAAGAAAAACAGCAC
CTTTGTCAGAGTGCATGAGAAGGACAAGACCCACACCTGTCCTCCATGTCCTGCAC
CTGAGCTGCTTGGAGGCCCCTCTGTGTTCCTGTTTCCTCCAAAGCCTAAGGACACC
CTGATGATCAGCAGAACACCTGAAGTGACCTGTGTGGTGGTGGATGTGTCCCATGA
GGACCCAGAAGTGAAGTTCAATTGGTATGTGGATGGGGTTGAAGTGCACAATGCCA
AGACCAAGCCTAGAGAGGAACAGTACAACTCCACCTACAGAGTGGTGTCAGTGCT
GACAGTGCTGCACCAGGACTGGCTGAATGGCAAAGAGTACAAGTGCAAGGTGTCC
AACAAGGCCCTGCCTGCTCCTATTGAGAAAACCATCTCCAAGGCCAAGGGCCAGCC
TAGGGAACCCCAGGTTTACACACTGCCACCTAGCAGGGATGAGCTGACAAAGAAC
CAGGTGTCCCTGACCTGCCTGGTCAAGGGCTTCTACCCCTCTGACATTGCTGTGGAA
TGGGAGAGCAATGGCCAGCCTGAGAACAACTACAAGACAACCCCTCCTGTGCTGG
ACTCTGATGGCTCATTCTTCCTGTACAGCAAGCTGACTGTGGACAAGAGCAGATGG
CAGCAGGGAAATGTGTTCAGCTGCTCTGTGATGCATGAGGCCCTGCACAACCACTA
CACCCAGAAAAGCCTGAGCCTGTCTCCTGGCAAGTGAGCGGCCGCAAGCTTCGGG
TGGCATCCCTGTGACCCCTCCCCAGTGCCTCTCCTGGCCCTGGAAGTTGCCACTCCA
 
GTGCCCACCAGCCTTGTCCTAATAAAATTAAGTTGCATCATTTTGTCTGACTAGGTGT
CCTTCTATAATATTATGGGGTGGAGGGGGGTGGTATGGAGCAAGGGGCAAGTTGGG
AAGACAACCTGTAGGGCCTGCGGGGTCTATTGGGAACCAAGCTGGAGTGCAGTGG
CACAATCTTGGCTCACTGCAATCTCCGCCTCCTGGGTTCAAGCGATTCTCCTGCCTC
AGCCTCCCGAGTTGTTGGGATTCCAGGCATGCATGACCAGGCTCAGCTAATTTTTGT
TTTTTTGGTAGAGACGGGGTTTCACCATATTGGCCAGGCTGGTCTCCAACTCCTAAT
CTCAGGTGATCTACCCACCTTGGCCTCCCAAATTGCTGGGATTACAGGCGTGAACCA
CTGCTCCCTTCCCTGTCCTTCGG3’
SEQ ID NO: 43 (CB7-mROR1 sp-Aflibercept opt3-hGH pA) 
5'ACGCGTCTAGTTATTAATAGTAATCAATTACGGGGTCATTAGTTCATAGCCCAT
ATATGGAGTTCCGCGTTACATAACTTACGGTAAATGGCCCGCCTGGCTGACCGCCCA
ACGACCCCCGCCCATTGACGTCAATAATGACGTATGTTCCCATAGTAACGCCAATAG
GGACTTTCCATTGACGTCAATGGGTGGAGTATTTACGGTAAACTGCCCACTTGGCAG
TACATCAAGTGTATCATATGCCAAGTACGCCCCCTATTGACGTCAATGACGGTAAATG
GCCCGCCTGGCATTATGCCCAGTACATGACCTTACGGGACTTTCCTACTTGGCAGTA
CATCTACGTATTAGTCATCGCTATTACCATGGTCGAGGTGAGCCCCACGTTCTGCTTC
ACTCTCCCCATCTCCCCCCCCTCCCCACCCCCAATTTTGTATTTATTTATTTTTTAATTA
TTTTGTGCAGCGATGGGGGCGGGGGGGGGGGGGGCGCGCGCCAGGCGGGGCGGG
GCGGGGCGAGGGGCGGGGCGGGGCGAGGCGGAGAGGTGCGGCGGCAGCCAATCA
GAGCGGCGCGCTCCGAAAGTTTCCTTTTATGGCGAGGCGGCGGCGGCGGCGGCCCT
ATAAAAAGCGAAGCGCGCGGCGGGCGGGAGTCGCTGCGCGCTGCCTTCGCCCCGT
GCCCCGCTCCGCCGCCGCCTCGCGCCGCCCGCCCCGGCTCTGACTGACCGCGTTAC
TCCCACAGGTGAGCGGGCGGGACGGCCCTTCTCCTCCGGGCTGTAATTAGCGCTTG
GTTTAATGACGGCTTGTTTCTTTTCTGTGGCTGCGTGAAAGCCTTGAGGGGCTCCGG
GAGGGCCCTTTGTGCGGGGGGAGCGGCTCGGGGGGTGCGTGCGTGTGTGTGTGCG
TGGGGAGCGCCGCGTGCGGCTCCGCGCTGCCCGGCGGCTGTGAGCGCTGCGGGCG
CGGCGCGGGGCTTTGTGCGCTCCGCAGTGTGCGCGAGGGGAGCGCGGCCGGGGGC
GGTGCCCCGCGGTGCGGGGGGGGCTGCGAGGGGAACAAAGGCTGCGTGCGGGGT
GTGTGCGTGGGGGGGTGAGCAGGGGGTGTGGGCGCGTCGGTCGGGCTGCAACCCC
CCCTGCACCCCCCTCCCCGAGTTGCTGAGCACGGCCCGGCTTCGGGTGCGGGGCTC
CGTACGGGGCGTGGCGCGGGGCTCGCCGTGCCGGGCGGGGGGTGGCGGCAGGTGG
GGGTGCCGGGCGGGGCGGGGCCGCCTCGGGCCGGGGAGGGCTCGGGGGAGGGGC
GCGGCGGCCCCCGGAGCGCCGGCGGCTGTCGAGGCGCGGCGAGCCGCAGCCATTG
CCTTTTATGGTAATCGTGCGAGAGGGCGCAGGGACTTCCTTTGTCCCAAATCTGTGC
 
GGAGCCGAAATCTGGGAGGCGCCGCCGCACCCCCTCTAGCGGGCGCGGGGCGAAG
CGGTGCGGCGCCGGCAGGAAGGAAATGGGCGGGGAGGGCCTTCGTGCGTCGCCGC
GCCGCCGTCCCCTTCTCCCTCTCCAGCCTCGGGGCTGTCCGCGGGGGGACGGCTGC
CTTCGGGGGGGACGGGGCAGGGCGGGGTTCGGCTTCTGGCGTGTGACCGGCGGCT
CTAGAGCCTCTGCTAACCATGTTCATGCCTTCTTCTTTTTCCTACAGCTCCTGGGCAA
CGTGCTGGTTATTGTGCTGTCTCATCATTTTGGCAAAGAATTACTAGATCGCCACCAT
GCACAGACCCAGAAGGAGGGGCACCAGACCTCCACCTCTGGCTCTGCTGGCAGCC
CTGCTGCTGGCAGCTAGAGGAGCAGATGCCTCTGACACAGGCAGACCCTTTGTGGA
AATGTACTCTGAGATCCCTGAGATCATCCACATGACAGAGGGCAGAGAGCTGGTCA
TCCCCTGCAGAGTGACAAGCCCCAACATCACAGTGACCCTGAAGAAGTTCCCTCTG
GACACACTGATCCCTGATGGCAAGAGAATCATCTGGGACAGCAGAAAGGGCTTCAT
CATCAGCAATGCCACCTACAAAGAGATTGGCCTGCTGACCTGTGAAGCCACAGTGA
ATGGCCACCTGTACAAGACCAACTACCTGACACACAGACAGACCAACACCATCATT
GATGTGGTGCTGAGCCCCAGCCATGGCATTGAGCTGTCTGTGGGAGAGAAGCTGGT
GCTGAACTGCACAGCCAGAACAGAGCTGAATGTGGGCATTGACTTCAACTGGGAGT
ACCCCAGCAGCAAGCACCAGCACAAGAAACTGGTCAACAGGGACCTGAAAACCC
AGAGTGGCTCTGAGATGAAGAAATTCCTGAGCACCCTGACCATTGATGGGGTCACC
AGATCTGACCAGGGCCTGTACACATGTGCTGCCAGCTCTGGACTGATGACCAAGAA
AAACAGCACCTTTGTCAGAGTGCATGAGAAGGACAAGACCCACACCTGTCCTCCAT
GTCCTGCACCTGAGCTGCTTGGAGGCCCCTCTGTGTTCCTGTTTCCTCCAAAGCCTA
AGGACACCCTGATGATCAGCAGAACACCTGAAGTGACCTGTGTGGTGGTGGATGTG
TCCCATGAGGACCCAGAAGTGAAGTTCAATTGGTATGTGGATGGGGTTGAAGTGCA
CAATGCCAAGACCAAGCCTAGAGAGGAACAGTACAACTCCACCTACAGAGTGGTG
TCAGTGCTGACAGTGCTGCACCAGGACTGGCTGAATGGCAAAGAGTACAAGTGCA
AGGTGTCCAACAAGGCCCTGCCTGCTCCTATTGAGAAAACCATCTCCAAGGCCAAG
GGCCAGCCTAGGGAACCCCAGGTTTACACACTGCCACCTAGCAGGGATGAGCTGAC
AAAGAACCAGGTGTCCCTGACCTGCCTGGTCAAGGGCTTCTACCCCTCTGACATTG
CTGTGGAATGGGAGAGCAATGGCCAGCCTGAGAACAACTACAAGACAACCCCTCC
TGTGCTGGACTCTGATGGCTCATTCTTCCTGTACAGCAAGCTGACTGTGGACAAGA
GCAGATGGCAGCAGGGAAATGTGTTCAGCTGCTCTGTGATGCATGAGGCCCTGCAC
AACCACTACACCCAGAAAAGCCTGAGCCTGTCTCCTGGCAAGTGAGCGGCCGCAA
GCTTCGGGTGGCATCCCTGTGACCCCTCCCCAGTGCCTCTCCTGGCCCTGGAAGTT
GCCACTCCAGTGCCCACCAGCCTTGTCCTAATAAAATTAAGTTGCATCATTTTGTCTG
ACTAGGTGTCCTTCTATAATATTATGGGGTGGAGGGGGGTGGTATGGAGCAAGGGGC
AAGTTGGGAAGACAACCTGTAGGGCCTGCGGGGTCTATTGGGAACCAAGCTGGAG
 
TGCAGTGGCACAATCTTGGCTCACTGCAATCTCCGCCTCCTGGGTTCAAGCGATTCT
CCTGCCTCAGCCTCCCGAGTTGTTGGGATTCCAGGCATGCATGACCAGGCTCAGCTA
ATTTTTGTTTTTTTGGTAGAGACGGGGTTTCACCATATTGGCCAGGCTGGTCTCCAA
CTCCTAATCTCAGGTGATCTACCCACCTTGGCCTCCCAAATTGCTGGGATTACAGGC
GTGAACCACTGCTCCCTTCCCTGTCCTTCGG3'
pssAAV-CMV-Fluc-hGH pA expression vector (SEQ ID NO: 49; where Fluc was marked in bold, italics, and underline; the upstream ITR was marked with a wavy line; and the downstream ITR was marked with a double underline) 
5'TTGGCCACTCCCTCTCTGCGCGCTCGCTCGCTCACTGAGGCCGGGCGACCAA
AGGTCGCCCGACGCCCGGGCTTTGCCCGGGCGGCCTCAGTGAGCGAGCGAGCGCG
CAGAGAGGGAGTGGCCAACTCCATCACTAGGGGTTCCTCTCGAGACGCGTCTAGTT
ATTAATAGTAATCAATTACGGGGTCATTAGTTCATAGCCCATATATGGAGTTCCGCGTT
ACATAACTTACGGTAAATGGCCCGCCTGGCTGACCGCCCAACGACCCCCGCCCATT
GACGTCAATAATGACGTATGTTCCCATAGTAACGCCAATAGGGACTTTCCATTGACG
TCAATGGGTGGAGTATTTACGGTAAACTGCCCACTTGGCAGTACATCAAGTGTATCA
TATGCCAAGTACGCCCCCTATTGACGTCAATGACGGTAAATGGCCCGCCTGGCATTA
TGCCCAGTACATGACCTTATGGGACTTTCCTACTTGGCAGTACATCTACGTATTAGTC
ATCGCTATTACCATGGTGATGCGGTTTTGGCAGTACATCAATGGGCGTGGATAGCGG
TTTGACTCACGGGGATTTCCAAGTCTCCACCCCATTGACGTCAATGGGAGTTTGTTT
TGGCACCAAAATCAACGGGACTTTCCAAAATGTCGTAACAACTCCGCCCCATTGAC
GCAAATGGGCGGTAGGCGTGTACGGTGGGAGGTCTATATAAGCAGAGCTCGTTTAG
TGAACCGTCAGATCGCCTGGAGACGCCATCCACGCTGTTTTGACCTCCATAGAAGA
CACCGGGACCGATCCAGCCTCCGCGGATTCGAATCCCGGCCGGGAACGGTGCATTG
GAACGCGGATTCCCCGTGCCAAGAGTGACGTAAGTACCGCCTATAGAGTCTATAGG
CCCACAAAAAATGCTTTCTTCTTTTAATATACTTTTTTGTTTATCTTATTTCTAATACTT
TCCCTAATCTCTTTCTTTCAGGGCAATAATGATACAATGTATCATGCCTCTTTGCACCA
TTCTAAAGAATAACAGTGATAATTTCTGGGTTAAGGCAATAGCAATATTTCTGCATAT
AAATATTTCTGCATATAAATTGTAACTGATGTAAGAGGTTTCATATTGCTAATAGCAGC
TACAATCCAGCTACCATTCTGCTTTTATTTTATGGTTGGGATAAGGCTGGATTATTCTG
AGTCCAAGCTAGGCCCTTTTGCTAATCATGTTCATACCTCTTATCTTCCTCCCACAGC
TCCTGGGCAACGTGCTGGTCTGTGTGCTGGCCCATCACTTTGGCAAAGAATTGGGAT
AATTACTAGATCATGGAAGATGCCAAAAACATTAAGAAGGGCCCAGCGCCATTCTACC
CACTCGAAGACGGGACCGCCGGCGAGCAGCTGCACAAAGCCATGAAGCGCTACGCC
CTGGTGCCCGGCACCATCGCCTTTACCGACGCACATATCGAGGTGGACATTACCTAC
 
GCCGAGTACTTCGAGATGAGCGTTCGGCTGGCAGAAGCTATGAAGCGCTATGGGCTG
AATACAAACCATCGGATCGTGGTGTGCAGCGAGAATAGCTTGCAGTTCTTCATGCCC
GTGTTGGGTGCCCTGTTCATCGGTGTGGCTGTGGCCCCAGCTAACGACATCTACAAC
GAGCGCGAGCTGCTGAACAGCATGGGCATCAGCCAGCCCACCGTCGTATTCGTGAG
CAAGAAAGGGCTGCAAAAGATCCTCAACGTGCAAAAGAAGCTACCGATCATACAAAA
GATCATCATCATGGATAGCAAGACCGACTACCAGGGCTTCCAAAGCATGTACACCTTC
GTGACTTCCCATTTGCCACCCGGCTTCAACGAGTACGACTTCGTGCCCGAGAGCTTC
GACCGGGACAAAACCATCGCCCTGATCATGAACAGTAGTGGCAGTACCGGATTGCCC
AAGGGCGTAGCCCTACCGCACCGCACCGCTTGTGTCCGATTCAGTCATGCCCGCGAC
CCCATCTTCGGCAACCAGATCATCCCCGACACCGCTATCCTCAGCGTGGTGCCATTTC
ACCACGGCTTCGGCATGTTCACCACGCTGGGCTACTTGATCTGCGGCTTTCGGGTCG
TGCTCATGTACCGCTTCGAGGAGGAGCTATTCTTGCGCAGCTTGCAAGACTATAAGAT
TCAATCTGCCCTGCTGGTGCCCACACTATTTAGCTTCTTCGCTAAGAGCACTCTCATC
GACAAGTACGACCTAAGCAACTTGCACGAGATCGCCAGCGGCGGGGCGCCGCTCAG
CAAGGAGGTAGGTGAGGCCGTGGCCAAACGCTTCCACCTACCAGGCATCCGCCAGG
GCTACGGCCTGACAGAAACAACCAGCGCCATTCTGATCACCCCCGAAGGGGACGAC
AAGCCTGGCGCAGTAGGCAAGGTGGTGCCCTTCTTCGAGGCTAAGGTGGTGGACTT
GGACACCGGTAAGACACTGGGTGTGAACCAGCGCGGCGAGCTGTGCGTCCGTGGCC
CCATGATCATGAGCGGCTACGTTAACAACCCCGAGGCTACAAACGCTCTCATCGACA
AGGACGGCTGGCTGCACAGCGGCGACATCGCCTACTGGGACGAGGACGAGCACTTC
TTCATCGTGGACCGGCTGAAGAGCCTGATCAAATACAAGGGCTACCAGGTAGCCCCA
GCCGAACTGGAGAGCATCCTGCTGCAACACCCCAACATCTTCGACGCCGGGGTCGC
CGGCCTGCCCGACGACGATGCCGGCGAGCTGCCCGCCGCAGTCGTCGTGCTGGAAC
ACGGTAAAACCATGACCGAGAAGGAGATCGTGGACTATGTGGCCAGCCAGGTTACAA
CCGCCAAGAAGCTGCGCGGTGGTGTTGTGTTCGTGGACGAGGTGCCTAAAGGACTG
ACCGGCAAGTTGGACGCCCGCAAGATCCGCGAGATTCTCATTAAGGCCAAGAAGGG
CGGCAAGATCGCCGTGTAAGCGGCCGCAAGCTTCGGGTGGCATCCCTGTGACCCCT
CCCCAGTGCCTCTCCTGGCCCTGGAAGTTGCCACTCCAGTGCCCACCAGCCTTGTC
CTAATAAAATTAAGTTGCATCATTTTGTCTGACTAGGTGTCCTTCTATAATATTATGGG
GTGGAGGGGGGTGGTATGGAGCAAGGGGCAAGTTGGGAAGACAACCTGTAGGGCC
TGCGGGGTCTATTGGGAACCAAGCTGGAGTGCAGTGGCACAATCTTGGCTCACTGC
AATCTCCGCCTCCTGGGTTCAAGCGATTCTCCTGCCTCAGCCTCCCGAGTTGTTGGG
ATTCCAGGCATGCATGACCAGGCTCAGCTAATTTTTGTTTTTTTGGTAGAGACGGGG
TTTCACCATATTGGCCAGGCTGGTCTCCAACTCCTAATCTCAGGTGATCTACCCACCT
TGGCCTCCCAAATTGCTGGGATTACAGGCGTGAACCACTGCTCCCTTCCCTGTCCTT
 
CGGTCTAGAAGGAACCCCTAGTGATGGAGTTGGCCACTCCCTCTCTGCGCGCTCGC
TCGCTCACTGAGGCCGGGCGACCAAAGGTCGCCCGACGCCCGGGCTTTGCCCGGG
CGGCCTCAGTGAGCGAGCGAGCGCGCAGAGAGGGAGTGG3'
pssAAV-CBh-Fluc-hGH pA expression vector (SEQ ID NO: 50; where Fluc was marked in bold, italics, and underline; the upstream ITR was marked with a wavy line; and the downstream ITR was marked with a double underline) 
5'TTGGCCACTCCCTCTCTGCGCGCTCGCTCGCTCACTGAGGCCGGGCGACCAA
AGGTCGCCCGACGCCCGGGCTTTGCCCGGGCGGCCTCAGTGAGCGAGCGAGCGCG
CAGAGAGGGAGTGGCCAACTCCATCACTAGGGGTTCCTCTCGAGACGCGTCTAGTT
ATTAATAGTAATCAATTACGGGGTCATTAGTTCATAGCCCATATATGGAGTTCCGCGTT
ACATAACTTACGGTAAATGGCCCGCCTGGCTGACCGCCCAACGACCCCCGCCCATT
GACGTCAATAATGACGTATGTTCCCATAGTAACGCCAATAGGGACTTTCCATTGACG
TCAATGGGTGGAGTATTTACGGTAAACTGCCCACTTGGCAGTACATCAAGTGTATCA
TATGCCAAGTACGCCCCCTATTGACGTCAATGACGGTAAATGGCCCGCCTGGCATTA
TGCCCAGTACATGACCTTACGGGACTTTCCTACTTGGCAGTACATCTACGTATTAGTC
ATCGCTATTACCATGGTCGAGGTGAGCCCCACGTTCTGCTTCACTCTCCCCATCTCCC
CCCCCTCCCCACCCCCAATTTTGTATTTATTTATTTTTTAATTATTTTGTGCAGCGATG
GGGGCGGGGGGGGGGGGGGCGCGCGCCAGGCGGGGCGGGGCGGGGCGAGGGGC
GGGGCGGGGCGAGGCGGAGAGGTGCGGCGGCAGCCAATCAGAGCGGCGCGCTCC
GAAAGTTTCCTTTTATGGCGAGGCGGCGGCGGCGGCGGCCCTATAAAAAGCGAAGC
GCGCGGCGGGCGGGGAGTCGCTGCGTTGCCTTCGCCCCGTGCCCCGCTCCGCGCCG
CCTCGCGCCGCCCGCCCCGGCTCTGACTGACCGCGTTACTCCCACAGGTGAGCGGG
CGGGACGGCCCTTCTCCTCCGGGCTGTAATTAGCAAGAGGTAAGGGTTTAAGGGAT
GGTTGGTTGGTGGGGTATTAATGTTTAATTACCTGTTTTACAGGCCTGAAATCACTTG
GTTTTAGGTTGGAATTACTAGATCATGGAAGATGCCAAAAACATTAAGAAGGGCCCA
GCGCCATTCTACCCACTCGAAGACGGGACCGCCGGCGAGCAGCTGCACAAAGCCAT
GAAGCGCTACGCCCTGGTGCCCGGCACCATCGCCTTTACCGACGCACATATCGAGGT
GGACATTACCTACGCCGAGTACTTCGAGATGAGCGTTCGGCTGGCAGAAGCTATGAA
GCGCTATGGGCTGAATACAAACCATCGGATCGTGGTGTGCAGCGAGAATAGCTTGCA
GTTCTTCATGCCCGTGTTGGGTGCCCTGTTCATCGGTGTGGCTGTGGCCCCAGCTAA
CGACATCTACAACGAGCGCGAGCTGCTGAACAGCATGGGCATCAGCCAGCCCACCGT
CGTATTCGTGAGCAAGAAAGGGCTGCAAAAGATCCTCAACGTGCAAAAGAAGCTACC
GATCATACAAAAGATCATCATCATGGATAGCAAGACCGACTACCAGGGCTTCCAAAGC
 
ATGTACACCTTCGTGACTTCCCATTTGCCACCCGGCTTCAACGAGTACGACTTCGTGC
CCGAGAGCTTCGACCGGGACAAAACCATCGCCCTGATCATGAACAGTAGTGGCAGTA
CCGGATTGCCCAAGGGCGTAGCCCTACCGCACCGCACCGCTTGTGTCCGATTCAGTC
ATGCCCGCGACCCCATCTTCGGCAACCAGATCATCCCCGACACCGCTATCCTCAGCG
TGGTGCCATTTCACCACGGCTTCGGCATGTTCACCACGCTGGGCTACTTGATCTGCG
GCTTTCGGGTCGTGCTCATGTACCGCTTCGAGGAGGAGCTATTCTTGCGCAGCTTGC
AAGACTATAAGATTCAATCTGCCCTGCTGGTGCCCACACTATTTAGCTTCTTCGCTAA
GAGCACTCTCATCGACAAGTACGACCTAAGCAACTTGCACGAGATCGCCAGCGGCGG
GGCGCCGCTCAGCAAGGAGGTAGGTGAGGCCGTGGCCAAACGCTTCCACCTACCAG
GCATCCGCCAGGGCTACGGCCTGACAGAAACAACCAGCGCCATTCTGATCACCCCCG
AAGGGGACGACAAGCCTGGCGCAGTAGGCAAGGTGGTGCCCTTCTTCGAGGCTAAG
GTGGTGGACTTGGACACCGGTAAGACACTGGGTGTGAACCAGCGCGGCGAGCTGTG
CGTCCGTGGCCCCATGATCATGAGCGGCTACGTTAACAACCCCGAGGCTACAAACGC
TCTCATCGACAAGGACGGCTGGCTGCACAGCGGCGACATCGCCTACTGGGACGAGG
ACGAGCACTTCTTCATCGTGGACCGGCTGAAGAGCCTGATCAAATACAAGGGCTACC
AGGTAGCCCCAGCCGAACTGGAGAGCATCCTGCTGCAACACCCCAACATCTTCGACG
CCGGGGTCGCCGGCCTGCCCGACGACGATGCCGGCGAGCTGCCCGCCGCAGTCGTC
GTGCTGGAACACGGTAAAACCATGACCGAGAAGGAGATCGTGGACTATGTGGCCAG
CCAGGTTACAACCGCCAAGAAGCTGCGCGGTGGTGTTGTGTTCGTGGACGAGGTGC
CTAAAGGACTGACCGGCAAGTTGGACGCCCGCAAGATCCGCGAGATTCTCATTAAGG
CCAAGAAGGGCGGCAAGATCGCCGTGTAAGCGGCCGCAAGCTTCGGGTGGCATCCC
TGTGACCCCTCCCCAGTGCCTCTCCTGGCCCTGGAAGTTGCCACTCCAGTGCCCAC
CAGCCTTGTCCTAATAAAATTAAGTTGCATCATTTTGTCTGACTAGGTGTCCTTCTAT
AATATTATGGGGTGGAGGGGGGTGGTATGGAGCAAGGGGCAAGTTGGGAAGACAA
CCTGTAGGGCCTGCGGGGTCTATTGGGAACCAAGCTGGAGTGCAGTGGCACAATCT
TGGCTCACTGCAATCTCCGCCTCCTGGGTTCAAGCGATTCTCCTGCCTCAGCCTCCC
GAGTTGTTGGGATTCCAGGCATGCATGACCAGGCTCAGCTAATTTTTGTTTTTTTGGT
AGAGACGGGGTTTCACCATATTGGCCAGGCTGGTCTCCAACTCCTAATCTCAGGTGA
TCTACCCACCTTGGCCTCCCAAATTGCTGGGATTACAGGCGTGAACCACTGCTCCCT
TCCCTGTCCTTCGGTCTAGAAGGAACCCCTAGTGATGGAGTTGGCCACTCCCTCTCT
GCGCGCTCGCTCGCTCACTGAGGCCGGGCGACCAAAGGTCGCCCGACGCCCGGGC
TTTGCCCGGGCGGCCTCAGTGAGCGAGCGAGCGCGCAGAGAGGGAGTGG3'
pssAAV-CB7-Fluc-hGH pA expression vector (SEQ ID NO: 51; where Fluc was marked in bold, italics, and underline; the upstream ITR was marked with a wavy line; and the downstream ITR was marked with a double underline)

5'TTGGCCACTCCCTCTCTGCGCGCTCGCTCGCTCACTGAGGCCGGGCGACCAA
AGGTCGCCCGACGCCCGGGCTTTGCCCGGGCGGCCTCAGTGAGCGAGCGAGCGCG
CAGAGAGGGAGTGGCCAACTCCATCACTAGGGGTTCCTCTCGAGACGCGTCTAGTT
ATTAATAGTAATCAATTACGGGGTCATTAGTTCATAGCCCATATATGGAGTTCCGCGTT
ACATAACTTACGGTAAATGGCCCGCCTGGCTGACCGCCCAACGACCCCCGCCCATT
GACGTCAATAATGACGTATGTTCCCATAGTAACGCCAATAGGGACTTTCCATTGACG
TCAATGGGTGGAGTATTTACGGTAAACTGCCCACTTGGCAGTACATCAAGTGTATCA
TATGCCAAGTACGCCCCCTATTGACGTCAATGACGGTAAATGGCCCGCCTGGCATTA
TGCCCAGTACATGACCTTACGGGACTTTCCTACTTGGCAGTACATCTACGTATTAGTC
ATCGCTATTACCATGGTCGAGGTGAGCCCCACGTTCTGCTTCACTCTCCCCATCTCCC
CCCCCTCCCCACCCCCAATTTTGTATTTATTTATTTTTTAATTATTTTGTGCAGCGATG
GGGGCGGGGGGGGGGGGGGCGCGCGCCAGGCGGGGCGGGGCGGGGCGAGGGGC
GGGGCGGGGCGAGGCGGAGAGGTGCGGCGGCAGCCAATCAGAGCGGCGCGCTCC
GAAAGTTTCCTTTTATGGCGAGGCGGCGGCGGCGGCGGCCCTATAAAAAGCGAAGC
GCGCGGCGGGCGGGAGTCGCTGCGCGCTGCCTTCGCCCCGTGCCCCGCTCCGCCGC
CGCCTCGCGCCGCCCGCCCCGGCTCTGACTGACCGCGTTACTCCCACAGGTGAGCG
GGCGGGACGGCCCTTCTCCTCCGGGCTGTAATTAGCGCTTGGTTTAATGACGGCTTG
TTTCTTTTCTGTGGCTGCGTGAAAGCCTTGAGGGGCTCCGGGAGGGCCCTTTGTGC
GGGGGGAGCGGCTCGGGGGGTGCGTGCGTGTGTGTGTGCGTGGGGAGCGCCGCGT
GCGGCTCCGCGCTGCCCGGCGGCTGTGAGCGCTGCGGGCGCGGCGCGGGGCTTTG
TGCGCTCCGCAGTGTGCGCGAGGGGAGCGCGGCCGGGGGCGGTGCCCCGCGGTGC
GGGGGGGGCTGCGAGGGGAACAAAGGCTGCGTGCGGGGTGTGTGCGTGGGGGGG
TGAGCAGGGGGTGTGGGCGCGTCGGTCGGGCTGCAACCCCCCCTGCACCCCCCTC
CCCGAGTTGCTGAGCACGGCCCGGCTTCGGGTGCGGGGCTCCGTACGGGGCGTGG
CGCGGGGCTCGCCGTGCCGGGCGGGGGGTGGCGGCAGGTGGGGGTGCCGGGCGG
GGCGGGGCCGCCTCGGGCCGGGGAGGGCTCGGGGGAGGGGCGCGGCGGCCCCCG
GAGCGCCGGCGGCTGTCGAGGCGCGGCGAGCCGCAGCCATTGCCTTTTATGGTAAT
CGTGCGAGAGGGCGCAGGGACTTCCTTTGTCCCAAATCTGTGCGGAGCCGAAATCT
GGGAGGCGCCGCCGCACCCCCTCTAGCGGGCGCGGGGCGAAGCGGTGCGGCGCCG
GCAGGAAGGAAATGGGCGGGGAGGGCCTTCGTGCGTCGCCGCGCCGCCGTCCCCT
TCTCCCTCTCCAGCCTCGGGGCTGTCCGCGGGGGGACGGCTGCCTTCGGGGGGGAC
GGGGCAGGGCGGGGTTCGGCTTCTGGCGTGTGACCGGCGGCTCTAGAGCCTCTGCT
AACCATGTTCATGCCTTCTTCTTTTTCCTACAGCTCCTGGGCAACGTGCTGGTTATTG
TGCTGTCTCATCATTTTGGCAAAGAATTACTAGATCATGGAAGATGCCAAAAACATTA
AGAAGGGCCCAGCGCCATTCTACCCACTCGAAGACGGGACCGCCGGCGAGCAGCTG

CACAAAGCCATGAAGCGCTACGCCCTGGTGCCCGGCACCATCGCCTTTACCGACGCA
CATATCGAGGTGGACATTACCTACGCCGAGTACTTCGAGATGAGCGTTCGGCTGGCA
GAAGCTATGAAGCGCTATGGGCTGAATACAAACCATCGGATCGTGGTGTGCAGCGAG
AATAGCTTGCAGTTCTTCATGCCCGTGTTGGGTGCCCTGTTCATCGGTGTGGCTGTG
GCCCCAGCTAACGACATCTACAACGAGCGCGAGCTGCTGAACAGCATGGGCATCAGC
CAGCCCACCGTCGTATTCGTGAGCAAGAAAGGGCTGCAAAAGATCCTCAACGTGCAA
AAGAAGCTACCGATCATACAAAAGATCATCATCATGGATAGCAAGACCGACTACCAGG
GCTTCCAAAGCATGTACACCTTCGTGACTTCCCATTTGCCACCCGGCTTCAACGAGTA
CGACTTCGTGCCCGAGAGCTTCGACCGGGACAAAACCATCGCCCTGATCATGAACAG
TAGTGGCAGTACCGGATTGCCCAAGGGCGTAGCCCTACCGCACCGCACCGCTTGTGT
CCGATTCAGTCATGCCCGCGACCCCATCTTCGGCAACCAGATCATCCCCGACACCGC
TATCCTCAGCGTGGTGCCATTTCACCACGGCTTCGGCATGTTCACCACGCTGGGCTA
CTTGATCTGCGGCTTTCGGGTCGTGCTCATGTACCGCTTCGAGGAGGAGCTATTCTT
GCGCAGCTTGCAAGACTATAAGATTCAATCTGCCCTGCTGGTGCCCACACTATTTAGC
TTCTTCGCTAAGAGCACTCTCATCGACAAGTACGACCTAAGCAACTTGCACGAGATC
GCCAGCGGCGGGGCGCCGCTCAGCAAGGAGGTAGGTGAGGCCGTGGCCAAACGCT
TCCACCTACCAGGCATCCGCCAGGGCTACGGCCTGACAGAAACAACCAGCGCCATTC
TGATCACCCCCGAAGGGGACGACAAGCCTGGCGCAGTAGGCAAGGTGGTGCCCTTC
TTCGAGGCTAAGGTGGTGGACTTGGACACCGGTAAGACACTGGGTGTGAACCAGCG
CGGCGAGCTGTGCGTCCGTGGCCCCATGATCATGAGCGGCTACGTTAACAACCCCGA
GGCTACAAACGCTCTCATCGACAAGGACGGCTGGCTGCACAGCGGCGACATCGCCTA
CTGGGACGAGGACGAGCACTTCTTCATCGTGGACCGGCTGAAGAGCCTGATCAAATA
CAAGGGCTACCAGGTAGCCCCAGCCGAACTGGAGAGCATCCTGCTGCAACACCCCA
ACATCTTCGACGCCGGGGTCGCCGGCCTGCCCGACGACGATGCCGGCGAGCTGCCC
GCCGCAGTCGTCGTGCTGGAACACGGTAAAACCATGACCGAGAAGGAGATCGTGGA
CTATGTGGCCAGCCAGGTTACAACCGCCAAGAAGCTGCGCGGTGGTGTTGTGTTCGT
GGACGAGGTGCCTAAAGGACTGACCGGCAAGTTGGACGCCCGCAAGATCCGCGAGA
TTCTCATTAAGGCCAAGAAGGGCGGCAAGATCGCCGTGTAAGCGGCCGCAAGCTTC
GGGTGGCATCCCTGTGACCCCTCCCCAGTGCCTCTCCTGGCCCTGGAAGTTGCCAC
TCCAGTGCCCACCAGCCTTGTCCTAATAAAATTAAGTTGCATCATTTTGTCTGACTAG
GTGTCCTTCTATAATATTATGGGGTGGAGGGGGGTGGTATGGAGCAAGGGGCAAGTT
GGGAAGACAACCTGTAGGGCCTGCGGGGTCTATTGGGAACCAAGCTGGAGTGCAG
TGGCACAATCTTGGCTCACTGCAATCTCCGCCTCCTGGGTTCAAGCGATTCTCCTGC
CTCAGCCTCCCGAGTTGTTGGGATTCCAGGCATGCATGACCAGGCTCAGCTAATTTT
TGTTTTTTTGGTAGAGACGGGGTTTCACCATATTGGCCAGGCTGGTCTCCAACTCCT

AATCTCAGGTGATCTACCCACCTTGGCCTCCCAAATTGCTGGGATTACAGGCGTGAA
CCACTGCTCCCTTCCCTGTCCTTCGGTCTAGAAGGAACCCCTAGTGATGGAGTTGGC
CACTCCCTCTCTGCGCGCTCGCTCGCTCACTGAGGCCGGGCGACCAAAGGTCGCCC
GACGCCCGGGCTTTGCCCGGGCGGCCTCAGTGAGCGAGCGAGCGCGCAGAGAGGG
AGTGG3'

## Claims

1. A fusion polypeptide, comprising a secretory signal peptide and an aflibercept polypeptide, wherein the secretory signal peptide is any one selected from a human albumin secretory signal peptide, a human Opticin protein secretory signal peptide, a human interleukin-2 secretory signal peptide, and a mouse receptor tyrosine kinase-like orphan receptor 1 secretory signal peptide.

2. The fusion polypeptide according to claim 1, wherein an amino acid sequence of the aflibercept polypeptide comprises an amino acid sequence as set forth in SEQ ID NO: 48; and/or
an amino acid sequence of the secretory signal peptide comprises an amino acid sequence as set forth in any one of SEQ ID NOs: 44 to 47.

3. The fusion polypeptide according to claim 1 or 2, wherein an amino acid sequence of the fusion polypeptide comprises any one selected from the following sequences:
(i) an amino acid sequence as set forth in any one of SEQ ID NO: 1, 30, 32 or 34;
(ii) an amino acid sequence having at least 80%, 82%, 85%, 87%, 90%, 92%, 95%, 96%, 97%, 98% or 99% identity to the amino acid sequence as set forth in any one of SEQ ID NO: 1, 30, 32 or 34, and retaining a function of the amino acid sequence as set forth in any one of SEQ ID NO: 1, 30, 32 or 34;
(iii) an amino acid sequence obtained by adding, substituting, deleting or inserting one or more amino acid residues in the amino acid sequence as set forth in any one of SEQ ID NO: 1, 30, 32 or 34, and retaining a function of the amino acid sequence as set forth in any one of SEQ ID NO: 1, 30, 32 or 34; or
(iv) an amino acid sequence encoded by a nucleotide sequence that hybridizes under stringency conditions with a polynucleotide sequence encoding the amino acid sequence as set forth in any one of SEQ ID NO: 1, 30, 32 or 34, and the amino acid sequence retaining a function of the amino acid sequence as set forth in any one of SEQ ID NO: 1, 30, 32 or 34, wherein the stringency conditions are moderate stringency conditions, moderate-high stringency conditions, high stringency conditions, or very high stringency conditions.

4. An isolated polynucleotide encoding the fusion polypeptide according to any one of claims 1 to 3,
preferably, the polynucleotide comprises a nucleotide sequence as set forth in any one of SEQ ID NO: 2, 29, 31 or 33, or a nucleotide sequence having at least 80%, 82%, 85%, 87%, 90%, 92%, 95%, 96%, 97%, 98% or 99% identity to the nucleotide sequence as set forth in any one of SEQ ID NO: 2, 29, 31 or 33.

5. A gene expression cassette, comprising the polynucleotide according to claim 4.

6. The gene expression cassette according to claim 5, wherein the gene expression cassette further comprises, upstream of the polynucleotide encoding the fusion polypeptide, one or more of a promoter, an enhancer, an intron, and a Kozak sequence.

7. The gene expression cassette according to claim 6, wherein the promoter comprises any one of a CBh promoter, a CB7 promoter, and a CMV promoter;
preferably, the CBh promoter comprises a sequence as set forth in SEQ ID NO: 3 or a sequence having at least 85% identity thereto; and/or
the CB7 promoter comprises a sequence as set forth in SEQ ID NO: 4 or a sequence having at least 85% identity thereto; and/or
the CMV promoter comprises a sequence as set forth in SEQ ID NO: 5 or a sequence having at least 85% identity thereto.

8. The gene expression cassette according to claim 6 or 7, wherein the intron comprises a human β-globin intron; and
preferably, the human β-globin intron comprises a sequence as set forth in SEQ ID NO: 6 or a sequence having at least 85% identity thereto.

9. The gene expression cassette according to any one of claims 5 to 8, wherein the gene expression cassette comprises a polyadenylation region located downstream of the polynucleotide encoding the fusion polypeptide;
optionally, the polyadenylation region is any one selected from a bovine growth hormone polyadenylation region, a human growth hormone polyadenylation region, and a β-globin polyadenylation region;
preferably, the polyadenylation region comprises the human growth hormone polyadenylation region; and
more preferably, the human growth hormone polyadenylation region comprises a sequence as set forth in SEQ ID NO: 7 or a sequence having at least 85% identity thereto.

10. The gene expression cassette according to any one of claims 5 to 9, wherein the gene expression cassette comprises a sequence as set forth in any one of SEQ ID NOs: 8 to 10 and 35 to 43 or a sequence having at least 85% identity to any one of SEQ ID NOs: 8 to 10 and 35 to 43.

11. A gene delivery vector, comprising the gene expression cassette according to any one of claims 5 to 10.

12. The gene delivery vector according to claim 11, wherein the gene delivery vector is a viral vector derived from a virus; and
preferably, the gene delivery vector is a recombinant adeno-associated virus.

13. The gene delivery vector according to claim 12, wherein the recombinant adeno-associated virus comprises a capsid protein, and the gene expression cassette is encapsidated in the capsid protein; and
optionally, the capsid protein is any one selected from adeno-associated virus serotypes AAV1, AAV2, AAV3, AAV4, AAV5, AAV6, AAV7, AAV8, AAV9, and AAV10, or a variant thereof.

14. The gene delivery vector according to claim 13, wherein the capsid protein is an AAV2 capsid protein or a variant thereof;
preferably, the capsid protein is an AAV2 capsid protein variant;
more preferably, the AAV2 capsid protein variant comprises a sequence as set forth in any one of SEQ ID NOs: 16 and 70 to 83, or a sequence having at least 85% identity to any one of SEQ ID NOs: 16 and 70 to 83; and
further preferably, the AAV2 capsid protein variant comprises a sequence as set forth in SEQ ID NO: 16 or 82, or a sequence having at least 85% identity to SEQ ID NO: 16 or 82.

15. A pharmaceutical composition, comprising the fusion polypeptide according to any one of claims 1 to 3, the polynucleotide according to claim 4, the gene expression cassette according to any one of claims 5 to 10 or the gene delivery vector according to any one of claims 11 to 14, and
optionally, a pharmaceutically acceptable carrier.

16. Use of the fusion polypeptide according to any one of claims 1 to 3, the polynucleotide according to claim 4, the gene expression cassette according to any one of claims 5 to 10 or the gene delivery vector according to any one of claims 11 to 14 in preparation of a medicament for preventing and/or treating a disease, wherein
optionally, the disease is an eye disease;
preferably, the eye disease is an eye disease associated with ocular neovascularization or with choroidal neovascularization; and
more preferably, the eye disease is one or more selected from wet age-related macular degeneration, macular edema secondary to retinal vein occlusion, diabetic macular edema, and diabetic retinopathy.

17. A method for preventing and/or treating a disease, comprising administering, to a subject, a prophylactically and/or therapeutically effective amount of the fusion polypeptide according to any one of claims 1 to 3, the polynucleotide according to claim 4, the gene expression cassette according to any one of claims 5 to 10, the gene delivery vector according to any one of claims 11 to 14 or the pharmaceutical composition according to claim 15, wherein
optionally, the disease is an eye disease;
preferably, the eye disease is an eye disease associated with ocular neovascularization or with choroidal neovascularization; and
more preferably, the eye disease is one or more selected from wet age-related macular degeneration, macular edema secondary to retinal vein occlusion, diabetic macular edema, and diabetic retinopathy.
